# EUROPEAN PATENT SPECIFICATION

(11) **EP 2 791 101 B1**
(45) Date of publication and mention of the grant of the patent: **18.09.2019**
(21) Application number: 12856859.9
(22) Date of filing: 11.12.2012
(51) Int. Cl.: C07C 53/00, A61K 31/685, A61K 31/404, A61K 31/366, A61K 31/585, A61P 29/00

(54) **TREATMENT OF NON-ALCOHOLIC STEATOHEPATITIS**
BEHANDLUNG VON NICHT-ALKOHOLISCHER STEATOHEPATITIS
TRAITEMENT DE LA STÉATOHÉPATITE NON ALCOOLIQUE

(30) Priority: 12.12.2011 US 201161569545 P; 12.12.2011 US 201161569481 P
(43) Date of publication of application: 22.10.2014
(73) Proprietor: Vascular Biogenics Ltd., Modiin 7178106 (IL)
(72) Inventor: MENDEL, Itzhak, Rehovot (IL); FEIGE, Erez, Hemed (IL); YACOV, Niva, Tel Aviv (IL); PROPHETA-MEIRAN, Oshrat, Petach Tikva (IL); BREITBART, Eyal, Hashmonaiim (IL); SALEM, Yaniv, Kyriat Ono (IL)
(74) Representative: Vossius & Partner Patentanwälte Rechtsanwälte mbB
(86) International application number: PCT/IB2012/002930
(87) International publication number: WO 2013/088245

(56) References cited:
- WO-A1-2011/083469
- US-A1- 2006 140 936
- US-A1- 2011 189 212
- LEITINGER N ET AL: "STRUCTURALLY SIMILAR OXIDIZED PHOSPHOLIPIDS DIFFERENTIALLY REGULATE ENDOTHELIAL BINDING OF MONOCYTES AND NEUTROPHILS", PROCEEDINGS OF THE NATIONAL ACADEMY OF SCIENCES, NATIONAL ACADEMY OF SCIENCES, US, vol. 96, no. 21, 12 October 1999 (1999-10-12), pages 12010-12015, XP002951991, ISSN: 0027-8424, DOI: 10.1073/PNAS.96.21.12010
- N Pidkovka: "POVPC induces the smooth muscle cells inflammatory phenotype -- Pidkovka et al. 21 (5): A517 -- The FASEB Journal", Faseb Journal, vol. 21, no. 5, A517, 1 January 2007 (2007-01-01), XP055189396, Retrieved from the Internet: URL:http://www.fasebj.org/cgi/content/meet ing_abstract/21/5/A517 [retrieved on 2015-05-15]
- MENDEL ITZHAK ET AL: "Treatment with Oxidized Phospholipids Directly Inhibits Nonalcoholic Steatohepatitis and Liver Fibrosis Without Affecting Steatosis", DIGESTIVE DISEASES AND SCIENCES, SPRINGER NEW YORK LLC, US, vol. 61, no. 9, 13 April 2016 (2016-04-13) , pages 2545-2553, XP036027833, ISSN: 0163-2116, DOI: 10.1007/S10620-016-4159-5 [retrieved on 2016-04-13]

## Description

### FIELD AND BACKGROUND OF THE INVENTION

The present invention relates to the compound 1-hexadecyl-2-(4'-carboxybutyl)-glycerol-3-phosphocholine for use in the treatment of an inflammatory disease or disorder which is non-alcoholic steatohepatitis (NASH).

Oxidized phospholipids have been previously described as useful in the treatment of medical conditions such as, for example, cardiovascular diseases, cerebrovascular diseases and inflammatory diseases and disorders.

International Patent Application No. PCT/IL2004/000453 (Publication No. WO 04/106486) describes oxidized lipids for prevention and treatment of inflammation associated with endogenous oxidized lipids. An exemplary such compound is described and known as CI-201 (1-hexadecyl-2-(4'-carboxybutyl)-glycerol-3-phosphocholine; also referred to in the art as VB-201).

International Patent Application No. PCT/IL01/01080 (Publication No. WO 02/41827) describes oxidized lipids for prevention and treatment of atherosclerosis and related diseases.

International Patent Application PCT/IL2011/000012 (published as WO 2011/083469) describes novel unit dosage forms, methods and treatment regimens, utilizing VB-201 are disclosed. International Patent Application PCT/IL2011/000012 further describes inhibition by VB-201 of toll-like receptor activation and chemotaxis.

Additional background art includes International Patent Application Nos. PCT/IL09/000949 (Publication No. WO 10/041242), PCT/IL09/001049 (Publication No. WO 10/052718), PCT/IL05/000735 (Publication No. WO 06/006161), PCT/IL02/00005 (Publication No. WO 02/053092) and PCT/IL08/000013 (Publication No. WO 08/084472).

Toll-like receptors (TLRs) are a family of receptors imperative for the innate immune response against microbial invasion. TLRs can be divided into two major subgroups based on their cellular localization. Plasma membrane expressed TLRs include TLR1, TLR2, TLR4, TLR5 and TLR6, whereas the intracellular TLRs include TLR3, TLR7, TLR8, and TLR9. The interaction between TLRs with their cognate agonists instigates a cascade of cues which include recruitment of the adaptor molecules MyD88/TRIF and downstream phosphorylation of MAPK kinases and NF-κB. These events culminate in the secretion of proinflammatory cytokines, including IL-12/23, IL-6 and TNF-α. TLR2 forms a heterodimer with TLR1 which recognizes bacterial triacylated lipopeptides, and a heterodimer with TLR6 which recognizes bacterial diacylated lipopeptides. TLR4 coupled to MD2 in complex with lipopolysaccharide-binding protein (LBP) and the co-receptor CD14 bind lipopolysaccharide (LPS) from gram negative bacteria.

Monocytes are key players in the immune system, with critical roles in innate and adaptive immunity, immune surveillance and particle scavenging. Whereas a subset of monocytes is "resident" and recruited to tissues independently of inflammatory stimuli to assist in steady-state surveillance, wound-healing and resolution of inflammation, the absolute majority (80-90 %) of human circulating monocytes is classified as "inflammatory" [Kamei & Carman, Curr Opin Hematol 2010, 17:43-52]. These monocytes can sense inflammatory stimuli and quickly migrate through the vascular or lymphatic endothelium to the periphery, where they can differentiate into macrophages and dendritic cells (DCs) which cooperate with additional cell subsets (such as Th1-cells) to promote inflammation. While playing a necessary role in host defense, monocytes were nonetheless identified as critical mediators of several inflammatory diseases, including atherosclerosis, rheumatoid arthritis (RA) and multiple sclerosis (MS) [Zhao, J Leukoc Biol. 2010, 88:41-55; Moore & Tabas, Cell. 2011, 145:341-355; Mildner et al., Brain. 2009, 132(Pt 9):2487-2500]. Suppressing the accumulation of unwanted monocytes/macrophages in a chronically inflamed tissue has therapeutic potential, and migration inhibitors have accordingly demonstrated promising anti-inflammatory results in animal models and clinical trials [Mackay, Nat Immunol. 2008, 9:988-998].

Extensive research has shown that chemokine receptors and adhesion molecules play a key role in regulation of leukocyte trafficking (reviewed in Kamei & Carman [Curr Opin Hematol 2010; 17:43-52] and Imhof & Aurrand-Lions [Nat Rev Immunol. 2004, 4:432-444]). A complex array of chemokines receptors, G-protein coupled receptors (GPCRs) that are differentially expressed on leukocyte lineages and subsets, regulates which cell types would migrate and to which tissue, under different conditions. Chemokines or chemotactic cytokines are secreted proteins that regulate migration and activation of leukocytes and stromal cells. In the case of inflammatory monocytes, exit from the bone marrow across a monolayer of endothelial cells (i.e. diapedesis) to enter the circulatory system (i.e. intravasation) and to migrate to the inflamed tissue is dependent on C-C motif receptor 2 (CCR2) signaling, in response to activation by chemokine C-C motif ligand (CCL) 2 (also known as monocyte chemotactic protein-1; MCP-1) and CCL7 (MCP-3). On the other hand, constitutive migration of resident monocytes to non-inflamed tissues is mostly dependent on CCL3 (also known as Macrophage inflammatory protein-1α; MIP-1α) and chemokine (C-X3-C motif) ligand 1 (CX3CL1) [Kamei & Carman, Curr Opin Hematol 2010, 17:43-52].

Atherosclerosis is a complex disorder involving lipid retention, inflammation, oxidative stress and endothelial dysfunction. Cellular and humoral immune responses act to influence the size and composition of the atheromatous plaque. Monocytes play a significant role in initiation and progression of atherosclerosis. Evidence to their critical role can be found in studies of the G-CSF mutation in mice which abolished aortic atherogenesis in the ApoE knockout (KO) model in spite of high lipid levels [Smith et al., Proc Natl Acad Sci USA. 1995, 92:8264-8268; Qiao et al., Am J Pathol. 1997, 15:1687-1699]. Conditions of atherosclerosis induce rapid influx of inflammatory monocytes to the vessel wall which is followed by differentiation to inflammatory macrophages and DCs. These cells, together with resident macrophages and dendritic cells that undergo phenotypic changes as well as infiltrating T-cells [Pinderski et al., Circ Res. 2002, 90:1064-1071; Potteaux et al., Arterioscler Thromb Vasc Biol. 2004, 24:1474-1478], modulate the inflammatory milieu within the arterial wall [Ley et al., Arterioscler Thromb Vasc Biol. 2011, 31:1506-1516]. Powerful regulators of the immune system in atherosclerosis include the anti-inflammatory cytokines IL-10 and transforming growth factor-β (TGF-β), whereas IL-12 appears to enhance atherogenesis by driving the commitment of T helper cells to the Th₁ lineage and serving as a potent chemoattractant of T cells to atheromatous lesions [Zhang et al., Circ Res. 2006, 98:524-531; Kleemann et al., Cardiovasc Res. 2008, 79:360-376].

Additional background art includes Jin & Lee [Immunity 2008, 29:182-191], Kim et al. [J Biol Chem 2005, 280:11347-11351], Kawai & Akira [Nature Immunol 2010, 11:373-384], and Perera et al. [J Immunol 2001, 166:574-581].

### SUMMARY OF THE INVENTION

The present invention provides the compound 1-hexadecyl-2-(4'-carboxybutyl)-glycerol-3-phosphocholine for use in the treatment of an inflammatory disease or disorder which is non-alcoholic steatohepatitis (NASH).

In particular, the invention relates to the following embodiments:
1. A compound, which is 1-hexadecyl-2-(4'-carboxybutyl)-glycerol-3-phosphocholine, for use in the treatment of an inflammatory disease or disorder, wherein said inflammatory disease or disorder is non-alcoholic steatohepatitis (NASH).
2. The compound for use according to embodiment 1, wherein said compound is (*R*)-1-hexadecyl-2-(4'-carboxybutyl)-*sn*-glycerol-3-phosphocholine.
3. The compound for use according to embodiment 1 or 2, wherein said compound exhibits at least two activities selected from the group consisting of:
   a) inhibiting CD14 activity and/or a signaling pathway associated with CD14 activity;
   b) inhibiting TLR2 activity and/or a signaling pathway associated with TLR2 activity; and
   c) inhibiting monocyte chemotaxis.
4. The compound for use according to embodiment 3, wherein said activities are: inhibiting CD14 activity and/or a signaling pathway associated with CD14 activity; and inhibiting TLR2 activity and/or a signaling pathway associated with TLR2 activity.
5. The compound for use according to embodiment 3, wherein said activities are: inhibiting CD14 activity and/or a signaling pathway associated with CD14 activity; and inhibiting monocyte chemotaxis.
6. The compound for use according to embodiment 3, wherein said activities are: inhibiting TLR2 activity and/or a signaling pathway associated with TLR2 activity; and inhibiting monocyte chemotaxis.
7. The compound for use according to embodiment 3, wherein said activities are: inhibiting CD14 activity and/or a signaling pathway associated with CD14 activity; inhibiting TLR2 activity and/or a signaling pathway associated with TLR2 activity; and inhibiting monocyte chemotaxis.
8. The compound for use according to any of embodiments 1 to 7, wherein said compound is to be administered to a human.
9. The compound for use according to any of embodiments 1 to 8, wherein said compound inhibits TLR4 activity.
10. The compound for use according to any of embodiments 1 to 9, wherein the 1-hexadecyl-2-(4'-carboxybutyl)-glycerol-3-phosphocholine is in the form of a pharmaceutically acceptable salt, hydrate or solvate.
11. The compound for use according to any of embodiments 1 to 9, wherein said compound is at least one agent capable of exhibiting at least two activities selected from
   (a) inhibiting CD14 activity and/or a signaling pathway associated with CD14 activity,
   (b) inhibiting TLR2 activity and/or a signaling pathway associated with TLR2 activity, and (c) inhibiting monocyte chemotaxis, and wherein said agent is selected from 1-hexadecyl-2-(4'-carboxybutyl)-glycerol-3-phosphocholine and pharmaceutically acceptable salts, hydrates and solvates thereof.
12. The compound for use according to any of embodiments 1 to 11, wherein said compound is to be administered orally.

### BRIEF DESCRIPTION OF THE DRAWINGS

Some embodiments of the invention as well as reference embodiments are herein described, by way of example only, with reference to the accompanying drawings. With specific reference now to the drawings in detail, it is stressed that the particulars shown are by way of example and for purposes of illustrative discussion. In this regard, the description taken with the drawings makes apparent to those skilled in the art how embodiments of the invention and reference embodiments may be practiced.

In the drawings:

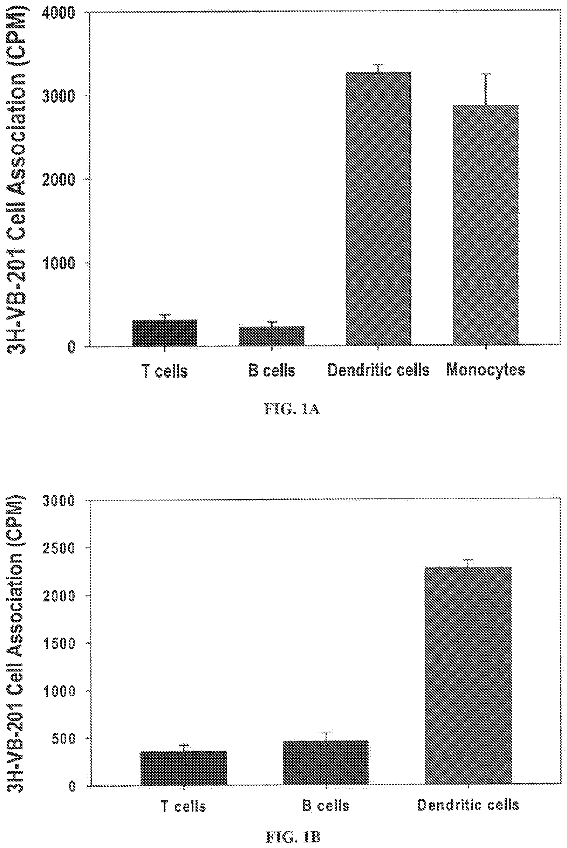

FIGs. 1A and 1B are graphs showing ³H-labeled VB-201 uptake by human CD14+ monocytes, monocyte-derived dendritic cells (DCs), CD4+ T cells, and CD19+ B cells (FIG. 1A), and by mouse CD11c+ mouse bone marrow-derived dendritic cells, CD90+ T cells, and CD45R+ B cells (FIG. 1B);

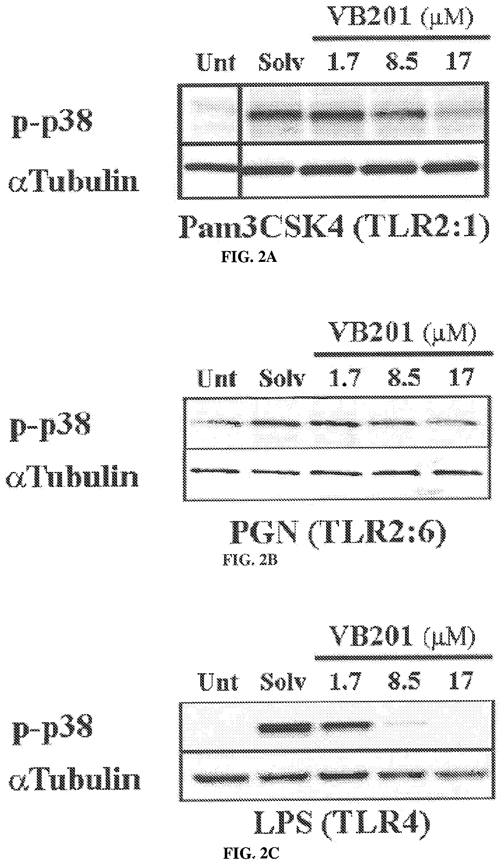

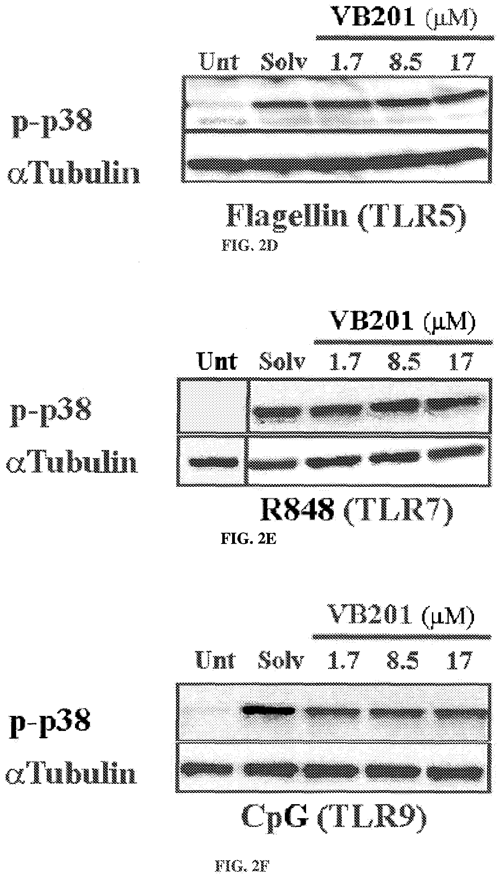

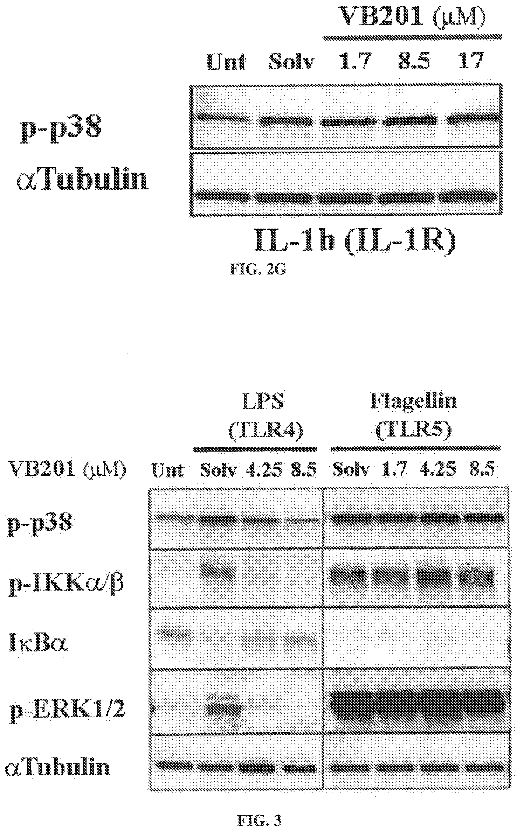

FIGs. 2A-2G present images of Western Blots of phosphorylated p38 (p-p38) in mouse bone marrow derived cells (BMDCs) pretreated with 1.7, 8.5 or 17 µM VB-201 or with solvent (Solv), or untreated (Unt), and then stimulated with Pam3CSK4 (a TLR2:1 agonist; Figure 2A), peptidoglycan (PGN, a TLR2:6 agonist; Figure 2B), lipopolysaccharide (LPS, a TLR4 agonist; Figure 2C), flagellin (a TLR5 agonist; Figure 2D), R848 (a TLR7 agonist; Figure 2E), CpG (a TLR9 agonist; Figure 2F) or IL-1β (an IL-1 receptor agonist; Figure 2G) (α-tubulin levels serve as a loading control);
FIG. 3 presents images of Western Blots of phosphorylated p38 (p-p38), phosphorylated IKKα/β (p-IKKα/β), IκBα, and phosphorylated ERK1/2 (p-ERK1/2) in human monocytes pretreated with 1.7, 8.5 or 17 µM VB-201 or with solvent (Solv), or untreated (Unt), and then stimulated with flagellin (a TLR5 agonist) or lipopolysaccharide (LPS, a TLR4 agonist) (α-tubulin levels serve as a loading control);

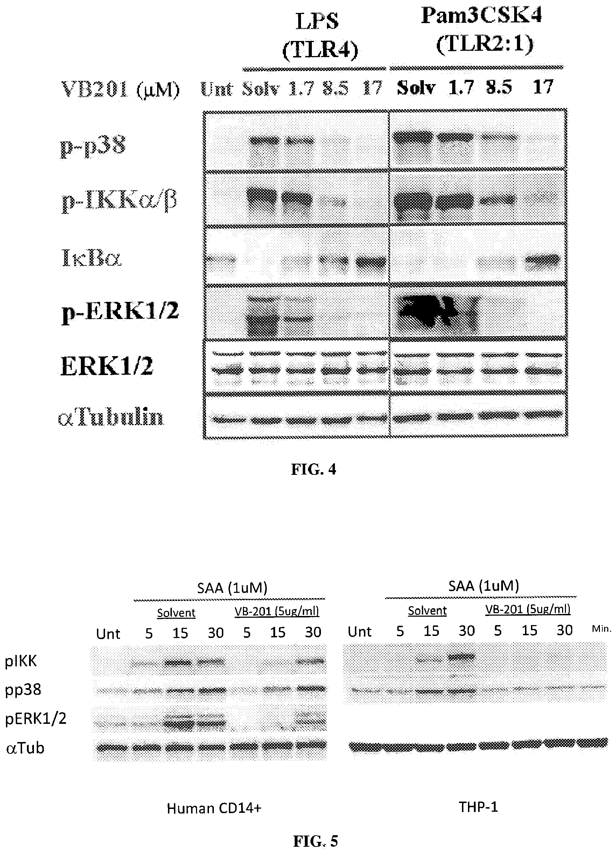

FIG. 4 presents images of Western Blots of phosphorylated p38 (p-p38), phosphorylated IKKα/β (p-IKKα/β), IκBα, and phosphorylated ERK1/2 (p-ERK1/2) in mouse peritoneal macrophages pretreated with 1.7, 8.5 or 17 µM VB-201 or with solvent (Solv), or untreated (Unt), and then stimulated with Pam3CSK4 (a TLR2:1 agonist) or lipopolysaccharide (LPS, a TLR4 agonist) (total ERK1/2 and α-tubulin levels serve as loading controls);
FIG. 5 presents images of Western Blots of phosphorylated p38 (pp38), phosphorylated IKK (pIKK), and phosphorylated ERK1/2 (pERK1/2) in human CD14+ cells (left) and in THP-1 cells (right) 5, 10 or 30 minutes after treatment with 1 µM serum amyloid A (SAA), following pretreatment with solvent or with 5 µg/ml VB-201, or with no SAA treatment (Unt) (α-tubulin (αTub) levels serve as loading controls);

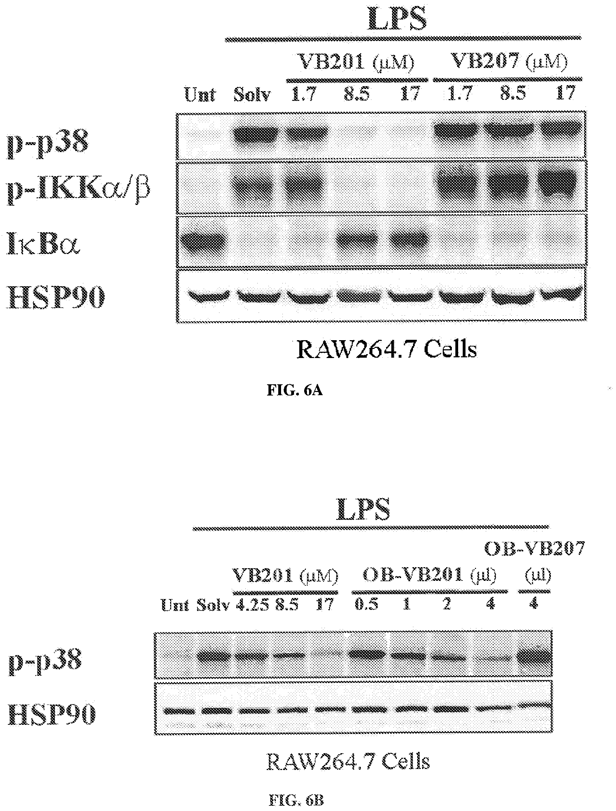

FIGs. 6A and 6B presents images of Western Blots of phosphorylated p38 (p-p38; Figures 6A and 6B), phosphorylated IKKα/β (p-IKKα/β; Figure 6A), and IκBα (Figure 6A) in RAW 264.7 macrophages pretreated with 1.7, 8.5 or 17 µM of VB-201 or VB-207 (Figure 6A), 4.25, 8.5 or 17 µM VB-201 (Figure 6B), or 0.5, 1, 2 or 4 µl of a solution of ovalbumin-biotin-labeled VB-201 (OB-VB201) or VB-207 (OB-VB207) (Figure 6B), with solvent (Solv), or untreated (Unt), and then stimulated with LPS (HSP90 levels serve as a loading control);

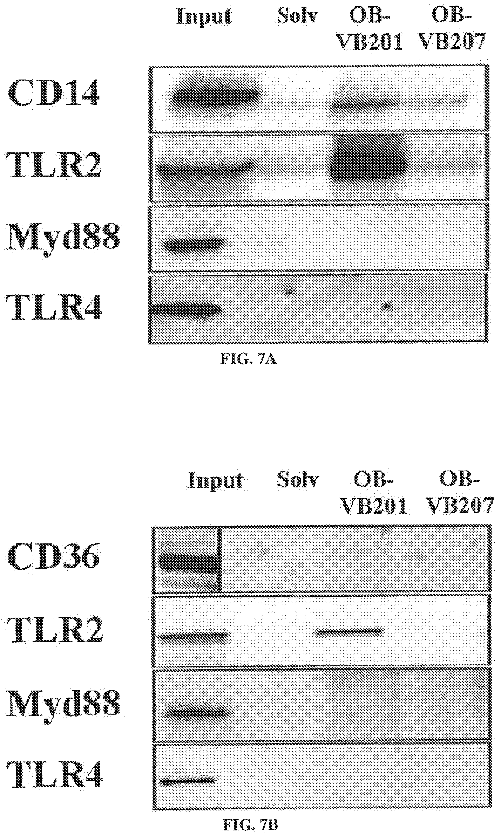

FIGs. 7A and 7B presents images of Western Blots of CD14 (Figure 7A), CD36 (Figure 7B), TLR2, MyD88 and TLR4 from lysates of primary human monocytes (Figure 7A) or THP-1 cells (Figure 7B) which precipitated with streptavidin beads following treatment with solvent (Solv) or ovalbumin-biotin-labeled VB-201 (OB-VB201) or VB-207 (OB-VB207); input lane represents 1 % of the whole cell lysate prior to precipitation, as a control;

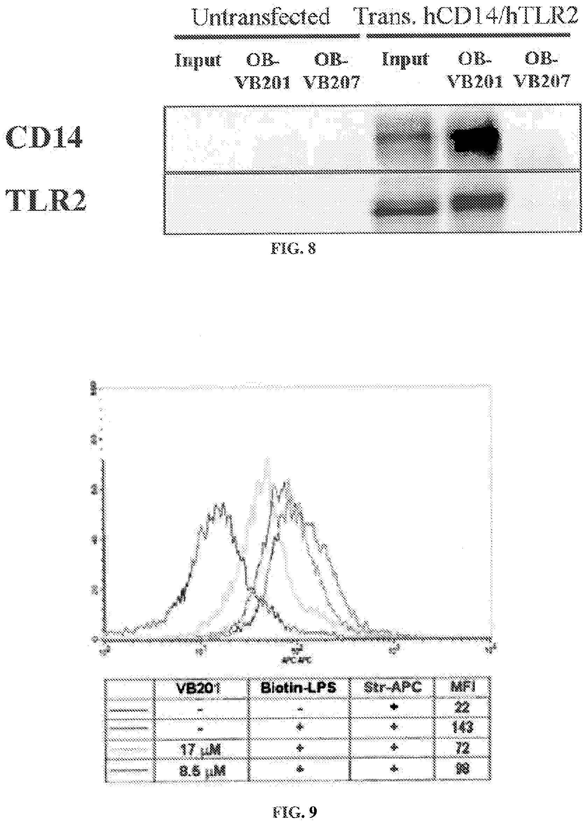

FIG. 8 presents images of Western Blots of CD14 and TLR2 from lysates of untransfected HEK293 cells or of transfected HEK 293 cells (Trans.) expressing high levels of CD14 (hCD14) or TLR2 (hTLR2), respectively, which precipitated with streptavidin beads following treatment with ovalbumin-biotin-labeled VB-201 (OB-VB201) or VB-207 (OB-VB207); input lane represents 1 % of the whole cell lysate prior to precipitation, as a control;
FIG. 9 is a graph showing the effect of treatment with 0, 8.5 or 17 µM VB-201, on binding of biotinylated lipopolysaccharide (Biotin-LPS) to RAW 264.7 cells, which was detected by staining with streptavidin-APC (Str-APC);

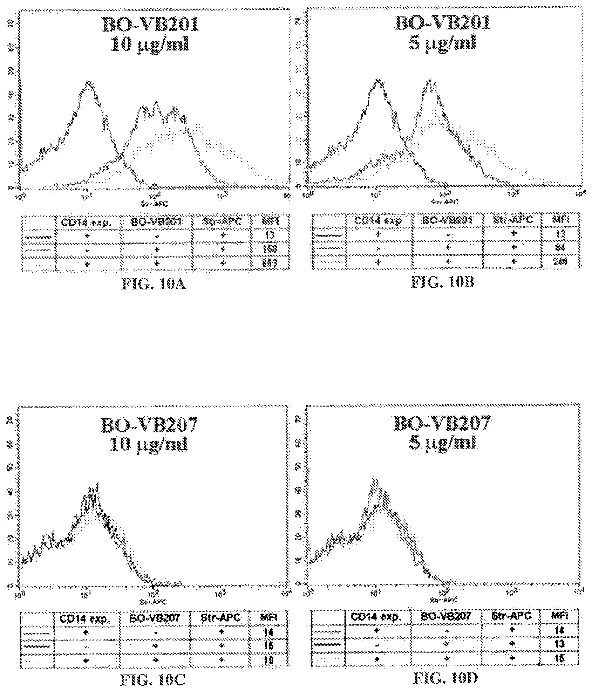

FIGs. 10A-10D are graphs showing the effect of CD14 expression on cell fluorescence in HEK 293 cells treated with 5 µg/ml (Figures 10B and 10D) or 10 µg/ml (Figure 10A and 10C) of ovalbumin-biotin-labeled VB-201 (BO-VB201 protein concentration; Figures 10A and 10B) or ovalbumin-biotin-labeled VB-207 (BO-VB207 protein concentration; Figures 10C and 10D); binding of BO-VB201 and BO-VB207 was detected by staining with streptavidin-APC (Str-APC);

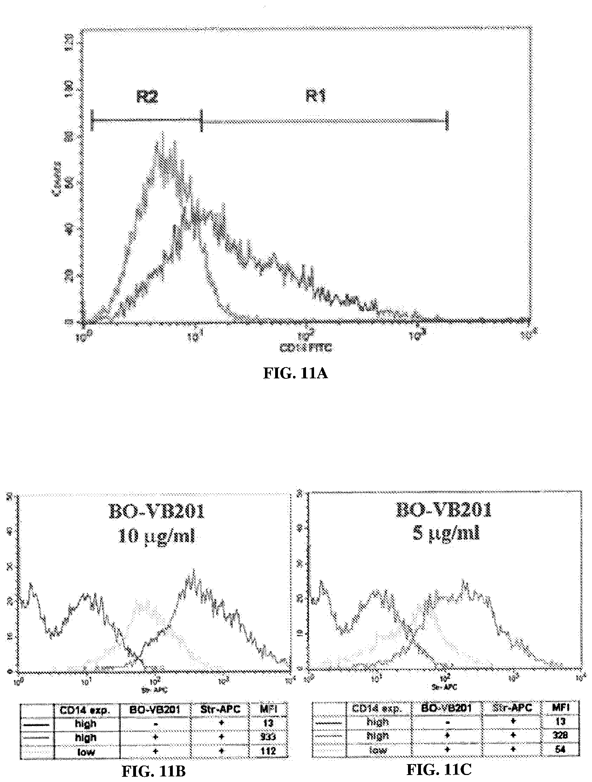

FIGs. 11A-11C are a graph showing high expression (R1) or low expression (R2) of CD14 in transfected HEK 293 cells (Figure 11A), and graphs showing the effect of high and low CD14 expression on fluorescence of HEK 293 cells treated with 5 µg/ml (protein concentration) (Figure 11C) or 10 µg/ml (protein concentration) (Figure 11B) of BO-VB201; binding of BO-VB201 was detected by staining with streptavidin-APC (Str-APC);

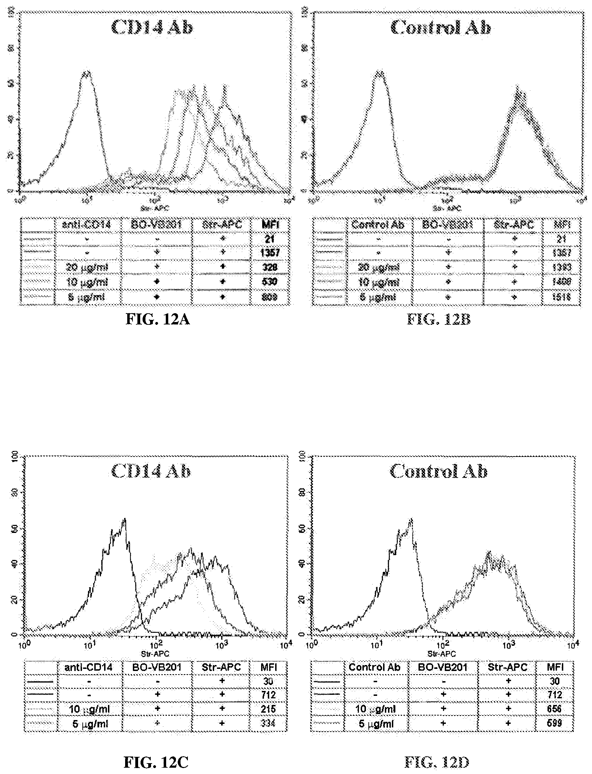

FIGs. 12A-12D are graphs showing the effect of 0, 5, 10 and 20 µg/ml anti-CD 14 antibodies (Figures 12A and 12C) and control antibodies (Figures 12B and 12D) on binding of ovalbumin-biotin-labeled VB-201 (BO-VB201) to HEK-Blue-4 cells (Figures 12A and 12B) and human monocytes (Figures 12C and 12D); binding of BO-VB201 was detected by staining with streptavidin-APC (Str-APC);

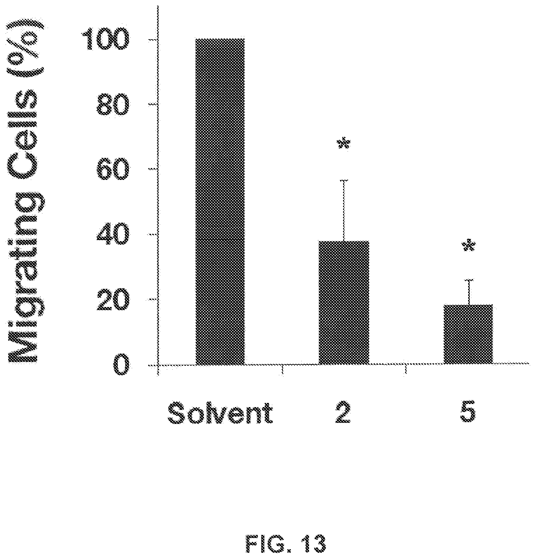

FIGs 13 is a bar graph showing the amount of THP-1 cells in a trans-well chemotaxis assay, following pretreatment for 30 minutes with solvent, 2 or 5 µg/ml VB-201 (results with solvent are defined as 100 %; asterisks indicate p < 0.05);

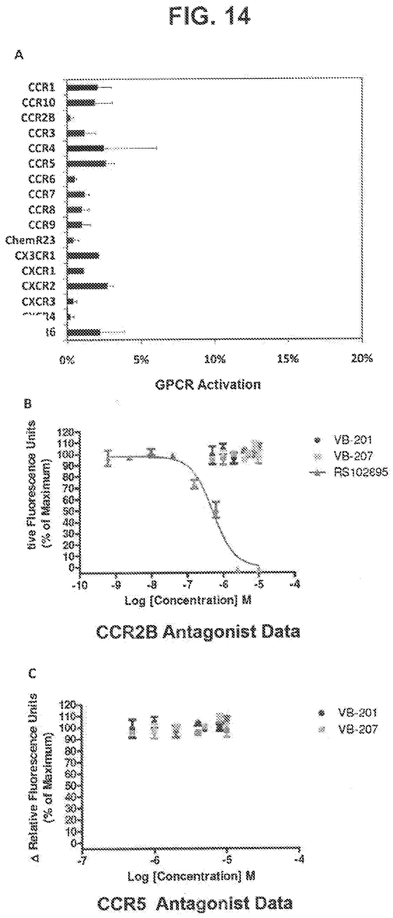

FIG. 14A is a bar graph showing the effect of 12.5 µM VB-201 on calcium flux in a G-protein coupled receptor (GPCR) activation assay for various chemokine receptors; Figure 14B and 14C are graphs demonstrating that VB-201 does not act as an antagonist of the CCR2B or CCR5 chemokine receptors.

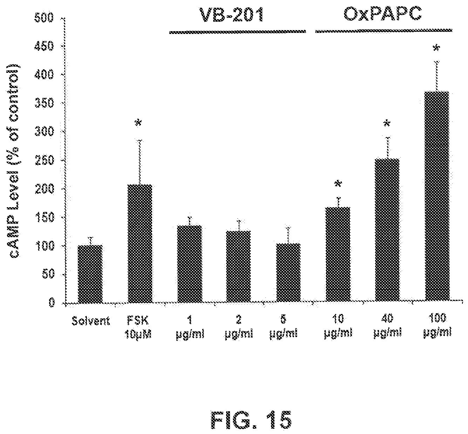

FIG. 15 is a bar graph showing cAMP levels in human monocyte derived dendritic cells treated with solvent, forskolin (FSK), or escalating doses of VB-201 or oxidized PAPC (Ox-PAPC) (asterisks indicate p < 0.05);

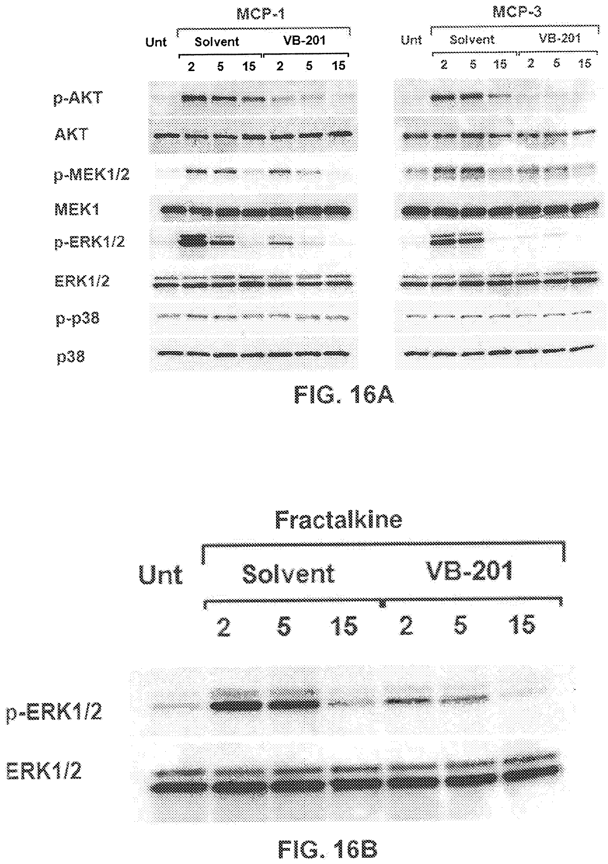

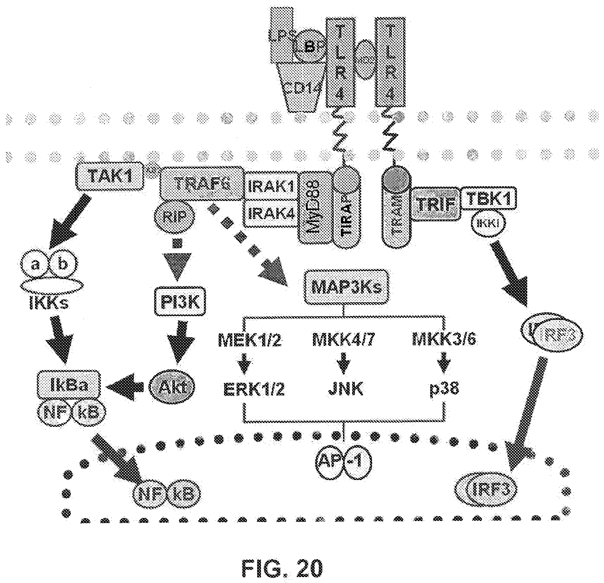

FIGs. 16A and 16B presents images of Western Blots of phosphorylated Akt (p-AKT; Figure 20A), phosphorylated MEK1/2 (p-MEK1/2; Figure 20A), phosphorylated p38 (p-p38; Figure 16A) and phosphorylated ERK1/2 (p-ERK1/2; Figures 16A and 16B) in monocytes incubated for 20 minutes with solvent or 5 µg/ml VB-201 prior to stimulation for 2, 5 or 15 minutes with 20 ng/ml MCP-1 (Figure 16A), 50 ng/ml MCP-3 (Figure 16A) or 50 ng/ml Fractalkine (Figure 16B) (levels of total (phosphorylated and non-phosphorylated) protein serve as controls);

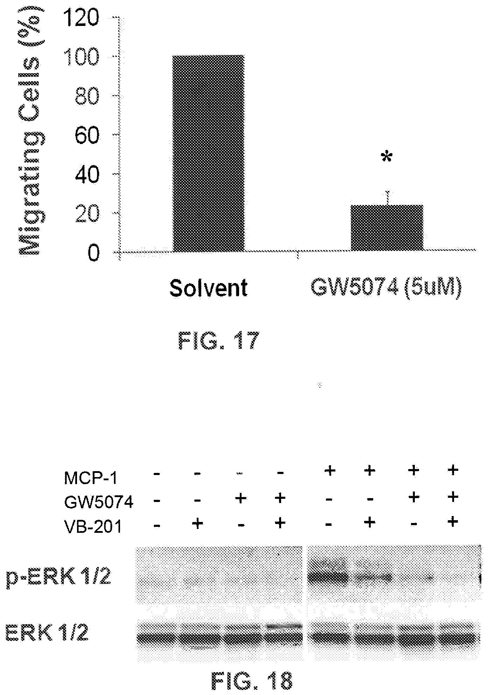

FIG. 17 is a bar graph showing the amount of migrating monocytes in a trans-well chemotaxis assay, following pretreatment with solvent or 5 µM of GW5074 (results with solvent are defined as 100 %; asterisk indicates p < 0.05);
FIG. 18 presents images of Western Blots of phosphorylated ERK1/2 (p-ERK1/2) in monocytes with and without MCP-1 stimulation, 5 µg/ml VB-201 and/or 2.5 µM GW5074 (total ERK1/2 levels serve as a loading control);

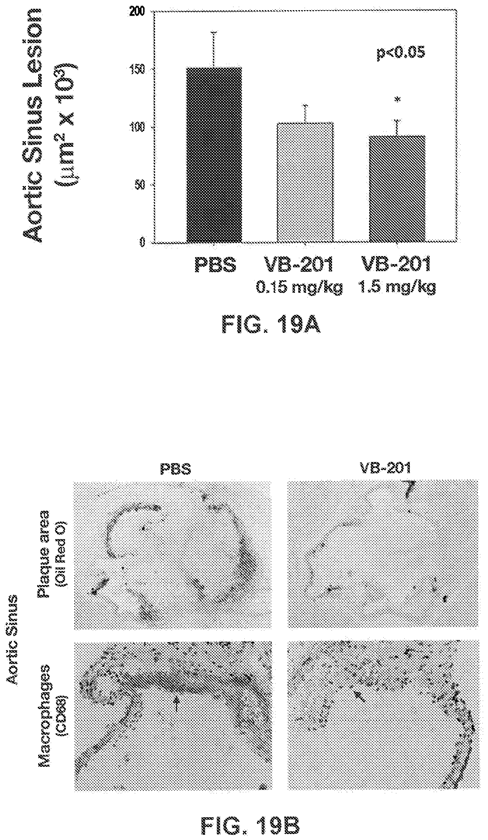

FIGs. 19A and 19B present a bar graph showing aortic sinus lesion areas in ApoE knockout mice treated daily for 8 weeks with PBS or 0.15 or 1.5 mg/kg VB-201 (Figure 19A) and images of atherosclerotic plaque from the mice, stained to show plaque area (Oil Red O stain) and macrophages (anti-CD68 stain) in the plaque (Figure 19B);
FIG. 20 is a scheme depicting features of CD14 and TLR4 signaling pathways (yellow circles indicate plasma membrane and brown circles indicate nucleus); and

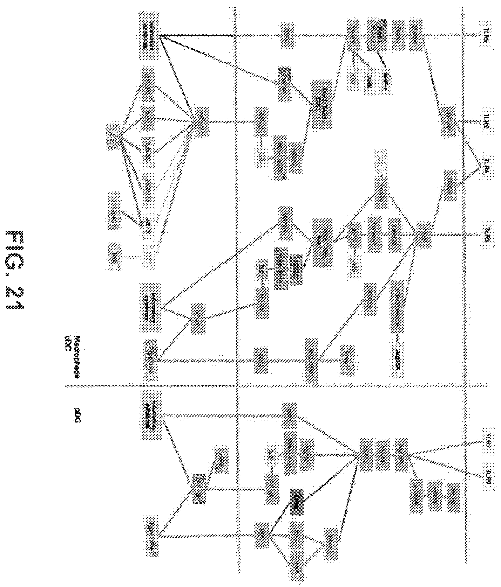

FIG. 21 is a scheme (from Kawai & Akira [Nature Immunol 2010, 11:373-384]) depicting features of toll-like receptor (TLR) signaling pathways; TLR-mediated responses are controlled mainly by the MyD88 dependent pathway, which is used by all TLRs except TLR3, and the TRIF-dependent pathway, which is used by TLR3 and TLR4; TRAM and TIRAP are adaptors used by TLR4 and TLR2-TLR4, respectively; in conventional dendritic cells (cDCs) and macrophages, MyD88 recruits IRAK4, IRAK1, IRAK2 and TRAF6 and induces inflammatory responses by activating NF-κB, MAPK and IRF5; TRAF6 activates TAK1 in complex with TAB2 and TAB3, and activates the IKK complex consisting of NEMO and IKKα/β, which catalyze IκB proteins for phosphorylation; NF-κB induces C/EBPδ, IκBζ, IκB-NS, Zc3h12a, ATF3 and tristeraprolin (TTP), which influence the genes encoding IL-6, IL-12p40 or TNF; TRIF recruits TRAF6, TRADD and TRAF3; TRADD interacts with Pellino-1 and RIP1; RIP1 and TRAF6 cooperatively activate TAK1, which leads to activation of MAPK and NF-κB; TRAF3 activates the kinases TBK1 and IKKi, which phosphorylate and activate IRF3, the last of which controls transcription of type I interferon; Nrdp1 is involved in TBK1-IKKi activation; the TRIF-dependent pathway leads to inflammasome activation during TLR4 signaling; in plasmacytoid dendritic cells (pDCs), TLR7 and TLR9 recruit MyD88 along with IRAK4 and TRAF6, which activate IRF5 and NF-κB for inflammatory cytokine induction and IRF7 for type I interferon induction; for IRF7 activation, IRAK1- and IKKα-dependent phosphorylation is required, and TRAF3 is located upstream of these kinases; OPNi is involved in IRF7 activation, and IRF8 facilitates NF-κB activation; the PI3K-mTOR-p70S6K axis enhances the TLR7 and TLR9 signaling pathways; IRF1 is involved in the induction of type I interferon by TLR7 and TLR9 in cDCs; TANK suppresses TRAF6, A20 suppresses TRAF6 and RIP1, ATG16A suppresses inflammasome activation and SHP-1 suppresses IRAK1 and IRAK2 (yellow - TLRs; green - stimulators; pink - negative regulators; blue - target genes).

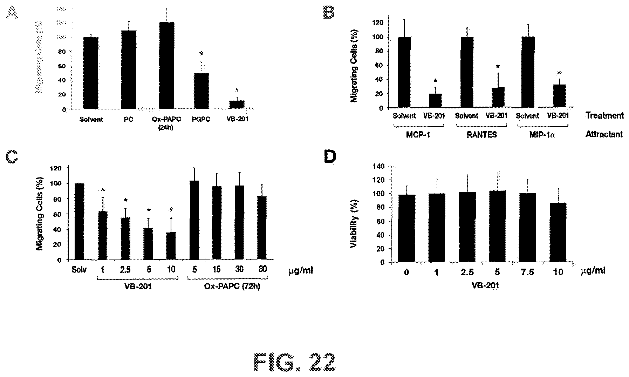

FIGS. 22A-D are bar graphs depicting the effectof VB-201 on chemotaxis of human monocytes *in vitro.* In FIG. 22A, human monocytes (CD14⁺) isolated from blood of healthy donors were pre-treated for 30 min with either solvent, Phosphatidylcholine (PC; 5 µg/ml), oxidized 1-palmitoyl-2-arachidonoyl-sn-glycero-3-phosphocholine (PAPC) (40 µg/ml, 24h oxidation), 1-palmitoyl-2-glutaryl phosphatidylcholine (PGPC) (5 µg/ml) or VB-201 (5 µg/ml), and then subjected to transwell chemotaxis assay, using MCP-1 and RANTES mix (50 ng/ml each) for attraction. The number of cells migrating to the lower compartment was determined by FACS, and normalized to the solvent control. Data are mean±SD from a representative experiment performed in triplicates. In FIG. 22B, CD14⁺ cells were treated with solvent or VB-201 (5 µg/ml) prior to chemotaxis assay using MCP-1 (50 ng/ml), MIP-1α (50 ng/ml) or RANTES (100 ng/ml) as attractants. Data are mean±SD (n=3, in triplicates). In FIG. 22C, the effect of escalating doses of VB-201 or oxidized-PAPC (Ox-PAPC) on CD14⁺ migration towards HUVEC supernatant. Data represent mean±SD (n=3, in triplicates). In FIG. 22D, an MTS assay in human monocytes subjected to escalating doses of VB-201 for 16 hrs, demonstrates that VB-201-inhibited chemotaxis is not due to reduced cell viability (mean±SD; n=7 in quadruplicates). Statistically significant (p<0.05) differences relative to solvent are marked with asterisk.

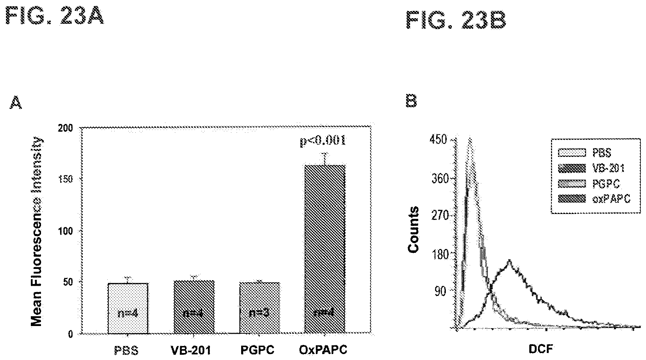

Figure 23A is a bar graph that depicts the effect of VB-201 on reactive oxygen species (ROS) formation in human monocytes. Human monocytes (CD14⁺) isolated from blood of healthy donors were incubated overnight with PBS, VB-201 (10 µg/ml), PGPC (10 µg/ml), or oxidized PAPC (OxPAPC) (100 µg/ml). Detection of ROS was performed through FACS quantification of the oxidation product dichlorodihydrofluorescein (DCF). Data are mean±SD from 3-4 experiments performed in duplicates. Figure 23B is a histogram from a representative experiment showing DCF levels in treated cells.

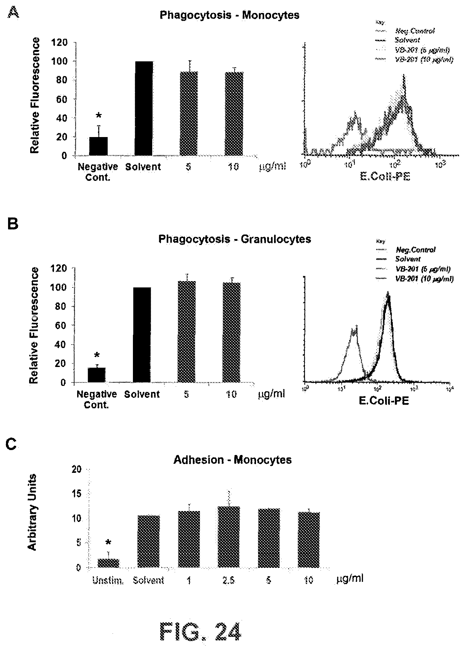

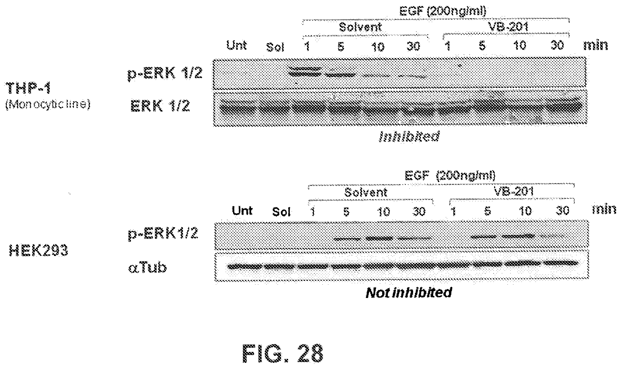

Figure 24A-C are bar graphs that depict the effects of VB-201 on monocyte phagocytosis and adhesion to endothelial cells. In FIG. 24A, the capacity of monocytes to phagocytose PE-labeled E.coli particles was tested at 37°C in the presence of VB-201 (5 or 10 µg/ml) or solvent control. For detection of non-specific background, cells were incubated with PE-particles at 4°C (negative control, no phagocytosis). PE intensity was quantified by FACS. Data are normalized to the solvent and represent mean ± SD (n=2). A representative histogram is presented. Fig. 28B is a bar graph that depicts a granulocyte phagocytosis assay. In FIG. 28C, calcein AM-labeled monocytes were pretreated with solvent control or with escalating doses of VB-201 for 30 min, prior to 15 min incubation with TNFα-stimulated HUVEC. Following washes, adherent monocytes were quantified by Fluorometer. Adherence of untreated monocytes to un-stimulated HUVEC is shown as reference, demonstrating the TNFα-mediated induction of adhesion molecules. Data are mean ± SD (n=2). *, p<0.05 vs solvent.

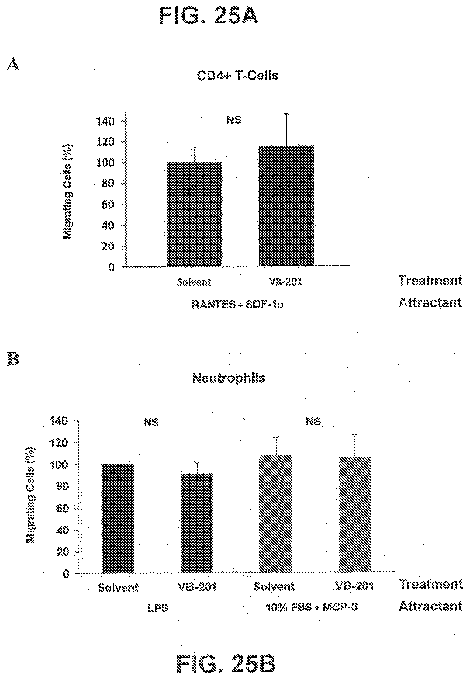

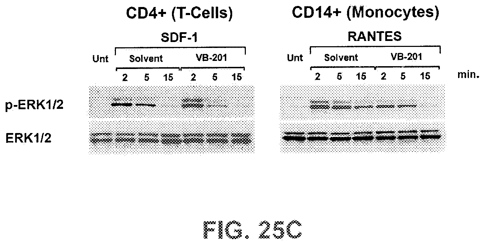

FIGS. 25A and 25B are bar graphs that depict the effect of VB-201 on migration of CD4⁺ T-cells and neutrophils. In FIG. 25A, human CD4⁺ cells isolated from healthy donors were pre-treated for 30 min with solvent or VB-201 (5 µg/ml) and then subjected to chemotaxis assay using RANTES (100ng/ml) and SDF-1α (50ng/ml) as attractants. The number of cells migrating to the lower compartment was determined by FACS, and normalized to the solvent control. Data are mean±SD from 3 different experiments performed in triplicates. In FIG. 25B, human neutrophils isolated from healthy donors were pre-treated for 30 min with solvent or VB-201 (5 µg/ml) and then subjected to chemotaxis towards 2% FBS/RPMI-1640 medium supplemented with 100ng/ml LPS (black bars), or alternatively, towards 10% FBS/RPMI-1640 supplemented with 100ng/ml MCP-3 (grey bars). Data are mean±SD from 4 different donors tested in triplicates. NS - No statistical difference can be found between VB-201 treatment and the solvent control. FIG. 25C presents images of Western Blots of phosphorylated ERK1/2 (p-ERK1/2) in human primary CD4+ T cells 2, 5 or 15 minutes after treatment with 50 ng/ml SDF-1α (left) or CD14+ monocytes 2, 5 or 15 minutes after treatment with 50 ng/ml RANTES (right), following pretreatment with solvent or VB-201, or with no pretreatment (Unt); T-cells and monocytes were isolated from the same donor (total ERK1/2 serves as a loading control);

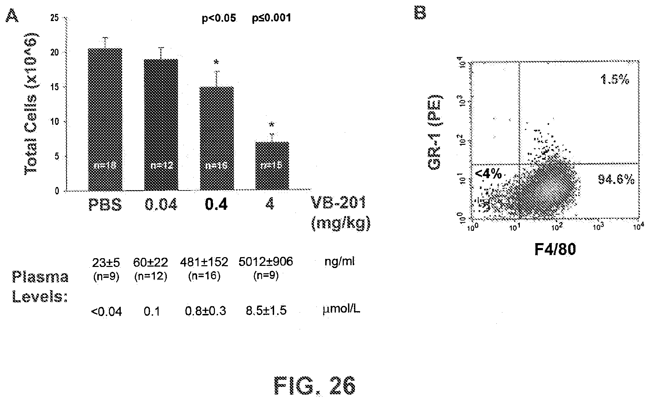

FIG. 26A is a bar graph depicting the effect of VB-201 on monocyte chemotaxis *in vivo.* C57B6J WT mice were orally administered with escalating doses of VB-201 as indicated. After 5 days of administration, thioglycollate was injected to the peritoneal cavity of the mice to induce monocyte migration. Four days later, mice were sacrificed and migrating cells were collected from the peritoneum and counted by hemocytometer. Data are mean ± SE, collected from 3-4 independent experiments. Plasma concentrations of VB-201 are shown for each group. FIG. 26B is a FACS analysis. To characterize their identity, migrating cells were stained with the macrophage marker F4/80 and the neutrophils marker GR-1. FACS analysis from a representative animal demonstrates that ∼95% of the cells isolated from the peritoneum are macrophages.

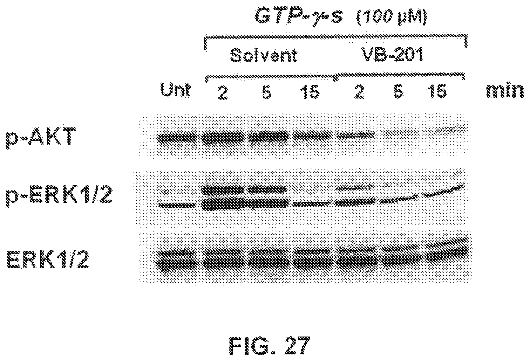

FIG. 27 is a Western blot that depicts the effect of VB-201 on GTP-γ-S-induced intracellular activation of the AKT and ERK pathways in the monocytic THP-1 cell line.
FIG. 28 is a Western blot that depicts the effect of VB-201 on EGF-induced activation of ERK in THP-1, but not HEK293 cells.

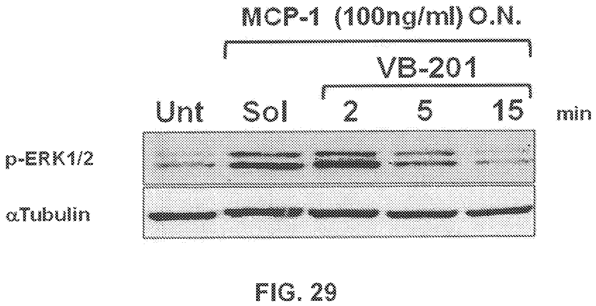

FIG. 29 is a Western blot that depicts the effect of VB-201 on ERK1/2 phosphorylation in human monocytes which were pre-stimulated with MCP-1 overnight.

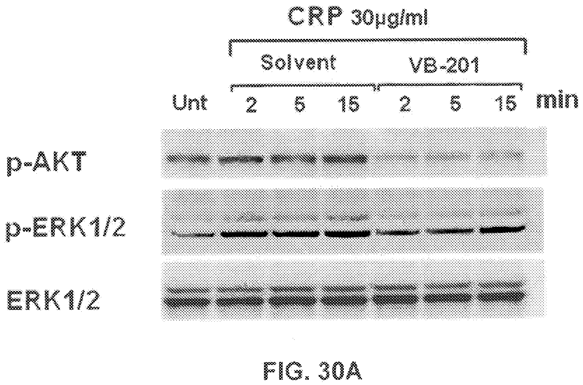

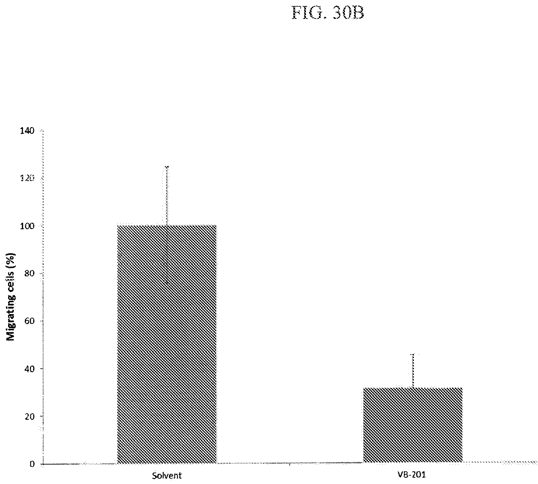

FIG. 30A is a Western blot that depicts the effect of VB-201 on CRP-induced activation of the AKT and ERK pathways in the monocytic THP-1 cell line. FIG. 30B is a bar graph that depicts the inhibition of C-reactive protein-induced chemotaxis of human monocytes.

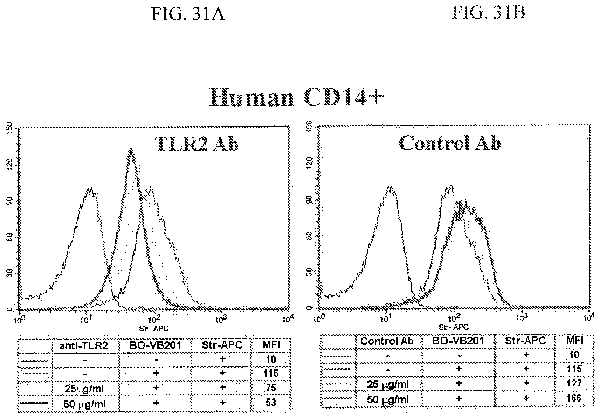

FIGS. 31A and 31B are FACS analyses that depict the binding of VB-201 to TLR2 expressed on the cell surface.

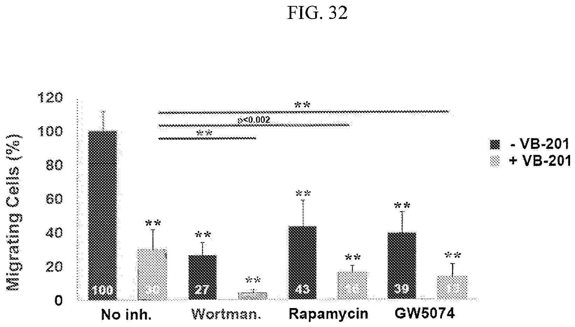

FIG. 32 is a bar graph that depicts the inhibitory effect of Wortmannin, Rapamycin and the RAF inhibitor GW5074 on monocyte chemotaxis.

### DETAILED DESCRIPTION OF THE INVENTION

The present invention relates to the compound 1-hexadecyl-2-(4'-carboxybutyl)-glycerol-3-phosphocholine for use in the treatment of an inflammatory disease or disorder which is non-alcoholic steatohepatitis (NASH).

The principles and operation of the present invention may be better understood with reference to the figures and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways in accordance with the claims. Also, it is to be understood that the phraseology and terminology employed herein is for the purpose of description and should not be regarded as limiting.

The oxidized phospholipid to be used in accordance with the present invention is 1-hexadecyl-2-(4'-carboxybutyl)-glycerol-3-phosphocholine. VB-201 is 1-hexadecyl-2-(4'-carboxy)butyl-*sn*-glycero-3-phosphocholine, also referred to as (*R*)-1-hexadecyl-2-(4'-carboxy)butyl-glycero-3-phosphocholine. The term VB-201 includes pharmaceutically acceptable salts or solvates (e.g., hydrates) thereof. In one embodiment, the activity that is inhibited is TLR2 activity.

In another embodiment, the activity that is inhibited is CD14 activity.

In another embodiment, the activity that is inhibited is monocyte chemotaxis activity.

In another embodiment, TLR2 activity and CD14 activity are inhibited.

In another embodiment, TLR2 activity and monocyte chemotaxis activity are inhibited.

In another embodiment, CD14 activity and monocyte chemotaxis activity are inhibited.

In another embodiment, TLR2 activity, CD14 activity and monocyte chemotaxis activity are inhibited.

VB-201 (also referred to herein and in the art as CI-201), an oxidized lipid derivative, has shown considerable promise as a therapeutically active agent in various *in vitro* models and *in vivo* animal models of inflammatory conditions. Additional oxidized lipids have also shown evidence of anti-inflammatory activity.

In an attempt to improve treatment of inflammatory diseases and disorders, the present inventors have studied in detail the effects and mechanism of action of VB-201. The protocols of these assays are described in detail in the Examples section that follows. Based on the data obtained in the studies conducted, the present inventors have developed improved treatment regimens utilizing VB-201.

In particular, the present inventors have conceived that a variety of agents may be used to provide the therapeutic mechanism of action exhibited by VB-201.

Referring now to the drawings and tables, Figures 1A and 1B show that VB-201 associates more with monocytes and dendritic cells than with T-cells and B-cells.

Figures 2A-5 show that VB-201 inhibits signaling by TLR2 (toll-like receptor 2) heterodimer activation and by TLR4 (toll-like receptor 4) activation, but not signaling by activation of TLR5, TLR7, TLR9 or IL-1β (interleukin-1β) receptor.

Figures 6A and 6B show that VB-201 and biotinylated VB-201 inhibit TLR4 signaling, but that VB-207 (1-octyl-2-(4'-carboxy)butyl-sn-glycero-3-phosphocholine) and biotinylated VB-207 do not. Figures 7A and 7B show that VB-201 binds to CD14 and TLR2, but not to TLR4, MyD88, or CD36, and that VB-201 does not bind to any of the aforementioned proteins. Figure 8 further shows that VB-201 binds to CD14 and TLR2. Figures 10A-12D further show that VB-201 binds to CD14. Figure 9 shows that VB-201 inhibits binding of lipopolysaccharide to CD14.

These results indicate that VB-201 and related compounds inhibit TLR2 activity by binding to TLR2, and further inhibit TLR4 and CD14 activity by binding to CD14, and thereby preventing binding of lipopolysaccharide to CD14. As CD14 transports lipopolysaccharide to TLR4, the inhibition of CD14 is believed to reduce binding of lipopolysaccharide to TLR4. The results further indicate that dendritic cells and monocytes are affected more than T-cells and B-cells.

Figure 26A shows that the oxidized lipids VB-201 and PGPC inhibit monocyte chemotaxis, whereas non-oxidized lipids and partially oxidized lipids do not.

Figures 19A and 19B show that reduction by VB-201 of aortic lesion formation is associated with a reduction in the number of macrophages at the lesion site.

Figures 25A and 25B show that VB-201 does not inhibit T-cell or neutrophil chemotaxis.

These results indicate that inhibition of monocyte chemotaxis plays a role in the therapeutic benefit of VB-201 and related compounds.

Figures 14,15 and 24 show that VB-201 does not inhibit monocyte phagocytosis, calcium signaling, cAMP signaling, or act as a chemoattractant or cytokine receptor antagonist.

Figures 16A and 16B show that VB-201 inhibits AKT, MEK and ERK activation by a variety of chemokines. Figures 17, 18 and 32 show that inhibitors of the MEK-ERK signaling pathway or PI3K/AKT/mTOR inhibit monocyte chemotaxis, and that a combination of VB-201 and a MEK-ERK pathway inhibitor, or with a PI3K/mTOR pathway inhibitor is particularly effective at inhibiting chemotaxis.

These results indicate that VB-201 specifically inhibits chemotaxis in monocytes, by inhibiting an intracellular process downstream of chemokine receptors, including the MEK-ERK pathway, and not by inhibiting monocyte activity in general.

Figure 20 depicts the signaling pathway associated with CD14 activity, including TLR4.

Figure 21 depicts the signaling pathways associated with various toll-like receptors, including TLR2, and TLR4 (which is associated with CD14 activity).

Taken together, the above results indicate that compounds which exhibit activities described hereinabove, namely inhibition of CD14 activity and/or a signaling pathway associated with CD14 activity, inhibition of TLR2 activity and/or a signaling pathway associated with TLR2 activity, and/or inhibition of monocyte chemotaxis, may provide the therapeutic benefits associated with VB-201 and related compounds, either alone or in combination with VB-201 or a related compound.

The above results further indicate that compounds which exhibit, or a combination of compounds which together exhibit, inhibition of CD14 activity and/or a signaling pathway associated with CD14 activity and inhibition of TLR2 activity and/or a signaling pathway associated with TLR2 activity may provide therapeutic benefits similar to those exhibited by VB-201 or a related compound.

The above results further indicate that compounds which exhibit, or a combination of compounds which together exhibit, inhibition of CD14 activity and/or a signaling pathway associated with CD14 activity; inhibition of TLR2 activity and/or a signaling pathway associated with TLR2 activity; and inhibition of monocyte chemotaxis, may provide therapeutic benefits similar to those exhibited by VB-201 or a related compound.

The above results further indicate that compounds which exhibit, or a combination of compounds which together exhibit, inhibition of CD14 activity and/or a signaling pathway associated with CD14 activity; and inhibition of monocyte chemotaxis, may provide therapeutic benefits similar to those exhibited by VB-201 or a related compound.

The above results further indicate that compounds which exhibit, or a combination of compounds which together exhibit, inhibition of TLR2 activity and/or a signaling pathway associated with TLR2 activity; and inhibition of monocyte chemotaxis, may provide therapeutic benefits similar to those exhibited by VB-201 or a related compound.

The above results further indicate that VB-201 or a related compound can be beneficially used in combination with any of the above-mentioned compounds or combination of compounds, for providing an improved therapeutic effect.

Hence, according to one aspect of embodiments disclosed herein, there is provided an agent for use in a method of treating an inflammatory disease or disorder, the method comprising administering to a subject in need thereof a therapeutically effective amount of at least one agent (e.g., one agent, two agents, or three agents). The at least one agent (e.g., the aforementioned one agent, the two agents or three agents) is capable of exhibiting at least two activities selected from the group consisting of:
a) inhibiting CD14 activity and/or a signaling pathway associated with CD14 activity,
b) inhibiting TLR2 activity and/or a signaling pathway associated with TLR2 activity, and
c) inhibiting monocyte chemotaxis.

For brevity, the abovementioned group is also referred to herein as the "three activities".

According to optional embodiments disclosed herein, the at least one agent comprises at least two agents (e.g., two agents, three agents).

It is to be understood that when more than one agent is used, it is the combination of the agents which is capable of exhibiting at least two of the three activities, although optionally, none of the agents exhibit more than one of the three activities by themselves.

Embodiments referring to at least two agents are considered herein as embodiments referring to a combination of agents or a combination of compounds.

In some embodiments, at least one agent exhibits at least two of the three activities described herein, and optionally all three of the activities described herein. Such an agent may be used alone (as it exhibits at least two of the three activities described herein, in accordance with requirements of embodiments of the invention), or in combination with at least one additional agent.

The use of an agent that exhibits at least two of the three activities described herein is optionally advantageous in that it allows for the use of fewer agents. For example, a single agent may be used instead of multiple agents in order to exhibit two or three of the activities, and/or two agents may be used instead of three agents in order to exhibit all three activities.

The use of fewer agents allows facilitated co-administration and/or co-formulation of the agents.

In some embodiments disclosed herein, at least one agent exhibits only one of the three activities described herein, and is used in combination with at least one other agent (e.g., one other agent), which exhibits at least one of the remaining two activities described herein, such that the combination of at least two agents exhibits at least two of the three activities described herein.

The use of an agent that exhibits only one of the three activities described herein is optionally advantageous in that it allows for more selectivity, with fewer undesirable activities. For example, two of the three activities described herein may be selectively obtained using two agents which each selectively exhibit one of the activities, and/or all three activities described herein may be selectively obtained using three agents which each selectively exhibit one of the activities.

According to optional embodiments, the at least one agent is capable of exhibiting an activity of inhibiting CD14 activity and/or a signaling pathway associated with CD14 activity (e.g., as described herein), as well as an activity of inhibiting TLR2 activity and/or a signaling pathway associated with TLR2 activity (e.g., as described herein).

Optionally, the at least one agent comprises at least one agent (optionally, one agent) which exhibits both of the aforementioned activities.

As disclosed herein, alternatively or additionally, the at least one agent comprises at least one agent (optionally, one agent) which exhibits an activity of inhibiting CD14 activity and/or a signaling pathway associated with CD14 activity (and optionally, this agent does not exhibit any of the other two activities described herein), and at least one other agent (optionally, one agent) which exhibits an activity of inhibiting TLR2 activity and/or a signaling pathway associated with TLR2 activity (and optionally, this agent does not exhibit any of the other two activities described herein).

According to optional embodiments, the at least one agent is capable of exhibiting an activity of inhibiting CD14 activity and/or a signaling pathway associated with CD14 activity (e.g., as described herein), as well as an activity of inhibiting monocyte chemotaxis (e.g., as described herein).

Optionally, the at least one agent comprises at least one agent (optionally, one agent) which exhibits both of the aforementioned activities.

As disclosed herein, alternatively or additionally, the at least one agent comprises at least one agent (optionally, one agent) which exhibits an activity of inhibiting CD14 activity and/or a signaling pathway associated with CD14 activity (and optionally, this agent does not exhibit any of the other two activities described herein), and at least one other agent (optionally, one agent) which exhibits an activity of inhibiting monocyte chemotaxis (and optionally, this agent does not exhibit any of the other two activities described herein).

According to optional embodiments, the at least one agent is capable of exhibiting an activity of inhibiting monocyte chemotaxis (e.g., as described herein), as well as an activity of inhibiting TLR2 activity and/or a signaling pathway associated with TLR2 activity (e.g., as described herein).

Optionally, the at least one agent comprises at least one agent (optionally, one agent) which exhibits both of the aforementioned activities.

As disclosed herein, alternatively or additionally, the at least one agent comprises at least one agent (optionally, one agent) which exhibits an activity of inhibiting monocyte chemotaxis (and optionally, this agent does not exhibit any of the other two activities described herein), and at least one other agent (optionally, one agent) which exhibits an activity of inhibiting TLR2 activity and/or a signaling pathway associated with TLR2 activity (and optionally, this agent does not exhibit any of the other two activities described herein).

In some embodiments, the at least one agent is capable of exhibiting all three activities described herein, namely, inhibiting CD14 activity and/or a signaling pathway associated with CD14 activity, inhibiting TLR2 activity and/or a signaling pathway associated with TLR2 activity, and inhibiting monocyte chemotaxis.

Optionally, the at least one agent comprises at least one agent (optionally, one agent) which exhibits all three of the aforementioned activities.

Alternatively or additionally, as disclosed herein, the at least one agent comprises at least one agent (optionally, one agent) which exhibits one of the three activities described herein (inhibiting monocyte chemotaxis), and at least one other agent (optionally, one agent) which exhibits the other two activities described herein.

Alternatively or additionally, as disclosed herein, the at least one agent comprises at least one agent (optionally, one agent) which exhibits an activity of inhibiting CD14 activity and/or a signaling pathway associated with CD14 activity (and optionally, this agent does not exhibit any of the other two activities described herein), at least one other agent (optionally, one agent) which exhibits an activity of inhibiting TLR2 activity and/or a signaling pathway associated with TLR2 activity (and optionally, this agent does not exhibit any of the other two activities described herein), and at least one other agent (optionally, one agent) which exhibits an activity of inhibiting monocyte chemotaxis (and optionally, this agent does not exhibit any of the other two activities described herein).

Herein disclosed examples of suitable agents which exhibit at least one of the activities described herein include, without limitation, a TLR2 (toll-like receptor 2) inhibitor, a TLR1 (toll-like receptor 1) inhibitor, a CD14 inhibitor, a TLR4 inhibitor, an MD-2 (lymphocyte antigen 96) inhibitor, an LBP (lipopolysaccharide-binding protein) inhibitor, a TLR6 (toll-like receptor 6) inhibitor, a MyD88 inhibitor, a TRAM (TRIF-related adaptor molecule) inhibitor, a TRIF (TIR domain-containing .adapter-inducing interferon-β) inhibitor, a TIRAP (TIR domain-containing adaptor protein) inhibitor, an IRAK1 (IL-1 receptor-associated kinase 1) inhibitor, an IRAK2 (IL-1 receptor-associated kinase 2) inhibitor, an IRAK4 (IL-1 receptor-associated kinase 4) inhibitor, a TRAF6 (TNF receptor-associated factor 6) inhibitor, a TRAF3 (TNF receptor-associated factor 3) inhibitor, a RIP1 (receptor (TNFRSF)-interacting serine-threonine kinase 1) inhibitor, a TAB1 (TGF-β activated kinase 1) inhibitor, a TAB2 (TGF-β activated kinase 2) inhibitor, a TAK1 (also known as "MAP3K7") inhibitor, an IKK (IκB kinase) inhibitor (e.g., an inhibitor of IKK-α, IKK-β, IKK-γ and/or IKK-ε), a RAS inhibitor, a RAF inhibitor, an MKK3 (MAP kinase kinase 3) inhibitor, an MKK6 (MAP kinase kinase 6) inhibitor, an MLK (mixed lineage kinase) inhibitor, a MKK4 (MAP kinase kinase 4) inhibitor, a MKK7 (MAP kinase kinase 7) inhibitor, a MEK1 (MAP kinase kinase 1) inhibitor, a MEK2 (MAP kinase kinase 2) inhibitor, a p38 (p38 MAP kinase) inhibitor, a JNK inhibitor, an ERK1/2 inhibitor (i.e., an inhibitor of ERK1 and/or ERK2), an NFκB inhibitor (which encompasses IκB activators), an AP-1 (activator protein 1) inhibitor, a CREB inhibitor, an IRF3 (interferon regulatory factor 3) inhibitor, an IRF5 (interferon regulatory factor 5) inhibitor, a BTK (Bruton's tyrosine kinase) inhibitor, a JAK2 (janus kinase 2) inhibitor, a Syk (spleen tyrosine kinase) inhibitor, a CD36 inhibitor, a scavenger receptor-A inhibitor, a Rac GTPase inhibitor, PI3K (phosphatidyl inositol 3-kinase) inhibitor, an AKT (protein kinase B) inhibitor, an mTORC1 (mammalian target of rapamycin complex 1) inhibitor, an mTORC2 (mammalian target of rapamycin complex 2) inhibitor, a PDK1 (phosphoinositide dependent protein kinase-1) inhibitor, a TNFAIP3 (TNF-α-induced protein 3) activator, a TANK (TRAF family member-associated NFκB activator) activator, an SHP-1 (Src homology region 2 domain-containing phosphatase-1) activator, a TOLLIP (toll interacting protein) activator, and/or an IRAK3 (IL-1 receptor-associated kinase 3) activator.

Herein, an "inhibitor" of a given biological moiety (referred to herein as a "target", e.g., a protein) refers to a compound which downregulates an activity of the biological moiety. The inhibition exhibited by an inhibitor, as defined herein, shows at least some selectivity, i.e., the inhibition is not a result of an interaction with a wide variety of compounds (e.g., proteins) in general.

Downregulation of activity of a protein target can be effected on the genomic and/or the transcript level using a variety of molecules which interfere with transcription and/or translation [e.g., RNA silencing agents (e.g., antisense, siRNA, shRNA, microRNA), Ribozyme and DNAzyme], or on the protein level using e.g., antagonists, enzymes that cleave the polypeptide, small molecules that interfere with the protein's activity (e.g., competitive ligands) and the like.

Following is a list of agents capable of downregulating expression level and/or activity of a target.

One example, of an agent capable of downregulating a target is an antibody or antibody fragment capable of specifically binding the target. Preferably, the antibody specifically binds at least one epitope of a target. As used herein, the term "epitope" refers to any antigenic determinant on an antigen to which the paratope of an antibody binds.

Epitopic determinants usually consist of chemically active surface groupings of molecules such as amino acids or carbohydrate side chains and usually have specific three dimensional structural characteristics, as well as specific charge characteristics.

The term "antibody" as used herein includes intact molecules as well as functional fragments thereof, such as Fab, F(ab')2, and Fv that are capable of binding to macrophages. These functional antibody fragments are defined as follows: (1) Fab, the fragment which contains a monovalent antigen-binding fragment of an antibody molecule, can be produced by digestion of whole antibody with the enzyme papain to yield an intact light chain and a portion of one heavy chain; (2) Fab', the fragment of an antibody molecule that can be obtained by treating whole antibody with pepsin, followed by reduction, to yield an intact light chain and a portion of the heavy chain; two Fab' fragments are obtained per antibody molecule; (3) (Fab')2, the fragment of the antibody that can be obtained by treating whole antibody with the enzyme pepsin without subsequent reduction; F(ab')2 is a dimer of two Fab' fragments held together by two disulfide bonds; (4) Fv, defined as a genetically engineered fragment containing the variable region of the light chain and the variable region of the heavy chain expressed as two chains; and (5) Single chain antibody ("SCA"), a genetically engineered molecule containing the variable region of the light chain and the variable region of the heavy chain, linked by a suitable polypeptide linker as a genetically fused single chain molecule.

Downregulation of a target can be also achieved by RNA silencing. As used herein, the phrase "RNA silencing" refers to a group of regulatory mechanisms [e.g. RNA interference (RNAi), transcriptional gene silencing (TGS), post-transcriptional gene silencing (PTGS), quelling, co-suppression, and translational repression] mediated by RNA molecules which result in the inhibition or "silencing" of the expression of a corresponding protein-coding gene. RNA silencing has been observed in many types of organisms, including plants, animals, and fungi.

As used herein, the term "RNA silencing agent" refers to an RNA which is capable of specifically inhibiting or "silencing" the expression of a target gene. In certain embodiments, the RNA silencing agent is capable of preventing complete processing (e.g, the full translation and/or expression) of an mRNA molecule through a post-transcriptional silencing mechanism. RNA silencing agents include noncoding RNA molecules, for example RNA duplexes comprising paired strands, as well as precursor RNAs from which such small non-coding RNAs can be generated. Exemplary RNA silencing agents include dsRNAs such as siRNAs, miRNAs and shRNAs. In one embodiment, the RNA silencing agent is capable of inducing RNA interference. In another embodiment, the RNA silencing agent is capable of mediating translational repression.

RNA interference refers to the process of sequence-specific post-transcriptional gene silencing in animals mediated by short interfering RNAs (siRNAs). The corresponding process in plants is commonly referred to as post-transcriptional gene silencing or RNA silencing and is also referred to as quelling in fungi. The process of post-transcriptional gene silencing is thought to be an evolutionarily-conserved cellular defense mechanism used to prevent the expression of foreign genes and is commonly shared by diverse flora and phyla. Such protection from foreign gene expression may have evolved in response to the production of double-stranded RNAs (dsRNAs) derived from viral infection or from the random integration of transposon elements into a host genome via a cellular response that specifically destroys homologous single-stranded RNA or viral genomic RNA.

Some embodiments disclosed herein contemplate use of dsRNA to downregulate protein expression from mRNA.

The term "siRNA" refers to small inhibitory RNA duplexes (generally between 18-30 basepairs) that induce the RNA interference (RNAi) pathway. Typically, siRNAs are chemically synthesized as 21mers with a central 19 bp duplex region and symmetric 2-base 3'-overhangs on the termini, although it has been recently described that chemically synthesized RNA duplexes of 25-30 base length can have as much as a 100-fold increase in potency compared with 21mers at the same location. The observed increased potency obtained using longer RNAs in triggering RNAi is theorized to result from providing Dicer with a substrate (27mer) instead of a product (21mer) and that this improves the rate or efficiency of entry of the siRNA duplex into RISC.

The strands of a double-stranded interfering RNA (e.g., an siRNA) may be connected to form a hairpin or stem-loop structure (e.g., an shRNA). Thus, as mentioned the RNA silencing agent may also be a short hairpin RNA (shRNA).

The term "shRNA", as used herein, refers to an RNA agent having a stem-loop structure, comprising a first and second region of complementary sequence, the degree of complementarity and orientation of the regions being sufficient such that base pairing occurs between the regions, the first and second regions being joined by a loop region, the loop resulting from a lack of base pairing between nucleotides (or nucleotide analogs) within the loop region. The number of nucleotides in the loop is a number between and including 3 to 23, or 5 to 15, or 7 to 13, or 4 to 9, or 9 to 11. Some of the nucleotides in the loop can be involved in base-pair interactions with other nucleotides in the loop. Examples of oligonucleotide sequences that can be used to form the loop include 5'-UUCAAGAGA-3' (Brummelkamp, T. R. et al. (2002) Science 296: 550) and 5'-UUUGUGUAG-3' (Castanotto, D. et al. (2002) RNA 8:1454). It will be recognized by one of skill in the art that the resulting single chain oligonucleotide forms a stem-loop or hairpin structure comprising a double-stranded region capable of interacting with the RNAi machinery.

It will be appreciated that the RNA silencing agent of some embodiments disclosed herein need not be limited to those molecules containing only RNA, but further encompasses chemically-modified nucleotides and non-nucleotides.

In some embodiments, the RNA silencing agent provided herein can be functionally associated with a cell-penetrating peptide. As used herein, a "cell-penetrating peptide" is a peptide that comprises a short (about 12-30 residues) amino acid sequence or functional motif that confers the energy-independent (i.e., non-endocytotic) translocation properties associated with transport of the membrane-permeable complex across the plasma and/or nuclear membranes of a cell.

According to another embodiment the RNA silencing agent may be a miRNA or a mimic thereof.

The term "microRNA", "miRNA", and "miR" are synonymous and refer to a collection of non-coding single-stranded RNA molecules of about 19-28 nucleotides in length, which regulate gene expression. miRNAs are found in a wide range of organisms (viruses.fwdarw.humans) and have been shown to play a role in development, homeostasis, and disease etiology.

The term "microRNA mimic" refers to synthetic non-coding RNAs that are capable of entering the RNAi pathway and regulating gene expression. miRNA mimics imitate the function of endogenous microRNAs (miRNAs) and can be designed as mature, double stranded molecules or mimic precursors (e.g., or pre-miRNAs). miRNA mimics can be comprised of modified or unmodified RNA, DNA, RNA-DNA hybrids, or alternative nucleic acid chemistries (e.g., LNAs or 2'-O,4'-C-ethylene-bridged nucleic acids (ENA)). For mature, double stranded miRNA mimics, the length of the duplex region can vary between 13-33, 18-24 or 21-23 nucleotides. The miRNA may also comprise a total of at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 nucleotides. The sequence of the miRNA may be the first 13-33 nucleotides of the pre-miRNA. The sequence of the miRNA may also be the last 13-33 nucleotides of the pre-miRNA.

Another agent capable of downregulating a target is a DNAzyme molecule capable of specifically cleaving an mRNA transcript or DNA sequence of the target. DNAzymes are single-stranded polynucleotides which are capable of cleaving both single and double stranded target sequences (Breaker, R.R. and Joyce, G. Chemistry and Biology 1995;2;655; Santoro, S.W. & Joyce, G.F. Proc. Natl, Acad. Sci. USA 1997;943:4262) A general model (the "10-23" model) for the DNAzyme has been proposed. "10-23" DNAzymes have a catalytic domain of 15 deoxyribonucleotides, flanked by two substrate-recognition domains of seven to nine deoxyribonucleotides each. This type of DNAzyme can effectively cleave its substrate RNA at purine:pyrimidine junctions (Santoro, S.W. & Joyce, G.F. Proc. Natl, Acad. Sci. USA 199; for rev of DNAzymes see Khachigian, LM [Curr Opin Mol Ther 4:119-21 (2002)].

Downregulation of a target can also be effected by using an antisense polynucleotide capable of specifically hybridizing with an mRNA transcript encoding the target.

Another agent capable of downregulating a target is a ribozyme molecule capable of specifically cleaving an mRNA transcript encoding a target. Ribozymes are being increasingly used for the sequence-specific inhibition of gene expression by the cleavage of mRNAs encoding proteins of interest [Welch et al., Curr Opin Biotechnol. 9:486-96 (1998)].

Another agent capable of downregulating a target would be any molecule which binds to and/or cleaves the target. Such molecules can be antagonists of the target, or inhibitory peptides of the target.

It will be appreciated that a non-functional analogue of at least a catalytic or binding portion of a target can be also used as an agent which downregulates the target.

Another agent which can be used to downregulate a target is a molecule which prevents target activation or substrate binding.

In some embodiments, an inhibitor of a given protein inhibits the protein by binding to the protein, by binding to a compound which binds to the protein (e.g., a substrate, a regulatory protein), and/or by binding to an oligonucleotide (e.g., mRNA) encoding the protein.

In some embodiments, the inhibitor is a small molecule (e.g., characterized by a molecular weight of less than 800 Da).

. In some embodiments, an inhibitor of a given protein inhibits the protein by binding to the protein and/or to an oligonucleotide (e.g., mRNA) encoding the protein.

Herein, an "activator" of a given compound (e.g., protein) refers to a compound which increases an activity of the compound by any means, including increasing a total amount of the compound, for example, by enhancing expression of a protein and/or by inhibiting destruction of a protein (e.g., by cellular proteases), as well as increasing an amount of an activated state of the compound, for example, by enhancing activation of a protein (e.g., enhancing phosphorylation) and/or inhibiting deactivation of a protein (e.g., inhibiting dephosphorylation), and/or by inhibiting any type of downregulation of a compound (e.g., a protein) described herein. The activation exhibited by an activator, as defined herein, shows at least some selectivity, i.e., the activation is not a result of an interaction with a wide variety of compounds (e.g., proteins) in general.

In some embodiments, an activator of a given protein activates the protein by binding to the protein, by binding to a compound which binds to the protein (e.g., a substrate, a regulatory protein), and/or by binding to an oligonucleotide (e.g., mRNA) encoding the protein.

In some embodiments, an activator of a given protein activates the protein by binding to the protein and/or to an oligonucleotide (e.g., mRNA) encoding the protein.

An agent which exhibits the activity of inhibiting CD14 activity and/or a signaling pathway associated with CD14 activity, as described herein, may optionally be a CD14 inhibitor (which exhibits an activity of inhibiting CD14), and/or an agent which inhibits the signaling pathway associated with CD14 (as opposed to inhibiting CD14 directly), including, without limitation, a TLR4 inhibitor, an MD-2 inhibitor, an LBP inhibitor, a TLR6 inhibitor, a MyD88 inhibitor, a TRAM inhibitor, a TRIF inhibitor, a TIRAP inhibitor, an IRAK1 inhibitor, an IRAK2 inhibitor, an IRAK4 inhibitor, a TRAF6 inhibitor, a TRAF3 inhibitor, a RIP1 inhibitor, a TAB1 inhibitor, a TAB2 inhibitor, a TAK1 inhibitor, an IKK inhibitor, a RAS inhibitor, a RAF inhibitor, an MKK3 inhibitor, an MKK6 inhibitor, an MLK inhibitor, a MKK4 inhibitor, a MKK7 inhibitor, a MEK1 inhibitor, a MEK2 inhibitor, a p38 inhibitor, a JNK inhibitor, an ERK1/2 inhibitor, an NFκB inhibitor, an AP-1 inhibitor, a CREB inhibitor, an IRF3 inhibitor, an IRF5 inhibitor, a BTK inhibitor, a JAK2 inhibitor, a Syk inhibitor, a CD36 inhibitor, a scavenger receptor-A inhibitor, a Rac inhibitor, PI3K inhibitor, an AKT inhibitor, an mTORC1 inhibitor, an mTORC2 inhibitor, a PDK1 inhibitor, a TNFAIP3 activator, a TANK activator, an SHP-1 activator, a TOLLIP activator, and an IRAK3 activator.

The protein MyD88 is involved in many signaling pathways (e.g., signaling pathways of many TLRs). Consequently, agents that affect MyD88 or proteins downstream from MyD88 will be relatively non-specific for the CD14-associated pathway.

Thus, in some embodiments, an agent which exhibits the activity of inhibiting CD14 activity and/or a signaling pathway associated with CD14 activity inhibits the CD14 activity (or signaling pathway) upstream of MyD88 (e.g., as depicted in Figure 24 and/or Figure 25).

Examples of such agents, which exhibit only one of the three activities described herein, include, without limitation, a CD14 inhibitor, a TLR4 inhibitor, an MD-2 inhibitor, an LBP inhibitor, a TRAM inhibitor, and a TRIF inhibitor.

In some embodiments, an agent which exhibits the activity of inhibiting CD14 activity and/or a signaling pathway associated with CD14 activity inhibits the CD14 activity (or signaling pathway) by inhibiting activity of TLR4, a protein which complexes with TLR4 (e.g., MD-2), or a protein upstream of TLR4 (e.g., CD14 and/or LBP). Without being bound by any particular theory, such agents are expected to be relatively selective and potent inhibitors of TLR4 signaling. In exemplary embodiments, the agent inhibits CD14 activity (e.g., by binding to CD14).

An agent which exhibits the activity of inhibiting TLR2 activity and/or a signaling pathway associated with TLR2 activity, as described herein, may optionally be a TLR2 inhibitor (which exhibits an activity of inhibiting TLR2), an inhibitor of a protein (e.g., TLR1 and/or TLR6) capable of forming a heterodimer with TLR2 (thereby at least partially inhibiting TLR2 activity), and/or an agent which inhibits the signaling pathway associated with TLR2 (as opposed to inhibiting TLR2 or a heterodimer thereof directly), including, without limitation, a MyD88 inhibitor, a TIRAP inhibitor, an IRAK1 inhibitor, an IRAK2 inhibitor, an IRAK4 inhibitor, a TRAF6 inhibitor, a TAB1 inhibitor, a TAB2 inhibitor, a TAK1 inhibitor, an IKK inhibitor, a RAS inhibitor, a RAF inhibitor, an MKK3 inhibitor, an MKK6 inhibitor, an MLK inhibitor, a MKK4 inhibitor, a MKK7 inhibitor, a MEK1 inhibitor, a MEK2 inhibitor, a p38 inhibitor, a JNK inhibitor, an ERK1/2 inhibitor, an NFκB inhibitor, an AP-1 inhibitor, a CREB inhibitor, an IRF3 inhibitor, an IRF5 inhibitor, a BTK inhibitor, a JAK2 inhibitor, a Syk inhibitor, a Rac inhibitor, PI3K inhibitor, an AKT inhibitor, an mTORC1 inhibitor, an mTORC2 inhibitor, a PDK1 inhibitor, a TNFAIP3 activator, a TANK activator, an SHP-1 activator, a TOLLIP activator, and/or an IRAK3 activator.

In some embodiments, an agent which exhibits the activity of inhibiting TLR2 activity and/or a signaling pathway associated with TLR2 activity is an agent which inhibits the TLR2 activity by inhibiting activity of TLR2, and/or a protein which complexes with TLR2, for example, a TLR2 inhibitor, a TLR1 inhibitor, and/or a TLR6 inhibitor. In exemplary embodiments, the agent inhibits TLR2 activity (e.g., by binding to TLR2).

In some embodiments, an agent exhibits both the activity of inhibiting CD14 activity and/or a signaling pathway associated with CD14 activity and the activity of inhibiting TLR2 activity and/or a signaling pathway associated with TLR2 activity.

Examples of agents which exhibit both of the aforementioned activities, include, without limitation, a TLR6 inhibitor, a MyD88 inhibitor, a TIRAP inhibitor, an IRAK1 inhibitor, an IRAK2 inhibitor, an IRAK4 inhibitor, a TRAF6 inhibitor, a TAB1 inhibitor, a TAB2 inhibitor, a TAK1 inhibitor, an IKK inhibitor, a RAS inhibitor, a RAF inhibitor, an MKK3 inhibitor, an MKK6 inhibitor, an MLK inhibitor, a MKK4 inhibitor, a MKK7 inhibitor, a MEK1 inhibitor, a MEK2 inhibitor, a p38 inhibitor, a JNK inhibitor, an ERK1/2 inhibitor, an NFκB inhibitor, an AP-1 inhibitor, a CREB inhibitor, an IRF3 inhibitor, an IRF5 inhibitor, a BTK inhibitor, a JAK2 inhibitor, a Syk inhibitor, a Rac inhibitor, PI3K inhibitor, an AKT inhibitor, an mTORC1 inhibitor, an mTORC2 inhibitor, a PDK1 inhibitor, a TNFAIP3 activator, a TANK activator, an SHP-1 activator, a TOLLIP activator, and an IRAK3 activator.

As CD14 signaling and TLR2 signaling share various common signaling proteins, such as TIRAP and MyD88, such an agent may optionally be a TIRAP inhibitor and/or a MyD88 inhibitor, and/or an inhibitor of the CD14 and TLR2 signaling pathways downstream of TIRAP and MyD88 (e.g., as depicted in Figure 24 and/or Figure 25).

Alternatively or additionally, such an agent may optionally inhibit a protein capable of forming a dimer which both TLR4 (which plays a role in CD14 signaling) and TLR2, for example, a TLR6 inhibitor.

In some embodiments, the agent that exhibits the activity of inhibiting CD14 activity and/or a signaling pathway associated with CD14 activity, does not inhibit activity of TLR5.

In some embodiments, the agent that exhibits the activity of inhibiting CD14 activity and/or a signaling pathway associated with CD14 activity, does not inhibit activity of TLR7.

In some embodiments, the agent that exhibits the activity of inhibiting CD14 activity and/or a signaling pathway associated with CD14 activity, does not inhibit activity of TLR9.

In some embodiments, the agent that exhibits the activity of inhibiting CD14 activity and/or a signaling pathway associated with CD14 activity, does not inhibit activity of TLR5, TLR7, or TLR9.

In some embodiments, the agent that exhibits the activity of inhibiting CD14 activity and/or a signaling pathway associated with CD14 activity, does not inhibit activity of TLR5, TLR7, TLR9 or IL-1β receptor.

In some embodiments, the agent that exhibits the activity of inhibiting CD14 activity and/or a signaling pathway associated with CD14 activity, does not inhibit activity of any TLR other than TLR2 and TLR4.

In some embodiments, the agent that exhibits the activity of inhibiting CD14 activity and/or a signaling pathway associated with CD14 activity, does not inhibit activity of a signaling pathway associated with the aforementioned TLRs (e.g., TLR5, TLR7, and/or TLR9) and/or IL-1β receptor.

In some embodiments, the agent that exhibits the activity of inhibiting TLR2 activity and/or a signaling pathway associated with TLR2 activity, does not inhibit activity of TLR5.

In some embodiments, the agent that exhibits the activity of inhibiting TLR2 activity and/or a signaling pathway associated with TLR2 activity, does not inhibit activity of TLR7.

In some embodiments, the agent that exhibits the activity of inhibiting TLR2 activity and/or a signaling pathway associated with TLR2 activity, does not inhibit activity of TLR9.

In some embodiments, the agent that exhibits the activity of inhibiting TLR2 activity and/or a signaling pathway associated with TLR2 activity, does not inhibit activity of TLR5, TLR7, or TLR9.

In some embodiments, the agent that exhibits the activity of inhibiting TLR2 activity and/or a signaling pathway associated with TLR2 activity, does not inhibit activity of TLR5, TLR7, TLR9, or IL-1β receptor.

In some embodiments, the agent that exhibits the activity of inhibiting TLR2 activity and/or a signaling pathway associated with TLR2 activity, does not inhibit activity of any TLR other than TLR2 and TLR4.

In some embodiments, the agent that exhibits the activity of inhibiting TLR2 activity and/or a signaling pathway associated with TLR2 activity, does not inhibit activity of a signaling pathway associated with the aforementioned TLRs (e.g., TLR5, TLR7, and/or TLR9) and/or IL-1β receptor.

As exemplified herein, inhibition of monocyte chemotaxis may be effected by inhibiting a MEK-ERK signaling pathway.

Hence, according to optional embodiments, an agent which exhibits the activity of inhibiting monocyte chemotaxis, as described herein, effects such inhibition by inhibiting a signaling pathway associated with MEK-ERK activity.

Herein, the phrase "inhibiting a signaling pathway associated with MEK-ERK activity" encompasses any inhibition of an ERK protein, a MEK protein, any protein activated by an ERK (e.g., via phosphorylation by ERK), and any protein which activates a MEK (e.g., a MEK kinase).

Examples of agents which exhibit an activity of inhibiting a signaling pathway associated with MEK-ERK activity include, without limitation, a RAF inhibitor, a MEK1 inhibitor, a MEK2 inhibitor, and an ERK1/2 inhibitor.

It is to be appreciated that the above MEK-ERK pathway inhibitors are also inhibitors of CD14 and TLR2 signaling pathways, although they are relatively non-selective inhibitors of CD14 and TLR2 signaling pathways.

In some embodiments, the agent(s) described herein does not inhibit activity of COX-1.

In some embodiments, the agent(s) described herein does not inhibit activity of COX-2.

In some embodiments, the agent(s) described herein does not inhibit activity of HMG-CoA reductase.

In some embodiments, the agent(s) described herein does not inhibit activity of 12-LO (12-lipoxygenase).

In some embodiments, the agent(s) described herein does not inhibit activity of TNFα receptor.

In some embodiments, the agent(s) described herein does not inhibit activity of Lp-PLA2.

In some embodiments, an agent which "does not inhibit activity" of a protein inhibits said activity by less than 20 % at a concentration of 10 µM of the agent, and in some embodiments, by less than 10 %.

As exemplified herein, inhibition of monocyte chemotaxis is also associated with inhibition of AKT.

Hence, according to optional embodiments, an agent which exhibits the activity of inhibiting monocyte chemotaxis, as described herein, effects such inhibition by inhibiting a signaling pathway associated with PI3K-AKT activity.

Herein, the phrase "inhibiting a signaling pathway associated with PI3K-AKT activity" encompasses any inhibition of a PI3K,AKT, or mTOR. Examples of agents which exhibit an activity of inhibiting a signaling pathway associated with PI3K-AKT activity include, without limitation, a PI3K inhibitor (e.g., as described herein), an AKT inhibitor (e.g., as described herein), and an mTOR inhibitor (e.g., as described herein)

It is to be appreciated that the above PI3K-AKT pathway inhibitors are also inhibitors of CD14 and TLR2 signaling pathways, although they are relatively non-selective inhibitors of CD14 and TLR2 signaling pathways.

In some embodiments, an agent which exhibits the activity of inhibiting monocyte chemotaxis, as described herein, does not inhibit T-cell chemotaxis.

In some embodiments, an agent which exhibits the activity of inhibiting monocyte chemotaxis, as described herein, does not inhibit chemotaxis of any cell other than monocytes.

Without being bound by any particular theory, it is believed that cell-specific inhibition of monocyte chemotaxis provides therapeutically beneficial anti-inflammatory effect, without generating undesirable inhibition of other biologically important functions.

In one example disclosed herein, the oxidized phospholipid useful in accordance with the present disclosure is selected from those disclosed in international patent application publications WO 2010/052718, WO 2010/041242, WO 2008/084472, WO 2004/106486, and WO 2002/041827.

In one embodiment, the oxidized phospholipid is 1-hexadecyl-2-(4'-carboxy)butyl-glycero-3-phosphocholine. In another embodiment, the oxidized phospholipid is *(R)*-1-hexadecyl-2-(4'-carboxy)butyl-*sn*-glycero-3-phosphocholine.

According to an aspect of some embodiments disclosed herein there is provided a pharmaceutical composition comprising at least two agents, the at least two agents being capable of:
a) inhibiting CD14 activity and/or a signaling pathway associated with CD14 activity, and
b) inhibiting TLR2 activity and/or a signaling pathway associated with TLR2 activity,
and a pharmaceutically acceptable carrier.

According to an aspect of some embodiments disclosed herein there is provided a kit comprising at least two agents capable of:
a) inhibiting CD14 activity and/or a signaling pathway associated with CD14 activity, and
b) inhibiting TLR2 activity and/or a signaling pathway associated with TLR2 activity.

According to some embodiments disclosed herein, the at least one agent comprises at least two agents.

According to an aspect of some embodiments disclosed herein there is provided a pharmaceutical composition comprising at least two agents, the at least two agents being capable of exhibiting at least two activities selected from the group consisting of:
a) inhibiting CD14 activity and/or a signaling pathway associated with CD14 activity,
b) inhibiting TLR2 activity and/or a signaling pathway associated with TLR2 activity, and
c) inhibiting monocyte chemotaxis,
and a pharmaceutically acceptable carrier.

According to an aspect of some embodiments disclosed herein there is provided a kit comprising at least two agents that exhibit at least two activities selected from the group consisting of:
a) inhibiting CD14 activity and/or a signaling pathway associated with CD14 activity,
b) inhibiting TLR2 activity and/or a signaling pathway associated with TLR2 activity, and
c) inhibiting monocyte chemotaxis.

According to some embodiments disclosed herein, the at least one agent comprises at least two agents.

According to some embodiments, one of the at least two agents is VB-201 (1-hexadecyl-2-(4'-carboxybutyl)-glycerol-3-phosphocholine).

According to some embodiments disclosed herein, the at least two agents further comprise at least one agent that exhibits the activity of inhibiting monocyte chemotaxis.

According to some embodiments disclosed herein, the at least two agents further comprise at least one agent that exhibits the activity of inhibiting TLR2 activity and/or a signaling pathway associated with TLR2 activity.

According to some embodiments disclosed herein, the at least two agents further comprise at least one agent that exhibits the activity of inhibiting CD14 activity and/or a signaling pathway associated with CD14 activity.

According to some embodiments disclosed herein, the at least two agents comprise at least one agent that exhibits the activity of inhibiting CD14 activity and/or a signaling pathway associated with CD14 activity, and at least one agent that exhibits the activity of inhibiting TLR2 activity and/or a signaling pathway associated with TLR2 activity.

According to some embodiments disclosed herein, the at least two agents further comprise at least one agent that exhibits the activity of inhibiting monocyte chemotaxis.

According to some embodiments disclosed herein, the at least two agents comprise at least one agent that exhibits the activity of inhibiting CD14 activity and/or a signaling pathway associated with CD14 activity, and at least one agent that exhibits the activity of inhibiting monocyte chemotaxis.

According to some embodiments disclosed herein, the at least two agents comprise at least one agent that exhibits the activity inhibiting TLR2 activity and/or a signaling pathway associated with TLR2 activity and at least one agent that exhibits the activity of inhibiting monocyte chemotaxis.

According to some embodiments disclosed herein, the at least two agents are selected from the group consisting of a TLR2 inhibitor, a TLR1 inhibitor, a CD14 inhibitor, a TLR4 inhibitor, an MD-2 inhibitor, an LBP inhibitor, a TLR6 inhibitor, a MyD88 inhibitor, a TRAM inhibitor, a TRIF inhibitor, a TIRAP inhibitor, an IRAK1 inhibitor, an IRAK2 inhibitor, an IRAK4 inhibitor, a TRAF6 inhibitor, a TRAF3 inhibitor, a RIP1 inhibitor, a TAB1 inhibitor, a TAB2 inhibitor, a TAK1 inhibitor, an IKK inhibitor, a RAS inhibitor, a RAF inhibitor, an MKK3 inhibitor, an MKK6 inhibitor, an MLK inhibitor, a MKK4 inhibitor, a MKK7 inhibitor, a MEK1 inhibitor, a MEK2 inhibitor, a p38 inhibitor, a JNK inhibitor, an ERK1/2 inhibitor, an NFκB inhibitor, an AP-1 inhibitor, a CREB inhibitor, an IRF3 inhibitor, an IRF5 inhibitor, a BTK inhibitor, a JAK2 inhibitor, a Syk inhibitor, a CD36 inhibitor, a scavenger receptor-A inhibitor, a Rac inhibitor, PI3K inhibitor, an AKT inhibitor, an mTORC1 inhibitor, an mTORC2 inhibitor, a PDK1 inhibitor, a TNFAIP3 activator, a TANK activator, an SHP-1 activator, a TOLLIP activator, and an IRAK3 activator.

According to some embodiments disclosed herein, the at least one agent comprises an agent that exhibits the activity of inhibiting CD14 activity and/or a signaling pathway associated with CD14 activity, the agent being selected from the group consisting of a CD14 inhibitor, a TLR4 inhibitor, an MD-2 inhibitor, an LBP inhibitor, a TLR6 inhibitor, a MyD88 inhibitor, a TRAM inhibitor, a TRIF inhibitor, a TIRAP inhibitor, an IRAK1 inhibitor, an IRAK2 inhibitor, an IRAK4 inhibitor, a TRAF6 inhibitor, a TRAF3 inhibitor, a RIP1 inhibitor, a TAB1 inhibitor, a TAB2 inhibitor, a TAK1 inhibitor, an IKK inhibitor, a RAS inhibitor, a RAF inhibitor, an MKK3 inhibitor, an MKK6 inhibitor, an MLK inhibitor, a MKK4 inhibitor, a MKK7 inhibitor, a MEK1 inhibitor, a MEK2 inhibitor, a p38 inhibitor, a JNK inhibitor, an ERK1/2 inhibitor, an NFκB inhibitor, an AP-1 inhibitor, a CREB inhibitor, an IRF3 inhibitor, an IRF5 inhibitor, a BTK inhibitor, a JAK2 inhibitor, a Syk inhibitor, a CD36 inhibitor, a scavenger receptor-A inhibitor, a Rac inhibitor, PI3K inhibitor, an AKT inhibitor, an mTORC1 inhibitor, an mTORC2 inhibitor, a PDK1 inhibitor, a TNFAIP3 activator, a TANK activator, an SHP-1 activator, a TOLLIP activator, and an IRAK3 activator.

According to some embodiments disclosed herein, the at least one agent comprises an agent that exhibits the activity of inhibiting CD14 activity and/or a signaling pathway associated with CD14 activity, the agent being selected from the group consisting of TAK-242, eritoran, E5531, CRX-526, NI0101, VIPER,AV411, 1A6, RP105, IC14 monoclonal antibody, MR1007, IMG-2005, Pepinh-TRIF, IMG-2006, 15409, IMG-2002, necrostatin-1, 5Z-7-oxozeanol, BX-795, BMS-345541, AS-206868/SPC-839, tipifarnib, salirasib, sorafenib, BMS-214662, RAF265, XL281, AAL-881, LBT-613, SB-590885, PLX-4720, PLX-4032, L-779,450, GW5074, SB-699393, SP600125, CEP-1347, U0126, GSK1120212, PD184352, PD-0325901, XL518, selumetinib, RDEA119, PD098059, SL-327, ARRY-438162, dilmapimod, SCIO-469, SCIO-323, VX-702, VX-745, AMG-548, BIRB-796, SB203580, SB202190, RO4402257, RO3201195, PH-797804, AZD-6703, TAK-715, PS540446, RWJ-67657, KC706, ARRY-797, CC-401, AS600292, AS601245, AS602801, FR180204, olomoucine, celastrol, triptolide, LGD1550, SR 11302, tanshinone IIA, A-CREB, cyclosporine A, tacrolimus, XX-650-23, AG490, R788, pitavastatin, eicosapentaenoic acid (EPA), docosahexaenoic acid (DHA), 4-hydroxynonenal, hexanal, 2,4-decadienal, GO:0035020, G0:0035021, GO:0035022, GO:0032314, Vav1, Vav2, Tiam1, clostridium difficile toxin B, NSC23766, EHT 1864, statins, an anti-angiotensin antibody, wortmannin, LY294002, IC187114, TG100-115, ZSTK474, PI-103, AR-12, PWT-458, PX-866, CAL-101, XL-147, GDC-0941, (NVP)-BEZ235, AS252424, TGX-221, XL-765, KP372-1, perifosine, triciribine, SR13668, AR-67, AR-42, GSK690693, A-443654, MK-2206, rapamycin, everolimus, temsirolimus, ridaforolimus, AZD-8055, OSI-027, INK-128, PP-242, UCN-01, celecoxib, OSU-03012, (NVP)-BAG956, BX-912, BX-320, hyaluronan, interleukin-1β (IL1β), and Bay11-7082.

According to some embodiments disclosed herein, the at least one agent comprises an agent that exhibits the activity of inhibiting TLR2 activity and/or a signaling pathway associated with TLR2 activity, the agent being selected from the group consisting of a TLR2 inhibitor, a TLR1 inhibitor, a TLR6 inhibitor, a MyD88 inhibitor, a TIRAP inhibitor, an IRAK1 inhibitor, an IRAK2 inhibitor, an IRAK4 inhibitor, a TRAF6 inhibitor, a TAB1 inhibitor, a TAB2 inhibitor, a TAK1 inhibitor, an IKK inhibitor, a RAS inhibitor, a RAF inhibitor, an MKK3 inhibitor, an MKK6 inhibitor, an MLK inhibitor, a MKK4 inhibitor, a MKK7 inhibitor, a MEK1 inhibitor, a MEK2 inhibitor, a p38 inhibitor, a JNK inhibitor, an ERK1/2 inhibitor, an NFκB inhibitor, an AP-1 inhibitor, a CREB inhibitor, an IRF3 inhibitor, an IRF5 inhibitor, a BTK inhibitor, a JAK2 inhibitor, a Syk inhibitor, a Rac inhibitor, PI3K inhibitor, an AKT inhibitor, an mTORC1 inhibitor, an mTORC2 inhibitor, a PDK1 inhibitor, a TNFAIP3 activator, a TANK activator, an SHP-1 activator, a TOLLIP activator, and an IRAK3 activator.

According to some embodiments disclosed herein, the at least one agent comprises an agent that exhibits the activity of inhibiting TLR2 activity and/or a signaling pathway associated with TLR2 activity, the agent being selected from the group consisting of OPN-305, OPN-401, AP177, IMG-2005, IMG-2006, 15409, IMG-2002, 5Z-7-oxozeanol, BX-795, BMS-345541, AS-206868/SPC-839, tipifarnib, salirasib, sorafenib, BMS-214662, RAF265, XL281, AAL-881, LBT-613, SB-590885, PLX-4720, PLX-4032, L-779,450, GW5074, SB-699393, SP600125, CEP-1347, U0126, GSK1120212, PD184352, PD-0325901, XL518, selumetinib, RDEA119, PD098059, SL-327, ARRY-438162, dilmapimod, SCIO-469, SCIO-323, VX-702, VX-745, AMG-548, BIRB-796, SB203580, SB202190, RO4402257, RO3201195, PH-797804, AZD-6703, TAK-715, PS540446, RWJ-67657, KC706, ARRY-797, CC-401, AS600292, AS601245, AS602801, FR180204, olomoucine, celastrol, triptolide, LGD1550, SR 11302, tanshinone IIA, A-CREB, cyclosporine A, tacrolimus, XX-650-23, AG490, R788, GO:0035020, G0:0035021, GO:0035022, GO:0032314, Vav1, Vav2, Tiam1, clostridium difficile toxin B, NSC23766, EHT 1864, statins, an anti-angiotensin antibody, wortmannin, LY294002, IC187114, TG100-115, ZSTK474, PI-103, AR-12, PWT-458, PX-866, CAL-101, XL-147, GDC-0941, (NVP)-BEZ235, AS252424, TGX-221, XL-765, KP372-1, perifosine, triciribine, SR13668, AR-67, AR-42, GSK690693, A-443654, MK-2206, rapamycin, everolimus, temsirolimus, ridaforolimus, AZD-8055, OSI-027, INK-128, PP-242, UCN-01, celecoxib, OSU-03012, (NVP)-BAG956, BX-912, BX-320, hyaluronan, interleukin-1β (IL1β), and Bay11-7082.

According to some embodiments, the agent that exhibits the activity of inhibiting CD14 activity and/or a signaling pathway associated with CD14 activity, and the agent that exhibits the activity of inhibiting TLR2 activity and/or a signaling pathway associated with TLR2 activity, does not inhibit activity of TLR5, TLR7, or TLR9.

According to some embodiments, the at least one agent comprises an agent which exhibits the activity of inhibiting monocyte chemotaxis and which does not inhibit T-cell chemotaxis.

According to some embodiments, the at least one agent comprises an agent which exhibits the activity of inhibiting monocyte chemotaxis, the inhibiting being effected by inhibiting a signaling pathway associated with MEK-ERK activity.

According to some embodiments disclosed herein, the at least two agents are capable of exhibiting the activity of inhibiting CD14 activity and/or a signaling pathway associated with CD14 activity and the activity of inhibiting TLR2 activity and/or a signaling pathway associated with TLR2 activity.

According to some embodiments, the at least two agents are capable of exhibiting the activity of inhibiting CD14 activity and/or a signaling pathway associated with CD14 activity, the activity of inhibiting TLR2 activity and/or a signaling pathway associated with TLR2 activity, and the activity of inhibiting monocyte chemotaxis.

According to some embodiments, the at least two agents comprise at least one agent that exhibits the activity of inhibiting CD14 activity and/or a signaling pathway associated with CD14 activity, and at least one agent that exhibits the activity of inhibiting TLR2 activity and/or a signaling pathway associated with TLR2 activity.

According to some embodiments disclosed herein, the at least two agents further comprise at least one agent that exhibits the activity of inhibiting monocyte chemotaxis.

According to some embodiments, the at least two agents comprise at least one agent that exhibits the activity of inhibiting CD14 activity and/or a signaling pathway associated with CD14 activity, and at least one agent that exhibits the activity of inhibiting monocyte chemotaxis.

According to some embodiments, the at least two agents comprise at least one agent that exhibits the activity inhibiting TLR2 activity and/or a signaling pathway associated with TLR2 activity and at least one agent that exhibits the activity of inhibiting monocyte chemotaxis.

According to some embodiments disclosed herein, the at least two agents are selected from the group consisting of a TLR2 inhibitor, a TLR1 inhibitor, a CD14 inhibitor, a TLR4 inhibitor, an MD-2 inhibitor, an LBP inhibitor, a TLR6 inhibitor, a MyD88 inhibitor, a TRAM inhibitor, a TRIF inhibitor, a TIRAP inhibitor, an IRAK1 inhibitor, an IRAK2 inhibitor, an IRAK4 inhibitor, a TRAF6 inhibitor, a TRAF3 inhibitor, a RIP1 inhibitor, a TAB1 inhibitor, a TAB2 inhibitor, a TAK1 inhibitor, an IKK inhibitor, a RAS inhibitor, a RAF inhibitor, an MKK3 inhibitor, an MKK6 inhibitor, an MLK inhibitor, a MKK4 inhibitor, a MKK7 inhibitor, a MEK1 inhibitor, a MEK2 inhibitor, a p38 inhibitor, a JNK inhibitor, an ERK1/2 inhibitor, an NFκB inhibitor, an AP-1 inhibitor, a CREB inhibitor, an IRF3 inhibitor, an IRF5 inhibitor, a BTK inhibitor, a JAK2 inhibitor, a Syk inhibitor, a CD36 inhibitor, a scavenger receptor-A inhibitor, a Rac inhibitor, PI3K inhibitor, an AKT inhibitor, an mTORC1 inhibitor, an mTORC2 inhibitor, a PDK1 inhibitor, a TNFAIP3 activator, a TANK activator, an SHP-1 activator, a TOLLIP activator, and an IRAK3 activator.

According to some embodiments disclosed herein, the at least two agents comprise an agent that exhibits the activity of inhibiting CD14 activity and/or a signaling pathway associated with CD14 activity, the agent being selected from the group consisting of a CD14 inhibitor, a TLR4 inhibitor, an MD-2 inhibitor, an LBP inhibitor, a TLR6 inhibitor, a MyD88 inhibitor, a TRAM inhibitor, a TRIF inhibitor, a TIRAP inhibitor, an IRAK1 inhibitor, an IRAK2 inhibitor, an IRAK4 inhibitor, a TRAF6 inhibitor, a TRAF3 inhibitor, a RIP1 inhibitor, a TAB1 inhibitor, a TAB2 inhibitor, a TAK1 inhibitor, an IKK inhibitor, a RAS inhibitor, a RAF inhibitor, an MKK3 inhibitor, an MKK6 inhibitor, an MLK inhibitor, a MKK4 inhibitor, a MKK7 inhibitor, a MEK1 inhibitor, a MEK2 inhibitor, a p38 inhibitor, a JNK inhibitor, an ERK1/2 inhibitor, an NFκB inhibitor, an AP-1 inhibitor, a CREB inhibitor, an IRF3 inhibitor, an IRF5 inhibitor, a BTK inhibitor, a JAK2 inhibitor, a Syk inhibitor, a CD36 inhibitor, a scavenger receptor-A inhibitor, a Rac inhibitor, PI3K inhibitor, an AKT inhibitor, an mTORC1 inhibitor, an mTORC2 inhibitor, a PDK1 inhibitor, a TNFAIP3 activator, a TANK activator, an SHP-1 activator, a TOLLIP activator, and an IRAK3 activator.

According to some embodiments disclosed herein, the agent that exhibits the activity of inhibiting CD14 activity and/or a signaling pathway associated with CD14 activity is selected from the group consisting of a CD14 inhibitor, a TLR4 inhibitor, an MD-2 inhibitor, an LBP inhibitor, a TRAM inhibitor, and a TRIF inhibitor.

According to some embodiments disclosed herein, the agent that exhibits the activity of inhibiting CD14 activity and/or a signaling pathway associated with CD14 activity is selected from the group consisting of a CD14 inhibitor, a TLR4 inhibitor, an MD-2 inhibitor, and an LBP inhibitor.

According to some embodiments disclosed herein, the at least two agents comprise an agent that exhibits the activity of inhibiting CD14 activity and/or a signaling pathway associated with CD14 activity, the agent being selected from the group consisting of TAK-242, eritoran, E5531, CRX-526, NI0101, VIPER,AV411, 1A6, RP105, IC14 monoclonal antibody, MR1007, IMG-2005, Pepinh-TRIF, IMG-2006, 15409, IMG-2002, necrostatin-1, 5Z-7-oxozeanol, BX-795, BMS-345541, AS-206868/SPC-839, tipifarnib, salirasib, sorafenib, BMS-214662, RAF265, XL281, AAL-881, LBT-613, SB-590885, PLX-4720, PLX-4032, L-779,450, GW5074, SB-699393, SP600125, CEP-1347, U0126, GSK1120212, PD184352, PD-0325901, XL518, selumetinib, RDEA119, PD098059, SL-327, ARRY-438162, dilmapimod, SCIO-469, SCIO-323, VX-702, VX-745, AMG-548, BIRB-796, SB203580, SB202190, RO4402257, RO3201195, PH-797804, AZD-6703, TAK-715, PS540446, RWJ-67657, KC706, ARRY-797, CC-401, AS600292, AS601245, AS602801, FR180204, olomoucine, celastrol, triptolide, LGD1550, SR 11302, tanshinone IIA, A-CREB, cyclosporine A, tacrolimus, XX-650-23, AG490, R788, pitavastatin, eicosapentaenoic acid (EPA), docosahexaenoic acid (DHA), 4-hydroxynonenal, hexanal, 2,4-decadienal, GO:0035020, G0:0035021, GO:0035022, GO:0032314, Vav1, Vav2, Tiam1, clostridium difficile toxin B, NSC23766, EHT 1864, statins, an anti-angiotensin antibody, wortmannin, LY294002, IC187114, TG100-115, ZSTK474, PI-103, AR-12, PWT-458, PX-866, CAL-101, XL-147, GDC-0941, (NVP)-BEZ235, AS252424, TGX-221, XL-765, KP372-1, perifosine, triciribine, SR13668, AR-67, AR-42, GSK690693, A-443654, MK-2206, rapamycin, everolimus, temsirolimus, ridaforolimus, AZD-8055, OSI-027, INK-128, PP-242, UCN-01, celecoxib, OSU-03012, (NVP)-BAG956, BX-912, BX-320, hyaluronan, interleukin-1β (ILl 0), and Bay11-7082.

According to some embodiments disclosed herein, the agent that exhibits the activity of inhibiting CD14 activity and/or a signaling pathway associated with CD14 activity is selected from the group consisting of TAK-242, eritoran, E5531, CRX-526, NI0101, VIPER, AV411, 1A6, RP105, IC14 monoclonal antibody, MR1007, and Pepinh-TRIF.

According to some embodiments disclosed herein, the agent that exhibits the activity of inhibiting CD14 activity and/or a signaling pathway associated with CD14 activity is selected from the group consisting of TAK-242, eritoran, E5531, CRX-526, NI0101, VIPER, AV411, 1A6, RP105, IC14 monoclonal antibody, and MR1007.

According to some embodiments disclosed herein, the at least two agents comprise an agent that exhibits the activity of inhibiting TLR2 activity and/or a signaling pathway associated with TLR2 activity, the agent being selected from the group consisting of a TLR2 inhibitor, a TLR1 inhibitor, a TLR6 inhibitor, a MyD88 inhibitor, a TIRAP inhibitor, an IRAK1 inhibitor, an IRAK2 inhibitor, an IRAK4 inhibitor, a TRAF6 inhibitor, a TAB1 inhibitor, a TAB2 inhibitor, a TAK1 inhibitor, an IKK inhibitor, a RAS inhibitor, a RAF inhibitor, an MKK3 inhibitor, an MKK6 inhibitor, an MLK inhibitor, a MKK4 inhibitor, a MKK7 inhibitor, a MEK1 inhibitor, a MEK2 inhibitor, a p38 inhibitor, a JNK inhibitor, an ERK1/2 inhibitor, an NFκB inhibitor, an AP-1 inhibitor, a CREB inhibitor, an IRF3 inhibitor, an IRF5 inhibitor, a BTK inhibitor, a JAK2 inhibitor, a Syk inhibitor, a Rac inhibitor, PI3K inhibitor, an AKT inhibitor, an mTORC1 inhibitor, an mTORC2 inhibitor, a PDK1 inhibitor, a TNFAIP3 activator, a TANK activator, an SHP-1 activator, a TOLLIP activator, and an IRAK3 activator.

According to some embodiments, the agent that exhibits the activity of inhibiting TLR2 activity and/or a signaling pathway associated with TLR2 activity is selected from the group consisting of a TLR2 inhibitor, a TLR1 inhibitor, and a TLR6 inhibitor.

According to some embodiments disclosed herein, the at least two agents comprise an agent that exhibits the activity of inhibiting TLR2 activity and/or a signaling pathway associated with TLR2 activity, the agent being selected from the group consisting of OPN-305, OPN-401, AP177, IMG-2005, IMG-2006, 15409, IMG-2002, 5Z-7-oxozeanol, BX-795, BMS-345541, AS-206868/SPC-839, tipifarnib, salirasib, sorafenib, BMS-214662, RAF265, XL281, AAL-881, LBT-613, SB-590885, PLX-4720, PLX-4032, L-779,450, GW5074, SB-699393, SP600125, CEP-1347, U0126, GSK1120212, PD184352, PD-0325901, XL518, selumetinib, RDEA119, PD098059, SL-327, ARRY-438162, dilmapimod, SCIO-469, SCIO-323, VX-702, VX-745, AMG-548, BIRB-796, SB203580, SB202190, RO4402257, RO3201195, PH-797804, AZD-6703, TAK-715, PS540446, RWJ-67657, KC706, ARRY-797, CC-401, AS600292, AS601245, AS602801, FR180204, olomoucine, celastrol, triptolide, LGD1550, SR 11302, tanshinone IIA, A-CREB, cyclosporine A, tacrolimus, XX-650-23, AG490, R788, GO:0035020, G0:0035021, GO:0035022, GO:0032314, Vav1, Vav2, Tiam1, clostridium difficile toxin B, NSC23766, EHT 1864, statins, an anti-angiotensin antibody, wortmannin, LY294002, IC187114, TG100-115, ZSTK474, PI-103, AR-12, PWT-458, PX-866, CAL-101, XL-147, GDC-0941, (NVP)-BEZ235, AS252424, TGX-221, XL-765, KP372-1, perifosine, triciribine, SR13668, AR-67, AR-42, GSK690693, A-443654, MK-2206, rapamycin, everolimus, temsirolimus, ridaforolimus, AZD-8055, OSI-027, INK-128, PP-242, UCN-01, celecoxib, OSU-03012, (NVP)-BAG956, BX-912, BX-320, hyaluronan, interleukin-1β (IL1β), and Bay11-7082.

According to some embodiments disclosed herein, the agent that exhibits the activity of inhibiting TLR2 activity and/or a signaling pathway associated with TLR2 activity is selected from the group consisting of OPN-305, OPN-401, and AP177.

According to some embodiments disclosed herein, the at least two agents comprise an agent which exhibits the activity of inhibiting monocyte chemotaxis and which does not inhibit T-cell chemotaxis.

According to some embodiments disclosed herein, the at least two agents comprise an agent that exhibits the activity of inhibiting monocyte chemotaxis, the inhibiting being effected by inhibiting a signaling pathway associated with MEK-ERK activity.

According to some embodiments disclosed herein, the agent which exhibits the activity of inhibiting a signaling pathway associated with MEK-ERK activity is selected from the group consisting of a RAF inhibitor, a MEK1 inhibitor, a MEK2 inhibitor, and an ERK1/2 inhibitor.

According to some embodiments disclosed herein, the agent which exhibits the activity of inhibiting a signaling pathway associated with MEK-ERK activity is selected from the group consisting of sorafenib, BMS-214662, RAF265, XL281, AAL-881, LBT-613, SB-590885, PLX-4720, PLX-4032, L-779,450, GW5074, SB-699393, U0126, GSK1120212, PD184352, PD-0325901, XL518, selumetinib, RDEA119, PD098059, SL-327, ARRY-438162, FR180204, and olomoucine.

According to some embodiments, the composition is identified for use in the treatment of an inflammatory disease or disorder.

According to some embodiments, the composition is packaged in a packaging material and identified in print, in or on the packaging material, for use in the treatment of an inflammatory disease or disorder.

According to some embodiments, each of the at least two agents is individually packaged within the kit.

According to some embodiments, the kit is identified for use in the treatment of an inflammatory disease or disorder.

According to some embodiments disclosed herein, the inflammatory disease or disorder is selected from the group consisting of an idiopathic inflammatory disease or disorder, a chronic inflammatory disease or disorder, an acute inflammatory disease or disorder, an autoimmune disease or disorder, an infectious disease or disorder, an inflammatory malignant disease or disorder, an inflammatory transplantation-related disease or disorder, an inflammatory degenerative disease or disorder, a disease or disorder associated with a hypersensitivity, an inflammatory cardiovascular disease or disorder, an inflammatory cerebrovascular disease or disorder, a peripheral vascular disease or disorder, an inflammatory glandular disease or disorder, an inflammatory gastrointestinal disease or disorder, an inflammatory cutaneous disease or disorder, an inflammatory hepatic disease or disorder, an inflammatory neurological disease or disorder, an inflammatory musculo-skeletal disease or disorder, an inflammatory renal disease or disorder, an inflammatory reproductive disease or disorder, an inflammatory systemic disease or disorder, an inflammatory connective tissue disease or disorder, an inflammatory tumor, necrosis, an inflammatory implant-related disease or disorder, an inflammatory aging process, an immunodeficiency disease or disorder, a proliferative disease or disorder and an inflammatory pulmonary disease or disorder.

According to some embodiments disclosed herein, the inflammatory disease or disorder is selected from the group consisting of atherosclerosis, rheumatoid arthritis, inflammatory bowel disease, multiple sclerosis, and psoriasis.

Representative chronic autoimmune/inflammatory diseases include, for example, idiopathic inflammatory diseases or disorders, chronic inflammatory diseases or disorders, acute inflammatory diseases or disorders, autoimmune diseases or disorders, infectious diseases or disorders, inflammatory malignant diseases or disorders, inflammatory transplantation-related diseases or disorders, inflammatory degenerative diseases or disorders, diseases or disorders associated with a hypersensitivity, inflammatory cardiovascular diseases or disorders (e.g., as described herein), inflammatory cerebrovascular diseases or disorders, peripheral vascular diseases or disorders, inflammatory glandular diseases or disorders, inflammatory gastrointestinal diseases or disorders, inflammatory cutaneous diseases or disorders, inflammatory hepatic diseases or disorders, inflammatory neurological diseases or disorders, inflammatory musculo-skeletal diseases or disorders, inflammatory renal diseases or disorders, inflammatory reproductive diseases or disorders, inflammatory systemic diseases or disorders, inflammatory connective tissue diseases or disorders, inflammatory tumors, necrosis, inflammatory implant-related diseases or disorders, inflammatory aging processes, immunodeficiency diseases or disorders, proliferative diseases and disorders, and inflammatory pulmonary diseases or disorders.

Non-limiting examples of hypersensitivities include Type I hypersensitivity, Type II hypersensitivity, Type III hypersensitivity, Type IV hypersensitivity, immediate hypersensitivity, antibody mediated hypersensitivity, immune complex mediated hypersensitivity, T lymphocyte mediated hypersensitivity, delayed type hypersensitivity, helper T lymphocyte mediated hypersensitivity, cytotoxic T lymphocyte mediated hypersensitivity, TH1 lymphocyte mediated hypersensitivity, and TH2 lymphocyte mediated hypersensitivity.

Non-limiting examples of cerebrovascular diseases or disorders include stroke, cerebrovascular inflammation, cerebral hemorrhage and vertebral arterial insufficiency.

Non-limiting examples of peripheral vascular diseases or disorders include gangrene, diabetic vasculopathy, ischemic bowel disease, thrombosis, diabetic retinopathy and diabetic nephropathy.

Non-limiting examples of autoimmune diseases or disorders include all of the diseases caused by an immune response such as an autoantibody or cell-mediated immunity to an autoantigen and the like. Representative examples are chronic rheumatoid arthritis, juvenile rheumatoid arthritis, systemic lupus erythematosus, scleroderma, mixed connective tissue disease, polyarteritis nodosa, polymyositis/dermatomyositis, Sjogren's syndrome, Bechet's disease, multiple sclerosis, autoimmune diabetes, Hashimoto's disease, psoriasis, primary myxedema, pernicious anemia, myasthenia gravis, chronic active hepatitis, autoimmune hemolytic anemia, idiopathic thrombocytopenic purpura, uveitis, vasculitides and heparin induced thrombocytopenia.

Non-limiting examples of inflammatory glandular diseases or disorders include pancreatic diseases or disorders, Type I diabetes, thyroid diseases or disorders, Graves' disease, thyroiditis, spontaneous autoimmune thyroiditis, Hashimoto's thyroiditis, idiopathic myxedema, ovarian autoimmunity, autoimmune anti-sperm infertility, autoimmune prostatitis and Type I autoimmune polyglandular syndrome.

Non-limiting examples of inflammatory gastrointestinal diseases or disorders include colitis, ileitis, Crohn's disease, chronic inflammatory intestinal disease, inflammatory bowel syndrome, inflammatory bowel disease, celiac disease, ulcerative colitis, an ulcer, a skin ulcer, a bed sore, a gastric ulcer, a peptic ulcer, a buccal ulcer, a nasopharyngeal ulcer, an esophageal ulcer, a duodenal ulcer and a gastrointestinal ulcer.

Non-limiting examples of inflammatory cutaneous diseases or disorders include acne, an autoimmune bullous skin disease, pemphigus vulgaris, bullous pemphigoid, pemphigus foliaceus, contact dermatitis and drug eruption.

Non-limiting examples of inflammatory hepatic diseases or disorders include autoimmune hepatitis, hepatic cirrhosis, non-alcoholic steatohepatitis (NASH), and biliary cirrhosis.

Non-limiting examples of inflammatory neurological diseases or disorders include multiple sclerosis, Alzheimer's disease, 'Parkinson's disease, myasthenia gravis, motor neuropathy, Guillain-Barre syndrome, autoimmune neuropathy, Lambert-Eaton myasthenic syndrome, paraneoplastic neurological disease or disorder, paraneoplastic cerebellar atrophy, non-paraneoplastic stiff man syndrome, progressive cerebellar atrophy, Rasmussen's encephalitis, amyotrophic lateral sclerosis, Sydeham chorea, Gilles de la Tourette syndrome, autoimmune polyendocrinopathy, dysimmune neuropathy, acquired neuromyotonia, arthrogryposis multiplex, Huntington's disease, AIDS associated dementia, amyotrophic lateral sclerosis (ALS), multiple sclerosis, stroke, an inflammatory retinal disease or disorder, an inflammatory ocular disease or disorder, optic neuritis, spongiform encephalopathy, migraine, headache, cluster headache, and stiff-man syndrome.

Non-limiting examples of inflammatory connective tissue diseases or disorders include autoimmune myositis, primary Sjogren's syndrome, smooth muscle autoimmune disease or disorder, myositis, tendinitis, a ligament inflammation, chondritis, a joint inflammation, a synovial inflammation, carpal tunnel syndrome, arthritis, rheumatoid arthritis, osteoarthritis, ankylosing spondylitis, a skeletal inflammation, an autoimmune ear disease or disorder, and an autoimmune disease or disorder of the inner ear.

Non-limiting examples of inflammatory renal diseases or disorders include autoimmune interstitial nephritis and/or renal cancer.

Non-limiting examples of inflammatory reproductive diseases or disorders include repeated fetal loss, ovarian cyst, or a menstruation associated disease or disorder.

Non-limiting examples of inflammatory systemic diseases or disorders include systemic lupus erythematosus, systemic sclerosis, septic shock, toxic shock syndrome, and cachexia.

Non-limiting examples of infectious disease or disorder include chronic infectious diseases or disorders, a subacute infectious disease or disorder, an acute infectious disease or disorder, a viral disease or disorder, a bacterial disease or disorder, a protozoan disease or disorder, a parasitic disease or disorder, a fungal disease or disorder, a mycoplasma disease or disorder, gangrene, sepsis, a prion disease or disorder, influenza, tuberculosis, malaria, acquired immunodeficiency syndrome, and severe acute respiratory syndrome.

Non-limiting examples of inflammatory transplantation-related diseases or disorders include graft rejection, chronic graft rejection, subacute graft rejection, acute graft rejection hyperacute graft rejection, and graft versus host disease or disorder. Exemplary implants include a prosthetic implant, a breast implant, a silicone implant, a dental implant, a penile implant, a cardiac implant, an artificial joint, a bone fracture repair device, a bone replacement implant, a drug delivery implant, a catheter, a pacemaker, an artificial heart, an artificial heart valve, a drug release implant, an electrode, and a respirator tube.

Non-limiting examples of inflammatory tumors include a malignant tumor, a benign tumor, a solid tumor, a metastatic tumor and a non-solid tumor.

Non-limiting examples of inflammatory pulmonary diseases or disorders include asthma, allergic asthma, emphysema, chronic obstructive pulmonary disease or disorder, sarcoidosis, bronchitis, and pulmonary fibrosis (e.g. idiopathic pulmonary fibrosis [IPF]).

An example of a proliferative disease or disorder is cancer.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

The term "prodrug" refers to an agent, which is converted into the active compound (the active parent drug) *in vivo.* Prodrugs are typically useful for facilitating the administration of the parent drug. They may, for instance, be bioavailable by oral administration whereas the parent drug is not. The prodrug may also have improved solubility as compared with the parent drug in pharmaceutical compositions. Prodrugs are also often used to achieve a sustained release of the active compound *in vivo.* An example, without limitation, of a prodrug would be a compound as described herein, having one or more carboxylic acid moieties, which is administered as an ester (the "prodrug"). Such a prodrug is hydrolysed *in vivo,* to thereby provide the free compound (the parent drug). The selected ester may affect both the solubility characteristics and the hydrolysis rate of the prodrug.

The phrase "pharmaceutically acceptable salt" refers to a charged species of the parent compound and its counter ion, which is typically used to modify the solubility characteristics of the parent compound and/or to reduce any significant irritation to an organism by the parent compound, while not abrogating the biological activity and properties of the administered compound. An example, without limitation, of a pharmaceutically acceptable salt would be a carboxylate anion and a cation such as, but not limited to, ammonium, sodium, potassium and the like.

The term "solvate" refers to a complex of variable stoichiometry (e.g., di-, tri-, tetra-, penta-, hexa-, and so on), which is formed by a solute (the compound of present embodiments) and a solvent, whereby the solvent does not interfere with the biological activity of the solute. Suitable solvents include, for example, ethanol, acetic acid and the like.

The term "hydrate" refers to a solvate, as defined hereinabove, where the solvent is water.

The present embodiments further encompass any enantiomer, diastereomer, pharmaceutically acceptable salts, prodrugs (disclosed herein), hydrates and solvates of the compound VB-201.

VB-201 (1-hexadecyl-2-(4'-carboxybutyl)-glycerol-3-phosphocholine) according to embodiments of the present invention may be a chiral enantiomer of 1-hexadecyl-2-(4'-carboxybutyl)-glycerol-3-phosphocholine, i.e., either the *(R)*- enantiomer (*(R)*-1-hexadecyl-2-(4'-carboxybutyl)-*sn*-glycerol-3-phosphocholine) or the *(S)*- enantiomer (*(S)*-1-hexadecyl-2-(4'-carboxybutyl)-glycerol-3-phosphocholine), or a mixture thereof (e.g., a racemate). According to exemplary embodiments, VB-201 is *(R)*-1-hexadecyl-2-(4'-carboxybutyl)-*sn*-glycerol-3-phosphocholine.

As discussed herein, CD14 and TLR2 each naturally bind to compounds comprising at least one fatty acid moiety (e.g., via a hydrophobic pocket).

In some embodiments disclosed herein, an agent capable of inhibiting CD14 activity and/or a signaling pathway associated with CD14 activity, an agent capable of inhibiting TLR2 activity and/or a signaling pathway associated with TLR2 activity, and/or an agent capable of exhibiting both activities, is a compound comprising a lipid moiety, such as, for example, a fatty acid moiety or a hydrocarbon moiety (e.g., comprising a chain of at least 10 carbon atoms).

Agents suitable for inhibiting CD14 activity and/or a signaling pathway associated with CD14 activity, inhibiting TLR2 activity and/or a signaling pathway associated with TLR2 activity, and/or inhibiting monocyte chemotaxis will be known to one of skill in the art, particularly in view of the guidance provided herein.

Examples of agents that exhibit the activity of inhibiting CD14 activity and/or a signaling pathway associated with CD14 activity, which may be used in embodiments disclosed herein, include, without limitation, TAK-242, eritoran, E5531, CRX-526, NI0101, VIPER,AV411, 1A6, RP105, IC14 monoclonal antibody, MR1007, IMG-2005, Pepinh-TRIF, IMG-2006, 15409, IMG-2002, necrostatin-1, 5Z-7-oxozeanol, BX-795, BMS-345541, AS-206868/SPC-839, tipifarnib, salirasib, sorafenib, BMS-214662, RAF265, XL281, AAL-881, LBT-613, SB-590885, PLX-4720, PLX-4032, L-779,450, GW5074, SB-699393, SP600125, CEP-1347, U0126, GSK1120212, PD184352, PD-0325901, XL518, selumetinib, RDEA119, PD098059, SL-327, ARRY-438162, dilmapimod, SCIO-469, SCIO-323, VX-702, VX-745, AMG-548, BIRB-796, SB203580, SB202190, RO4402257, RO3201195, PH-797804, AZD-6703, TAK-715, PS540446, RWJ-67657, KC706, ARRY-797, CC-401, AS600292, AS601245, AS602801, FR180204, olomoucine, celastrol, triptolide, LGD1550, SR 11302, tanshinone IIA, A-CREB, cyclosporine A, tacrolimus, XX-650-23, AG490, R788, pitavastatin, eicosapentaenoic acid (EPA), docosahexaenoic acid (DHA), 4-hydroxynonenal, hexanal, 2,4-decadienal, GO:0035020, G0:0035021, GO:0035022, GO:0032314, Vav1, Vav2, Tiam1, clostridium difficile toxin B, NSC23766, EHT 1864, statins, an anti-angiotensin antibody, wortmannin, LY294002, IC187114, TG100-115, ZSTK474, PI-103, AR-12, PWT-458, PX-866, CAL-101, XL-147, GDC-0941, (NVP)-BEZ235, AS252424, TGX-221, XL-765, KP372-1, perifosine, triciribine, SR13668, AR-67, AR-42, GSK690693, A-443654, MK-2206, rapamycin, everolimus, temsirolimus, ridaforolimus, AZD-8055, OSI-027, INK-128, PP-242, UCN-01, celecoxib, OSU-03012, (NVP)-BAG956, BX-912, BX-320, hyaluronan, interleukin-1β (IL1β), and Bay11-7082. Examples of relatively selective agents for inhibiting CD14 activity and/or a signaling pathway associated with CD14 activity, include, without limitation, TAK-242, eritoran, E5531, CRX-526, NI0101, VIPER, AV411, 1A6, RP105, IC14 monoclonal antibody, MR1007, and Pepinh-TRIF. In some embodiments, the agent is selected from the group consisting of TAK-242, eritoran, E5531, CRX-526, NI0101, VIPER, AV411, 1A6, RP105, IC14 monoclonal antibody, and MR1007.

Examples of agents that exhibit the activity of inhibiting TLR2 activity and/or a signaling pathway associated with TLR2 activity, which may be used in embodiments disclosed herein, include, without limitation, OPN-305, OPN-401, AP177, IMG-2005, IMG-2006, 15409, IMG-2002, 5Z-7-oxozeanol, BX-795, BMS-345541, AS-206868/SPC-839, tipifarnib, salirasib, sorafenib, BMS-214662, RAF265, XL281, AAL-881, LBT-613, SB-590885, PLX-4720, PLX-4032, L-779,450, GW5074, SB-699393, SP600125, CEP-1347, U0126, GSK1120212, PD184352, PD-0325901, XL518, selumetinib, RDEA119, PD098059, SL-327, ARRY-438162, dilmapimod, SCIO-469, SCIO-323, VX-702, VX-745, AMG-548, BIRB-796, SB203580, SB202190, RO4402257, RO3201195, PH-797804, AZD-6703, TAK-715, PS540446, RWJ-67657, KC706, ARRY-797, CC-401, AS600292, AS601245, AS602801, FR180204, olomoucine, celastrol, triptolide, LGD1550, SR 11302, tanshinone IIA, A-CREB, cyclosporine A, tacrolimus, XX-650-23, AG490, R788, GO:0035020, G0:0035021, GO:0035022, GO:0032314, Vav1, Vav2, Tiam1, clostridium difficile toxin B, NSC23766, EHT 1864, statins, an anti-angiotensin antibody, wortmannin, LY294002, IC187114, TG100-115, ZSTK474, PI-103, AR-12, PWT-458, PX-866, CAL-101, XL-147, GDC-0941, (NVP)-BEZ235, AS252424, TGX-221, XL-765, KP372-1, perifosine, triciribine, SR13668, AR-67, AR-42, GSK690693, A-443654, MK-2206, rapamycin, everolimus, temsirolimus, ridaforolimus, AZD-8055, OSI-027, INK-128, PP-242, UCN-01, celecoxib, OSU-03012, (NVP)-BAG956, BX-912, BX-320, hyaluronan, interleukin-1β (IL1β), and Bay11-7082. Examples of relatively selective agents for inhibiting TLR2 activity and/or a signaling pathway associated with TLR2 activity, include, without limitation, OPN-305, OPN-401, and AP177.

Examples of agents that exhibit an activity of inhibiting a signaling pathway associated with MEK-ERK activity, which may be used in embodiments disclosed herein, for example, as inhibitors of monocyte chemotaxis, include, without limitation, sorafenib, BMS-214662, RAF265, XL281, AAL-881, LBT-613, SB-590885, PLX-4720, PLX-4032, L-779,450, GW5074, SB-699393, U0126, GSK1120212, PD184352, PD-0325901, XL518, selumetinib, RDEA119, PD098059, SL-327, ARRY-438162, FR180204, and olomoucine.

1A6 is a monoclonal antibody which is an antagonist of TLR4, and may optionally be used in embodiments disclosed herein as a TLR4 inhibitor.

5Z-7-oxozeaenol (CAS No. 253863-19-3) is an ATP-competitive irreversible inhibitor of ERK2, TAK1 and MEK1, and may optionally be used in embodiments disclosed herein as a TAK1 inhibitor and/or as an ERK2 inhibitor, and/or as a MEK1 inhibitor, and optionally for inhibiting the MEK-ERK pathway.

A-443654 ((2S)-1-(1H-indol-3-yl)-3-[5-(3-methyl-2H-indazol-5-yl)pyridin-3-yl]oxypropan-2-amine) may optionally be used in embodiments disclosed herein as an AKT inhibitor.

AAL-881, an isoquinoline, is a small molecule inhibitor of Raf activity [Khazak et al., Expert Opin ther Targets 2007, 11:1587-1609; Sathornsumetee et al., Cancer Res 2006, 66:8722-8730], and may optionally be used in embodiments disclosed herein as a Raf inhibitor, and optionally for inhibiting the MEK-ERK pathway.

A-CREB may optionally be used in embodiments disclosed herein as a CREB inhibitor.

AG490 ((E)-2-cyano-3-(3,4-dihydrophenyl)-N-(phenylmethyl)-2-propenamide) may optionally be used in embodiments disclosed herein as a JAK2 inhibitor.

AMG-548 (developed by Amgen Inc.), is known in the art to inhibit p38 [Dominguez et al., Curr Opin Drug Discov Devel 2005, 8:421-430; Verkaar et al., Chem Biol 2011, 18:485-494] may optionally be used in embodiments disclosed herein as a p38 inhibitor.

An anti-angiotensin antibody may optionally be used in embodiments disclosed herein as a Rac inhibitor.

An anti-LBP antibody (e.g., clone biG 412, from Cell Sciences) may optionally be used in embodiments disclosed herein as an LBP inhibitor.

AP177 is an aptamer which specifically binds TLR2 [Chang et al., FASEB J 2009, 23:3078-3088], and may optionally be used in embodiments disclosed herein as a TLR2 inhibitor.

AR-12 (2-amino-N-(4-(5-(phenanthren-2-yl)-3-(trifluoromethyl)-1H-pyrazol-1-yl)phenyl)acetamide), also referred to in the art as OSU-03012, may optionally be used in embodiments disclosed herein as a PI3K inhibitor and/or as an AKT inhibitor and/or as a PDK1 inhibitor.

AR-42 (N-[4-[(hydroxyamino)carbonyl]phenyl]-αS-(1-methylethyl)-benzeneacetamide) may optionally be used in embodiments disclosed herein as an AKT inhibitor.

AR-67 (7-tert-butyldimethylsilyl-10-hydroxycamptothecin) may optionally be used in embodiments disclosed herein as an AKT inhibitor.

ARRY-438162, a MEK1/2 inhibitor developed by Array BioPharma, may optionally be used in embodiments disclosed herein as a MEK1 inhibitor and/or as a MEK2 inhibitor, and optionally for inhibiting the MEK-ERK pathway.

ARRY-797, developed by Array BioPharma, may optionally be used in embodiments disclosed herein as a p38 inhibitor.

AS-206868 (1-[[5-methoxy-2-(2-thienyl)-4-quinazolinyl]amino]-3-methyl-1H-pyrrole-2,5-dione), also referred to in the art as SPC 839, may optionally be used in embodiments disclosed herein as an IKK inhibitor.

AS252424 (5-[5-(4-fluoro-2-hydroxy-phenyl)-furan-2-ylmethylene]-thiazolidine-2,4-dione) may optionally be used in embodiments disclosed herein as a PI3K inhibitor.

AS600292 (N-((5-(4-(1H-benzo[d][1,2,3]triazol-1-yl)piperidin-1-ylsulfonyl)thiophen-2-yl)methyl)-4-chlorobenzamide) may optionally be used in embodiments disclosed herein as a JNK inhibitor.

AS601425 (1,3-benzothiazol-2-yl-(2-{[2-(3-pyridinyl)ethyl]amino}-4-pyrimidinyl)acetonitrile) may optionally be used in embodiments disclosed herein as a JNK inhibitor.

AS602801 (2-(1,3-benzothiazol-2-yl)-2-[2-({4-[(morpholin-4-yl)methyl]phenyl}methoxy)pyrimidin-4-yl]acetonitrile) may optionally be used in embodiments disclosed herein as a JNK inhibitor.

AV411 (2-methyl-1-(2-propan-2-ylpyrazolo[1,5-a]pyridin-3-yl)propan-1-one), also known in the art as ibudilast, may optionally be used in embodiments disclosed herein as a TLR4 inhibitor.

AZD-6703 is known in the art to inhibit p38 [Coulthard et al., Trends Mol Med 2009, 15:369-379] may optionally be used in embodiments disclosed herein as a p38 inhibitor.

AZD-6703 may optionally be used in embodiments disclosed herein as an mTORC1 inhibitor and/or as an mTORC2 inhibitor.

AZD-8055 ((5-(2,4-bis((3S)-3-methylmorpholin-4-yl)pyrido(2,3-d)pyrimidin-7-yl)-2-methoxyphenyl)methanol) may optionally be used in embodiments disclosed herein as an mTORC1 inhibitor and/or as an mTORC2 inhibitor.

Bay11-7082 may optionally be used in embodiments disclosed herein as an IκB activator.

BIRB-796 (N-[3-(1,1-dimethylethyl)-1-(4-methylphenyl)-1H-pyrazol-5-yl]-N'-[4-[2-(4-morpholinyl)ethoxy]-1-naphthalenyl]-urea), also known in the art as doramapimod, may optionally be used in embodiments disclosed herein as a p38 inhibitor.

BMS-214662 ((R)-7-cyano-2,3,4, 5-tetrahydro-1-(1H-imidazol-4-ylmethyl)-3-(phenylmethyl)-4-(2-thienylsulfonyl)-1H-1,4-benzodiazepine) is a benzodiazepine derivative that inhibits farnesylation of Ras, and may optionally be used in embodiments disclosed herein as a Ras inhibitor.

BMS-345541 (4(2'-aminoethyl)amino-1,8-dimethylimidazo(1,2-a)quinoxaline) is known in the art as an inhibitor of the catalytic subunits of IKK, and may optionally be used in embodiments disclosed herein as an IKK inhibitor.

BX-320, which is known in the art to inhibit PDK-1 [Feldman et al., J Biol Chem 2005, 280:19867-19874], may optionally be used in embodiments disclosed herein as a PDK1 inhibitor.

BX-795 (N-[3-[[5-iodo-4-[[3-[(2-thienylcarbonyl)amino]propyl]amino]-2-pyrimidinyl]amino]phenyl]-1-pyrrolidinecarboxamide) may optionally be used in embodiments disclosed herein as an IKK inhibitor and/or as a PDK1 inhibitor.

BX-912 (N-(3-(4-(2-(1H-imidazol-4-yl)ethylamino)-5-bromopyrimidin-2-ylamino)phenyl)pyrrolidine-1-carboxamide) may optionally be used in embodiments disclosed herein as a PDK1 inhibitor.

CAL-101 may optionally be used in embodiments disclosed herein as a PI3K inhibitor.

CC-401, which is known in the art to inhibit JNK [Uehara et al., Transplantation 2004, 15:324-332] may optionally be used in embodiments disclosed herein as a JNK inhibitor.

Celastrol ((9β,13α,14β,20α)-3-hydroxy-9,13-dimethyl-2-oxo-24,25,26-trinoroleana-1(10),3,5,7-tetraen-29-oic acid) is a triterpenoid quinone methide, which may be isolated from *Tripterygium wilfordi* (Thunder of God vine) and *Celastrus regelii*, and may optionally be used in embodiments disclosed herein as an NFκB inhibitor.

Celecoxib (4-[5-(4-methylphenyl)-3-(trifluoromethyl)pyrazol-1-yl]benzenesulfonamide) may optionally be used in embodiments disclosed herein as a PDK1 inhibitor. CEP-1347 (3,9-bis((ethylthio)methyl)-K252a), also referred to in the art as KT7515, is a derivative of the alkaloid K252a ((9*S*-(9α,10β,12α))-2,3,9,10,11,12-hexahydro-10-hydroxy-10-(methoxycarbonyl)-9-methyl-9,12-epoxy-1H-diindolo[1,2,3-fg:3',2',1'-kl]pyrrolo[3,4-i][1,6]benzodiazocin-1-one), and may optionally be used in embodiments disclosed herein as an MLK inhibitor, an MKK4 inhibitor, and/or an MKK7 inhibitor.

*Clostridium difficile* toxin B (e.g., MMDB ID 34928) may optionally be used in embodiments disclosed herein as a Rac inhibitor.

CRX-526 is a lipid A-mimetic known in the art as a TLR4 antagonist [Fort et al., J Immunol 2005, 15:6416-6423], and may optionally be used in embodiments disclosed herein as a TLR4 inhibitor.

Cyclosporin A may optionally be used in embodiments disclosed herein as a CREB inhibitor.

2,4-Decadienal (DDE) may optionally be used in embodiments disclosed herein as a CD36 inhibitor.

Dilmapimod (8-(2,6-difluorophenyl)-2-[(1,3-dihydroxypropan-2-yl)amino]-4-(4-fluoro-2-methylphenyl)pyrido[2,3-d]pyrimidin-7(8H)-one), also referred to in the art as SB-681323, may optionally be used in embodiments disclosed herein as a p38 inhibitor.

Docosahexaenoic acid (DHA) may optionally be used in embodiments disclosed herein as a CD36 inhibitor.

E5531 is a non-toxic derivative of lipid A [Kawata et al., Br J Pharmacol 1999, 127:853-862], and may optionally be used in embodiments disclosed herein as a TLR4 inhibitor.

EHT 1864 (5-(5-(7-(trifluoromethyl)quinolin-4-ylthio)pentyloxy)-2-(morpholinomethyl)-4H-pyran-4-one) may optionally be used in embodiments disclosed herein as a Rac inhibitor.

Eicosapentaenoic acid (EPA) may optionally be used in embodiments disclosed herein as a CD36 inhibitor.

Eritoran (also referred to in the art as E5564) is a compound structurally similar to LPS, and may optionally be used in embodiments disclosed herein as a TLR4 inhibitor.

Everolimus is a derivative of sirolimus (rapamycin), and may optionally be used in embodiments disclosed herein as an mTORC1 inhibitor and/or as an mTORC2 inhibitor.

FR180204 (5-(2-phenylpyrazolo[1,5-a]pyridin-3-yl)-2H-pyrazolo[3,4-c]pyridazin-3-amine) may optionally be used in embodiments disclosed herein as an ERK1/2 inhibitor, and optionally for inhibiting the MEK-ERK pathway.

GDC-0941 (2-(1H-indazol-4-yl)-6-(4-methanesulfonyl-piperazin-1ylmethyl)-4-morpholin-4-yl-thieno[3,2-d]pyrimidine) may optionally be used in embodiments disclosed herein as a PI3K inhibitor.

GSK1120212 (N-[3-[3-cyclopropyl-5-[(2-fluoro-4-iodophenyl)amino]-3,4,6,7-tetrahydro-6,8-dimethyl-2,4,7-trioxopyrido[4,3-d]pyrimidin-1(2H)-yl]phenyl]acetamide), also referred to in the art as JTP-74057, may optionally be used in embodiments disclosed herein as a MEK1 inhibitor and/or a MEK2 inhibitor, and optionally for inhibiting the MEK-ERK pathway.

GSK690693 (4-[2-(4-amino-1,2,5-oxadiazol-3-yl)-1-ethyl-7-((3S)-3-piperidinylmethoxy)-1H-imidazo[4,5-c]pyridin-4-yl]-2-methyl-3-butyn-2-ol) may optionally be used in embodiments disclosed herein as an AKT inhibitor.

GW5074 (3-(3,5-dibromo-4-hydroxy-benzylidene)-5-iodo-1,3-dihydro-indol-2-one) is an exemplary Raf inhibitor, and more particularly, an exemplary Raf inhibitor for inhibiting the MEK-ERK pathway.

Hexanal may optionally be used in embodiments disclosed herein as a CD36 inhibitor.

Hyaluronan may optionally be used in embodiments disclosed herein as an IRAK3 activator (e.g., by stabilization of IRAK3).

4-Hydroxynonenal (4-hydroxy-2-nonenal) may optionally be used in embodiments disclosed herein as a CD36 inhibitor.

15409 (1-(2-(4-morpholinyl)ethyl)-2-(3-nitrobenzoylamino)benzimidazole) may optionally be used in embodiments disclosed herein as an IRAK1 inhibitor and/or as an IRAK4 inhibitor.

IC14 monoclonal antibody is an anti-CD14 antibody [Verbon et al., J Immunol 2001, 168:3599-3605; Verbon et al., J Infect Dis 2003, 187:55-61], and may optionally be used in embodiments disclosed herein as a CD14 inhibitor.

IC187114 may optionally be used in embodiments disclosed herein as a PI3K inhibitor.

IMG-2002 (which may be obtained from Imgenex) is a peptide (SEQ ID NO: 1) known in the art as an inhibitor of TRAF6 [Ye et al., Nature 2002, 418:443-447], and which may optionally be used in embodiments disclosed herein as a TRAF6 inhibitor.

IMG-2005 (which may be obtained from Imgenex) is a peptide (SEQ ID NO: 2) known in the art as an inhibitor of MyD88 homodimerization [Brown & McIntyre, J Immunol 2011, 186:5489-5496; Siednienko et al., J Immunol 2011, 186:2514-2522]. IMG-2005 may optionally be used in embodiments disclosed herein as a MyD88 inhibitor.

IMG-2006 (which may be obtained from Imgenex) is a peptide (SEQ ID NO: 3) known in the art as an inhibitor of TIRAP binding to TLR2 and TLR4 [Liang et al., J Biol Chem 2007, 282:7532-7542; Scott & Billiar, J Biol Chem 2008, 283:29433-29446]. IMG-2006 may optionally be used in embodiments disclosed herein as a TIRAP inhibitor.

INK-128, which is known in the art to inhibit mTORC [Schenone et al., Curr Med Chem 2011, 2995-3014], may optionally be used in embodiments disclosed herein as an mTORC 1 inhibitor and/or as an mTORC2 inhibitor.

Interleukin-1β (IL1β) may optionally be used in embodiments disclosed herein as an IRAK3 activator (e.g., by induction of IRAK3).

KC706 may optionally be used in embodiments disclosed herein as a p38 inhibitor.

KP372-1 (CAS No. 329710-24-9) may optionally be used in embodiments disclosed herein as an AKT inhibitor and/or as a PDK1 inhibitor.

L-779,450 (2-chloro-5-[2-phenyl-5-(4-pyridinyl)-1*H*-imidazol-4-yl]phenol) may optionally be used in embodiments disclosed herein as a Raf inhibitor, and optionally for inhibiting the MEK-ERK pathway.

LBT-613, an isoquinoline, is a small molecule inhibitor of Raf activity [Ouyang et al., Clin Cancer Res 2006, 12:1785-1793; Hjelmeland et al., Mol Cancer Ther 2007, 6:2449-2457], and may optionally be used in embodiments disclosed herein as a Raf inhibitor, and optionally for inhibiting the MEK-ERK pathway.

LGD1550 ((2E,4E,6E)-3-methyl-7-(3,5-di-tert-butylphenyl)octatrienoic acid) may optionally be used in embodiments disclosed herein as an AP-1 inhibitor.

Lonafarnib (4-(2-(4-(8-chloro-3,10-dibromo-6,11-dihydro-5H-benzo(5,6)cyclohepta(1,2-b)pyridin-11-yl)-1-piperidinyl)-2-oxoethyl)-1-piperidinecarboxamide) inhibits Ras activity by inhibiting activity of farnesyltransferase, and may optionally be used in embodiments disclosed herein as a Ras inhibitor.

LY294002 (2-(4-morpholinyl)-8-phenyl-4H-1-benzopyran-4-one) may optionally be used in embodiments disclosed herein as a PI3K inhibitor.

MK-2206 (CAS No. 1032350-13-2) may optionally be used in embodiments disclosed herein as an AKT inhibitor.

MR1007 is a fusion protein comprising an anti-CD14 antibody and a modified light chain of inter-α-trypsin inhibitor [Nakamura et al., Critical Care 2007, 11(Suppl 4):P4; Nakamura et al., Critical Care 2008, 12(Suppl 2):P194], and may optionally be used in embodiments disclosed herein as a CD14 inhibitor.

NI0101 is an anti-human TLR4 monoclonal antibody, and may optionally be used in embodiments disclosed herein as a TLR4 inhibitor.

Necrostatin-1 (5-(1H-indol-3-ylmethyl)-3-methyl-2-thioxo-4-imidazolidinone) may optionally be used in embodiments disclosed herein as a RIP1 inhibitor.

NSC23766 (N6-[2-[[4-(diethylamino)-1-methylbutyl]amino]-6-methyl-4-pyrimidinyl]-2-methyl-4,6-quinolinediamine) may optionally be used in embodiments disclosed herein as a Rac inhibitor.

(NVP)-BAG956 (2-methyl-2-[4-(2-methyl-8-pyridin-3-ylethynyl-imidazo[4,5-c]quinolin-1-yl)-phenyl]-propionitrile) may optionally be used in embodiments disclosed herein as a PDK1 inhibitor.

(NVP)-BEZ235 (2-methyl-2-{4-[3-methyl-2-oxo-8-(quinolin-3-yl)-2,3-dihydro-1H-imidazo[4,5-c]quinolin-1-yl]phenyl}propanenitrile) may optionally be used in embodiments disclosed herein as a PI3K inhibitor.

Olomoucine (6-(benzylamino)-2-(2-hydroxyethylamino)-9-methylpurine) may optionally be used in embodiments disclosed herein as an ERK1/2 inhibitor, and optionally for inhibiting the MEK-ERK pathway.

OPN-305 is a humanized IgG4 monoclonal antibody against TLR2 [Hennessy et al. Nat Rev Drug Discov 2010, 9:293-307], and may optionally be used in embodiments disclosed herein as a TLR2 inhibitor.

OPN-401 is a a peptide derived from a viral protein [Hennessy et al. Nat Rev Drug Discov 2010, 9:293-307], and may optionally be used in embodiments disclosed herein as a TLR2 inhibitor.

OSI-027 (4-(4-amino-5-(7-methoxy-1H-indol-2-yl)imidazo[5,1-f][1,2,4]triazin-7-yl)cyclohexanecarboxylic acid) may optionally be used in embodiments disclosed herein as an mTORC1 inhibitor and/or as an mTORC2 inhibitor.

PD-0325901 (*N*-[(2*R*)-2,3-dihydroxypropoxy]-3,4-difluoro-2-[(2-fluoro-4-iodophenyl)amino]-benzamide) may optionally be used in embodiments disclosed herein as a MEK1 inhibitor and/or a MEK2 inhibitor, and optionally for inhibiting the MEK-ERK pathway.

PD098059 (2-(2-amino-3-methoxyphenyl)-4H-1-benzopyran-4-one) inhibits MEK activity, particularly MEK1 activity, and may optionally be used in embodiments disclosed herein as a MEK1 inhibitor and/or a MEK2 inhibitor, and optionally for inhibiting the MEK-ERK pathway.

PD184352 (2-(2-chloro-4-iodophenylamino)-N-(cyclopropylmethoxy)-3,4-difluorobenzamide), also referred to in the art as CI-2040, may optionally be used in embodiments disclosed herein as a MEK1 inhibitor and/or a MEK2 inhibitor, and optionally for inhibiting the MEK-ERK pathway.

Pepinh-TRIF is a peptide (SEQ ID NO: 4) known in the art for interfering with TLR-TRIF interactions [Toshchakov et al., J Immunol 2005, 175:494-500], and may optionally be used in embodiments disclosed herein as a TRIF inhibitor.

Perifosine may optionally be used in embodiments disclosed herein as an AKT inhibitor and/or as a PI3K inhibitor.

PH-797804 ([3-[3-bromo-4-[(2,4-difluorophenyl)methoxy]-6-methyl-2-oxo-1(2H)-pyridinyl]-N,4-dimethyl]benzamide) may optionally be used in embodiments disclosed herein as a p38 inhibitor.

PI-103 (3-[4-(4-morpholinyl)pyrido[3',2':4,5]furo[3,2-d]pyrimidin-2-yl]-phenol) may optionally be used in embodiments disclosed herein as a PI3K inhibitor.

Pitavastatin (a statin; (3R,5S,6E)-7-[2-cyclopropyl-4-(4-fluorophenyl)quinolin-3-yl]-3,5-dihydroxyhept-6-enoic acid) may optionally be used in embodiments disclosed herein as a CD36 inhibitor.

PLX-4032 (N-(3-(5-(4-chlorophenyl)-1H-pyrrolo[2,3-b]pyridine-3-carbonyl)-2,4-difluorophenyl)propane-1-sulfonamide), also referred to in the art as vemurafenib, inhibits Raf activity, particularly B-Raf activity, and may optionally be used in embodiments disclosed herein as a Raf inhibitor, and optionally as Raf inhibitor for inhibiting the MEK-ERK pathway.

PLX-4720 (N-(3-(5-chloro-1H-pyrrolo[2,3-b]pyridine-3-carbonyl)-2,4-difluorophenyl)propane-1-sulfonamide) is a 7-azaindole derivative that inhibits Raf, particularly B-Raf, and may optionally be used in embodiments disclosed herein as a Raf inhibitor, and optionally as Raf inhibitor for inhibiting the MEK-ERK pathway.

PP-242 (2-(4-amino-1-isopropyl-1H-pyrazolo[3,4-d]pyrimidin-3 -yl)-1H-indol-5-ol) may optionally be used in embodiments disclosed herein as an mTORC1 inhibitor and/or as an mTORC2 inhibitor.

PS540446 (4-(5-(cyclopropylcarbamoyl)-2-methylphenylamino)-5-methyl-N-propylpyrrolo[1,2-f][1,2,4]triazine-6-carboxamide) may optionally be used in embodiments disclosed herein as a p38 inhibitor.

PWT-458 (17β-hydroxy-11-(acetyloxy)-1S,6bR,7,8,9aS,10,11R,11bR-octahydro-1-(methoxymethyl)-9a,11b-dimethyl-3H-furo[4,3,2-de]indeno[4,5-h]-2-benzopyran-3,6, 9-trione), aPEGylated wortmannin derivative, may optionally be used in embodiments disclosed herein as a PI3K inhibitor.

PX-866 may optionally be used in embodiments disclosed herein as a PI3K inhibitor.

R788 ([6-({5-fluoro-2-[(3,4,5-trimethoxyphenyl)amino]pyrimidin-4-yl}amino)-2,2-dimethyl-3-oxo-2,3-dihydro-4H-pyrido[3,2-b][1,4]oxazin-4-yl]methyl phosphate), also referred to in the art as fostamatinib, may optionally be used in embodiments disclosed herein as a Syk inhibitor.

RAF265 (1-methyl-5-(2-(4-(trifluoromethyl)-1H-imidazol-2-yl)pyridin-4-yloxy)-N-(4-(trifluoromethyl)phenyl)-1H-benzo[d]imidazol-2-amine) is a Raf inhibitor developed by Novartis, and may optionally be used in embodiments disclosed herein as a Raf inhibitor, and optionally as Raf inhibitor for inhibiting the MEK-ERK pathway.

Rapamycin, also referred to in the art as sirolimus, may optionally be used in embodiments disclosed herein as an mTORC1 inhibitor and/or as an mTORC2 inhibitor.

RDEA119 (N-(3,4-difluoro-2-(2-fluoro-4-iodophenylamino)-6-methoxyphenyl)-1-(2,3-dihydroxypropyl)cyclopropane-1-sulfonamide), also referred to in the art as BAY-869766, may optionally be used in embodiments disclosed herein as a MEK1 inhibitor and/or a MEK2 inhibitor, and optionally for inhibiting the MEK-ERK pathway.

Ridaforolimus may optionally be used in embodiments disclosed herein as an mTORC1 inhibitor and/or as an mTORC2 inhibitor.

RO3201195 (S-[5-amino-1-(4-fluorophenyl)-1H-pyrazol-4-yl]-[3-(2,3-dihydroxypropoxy)phenyl]methanone) may optionally be used in embodiments disclosed herein as a p38 inhibitor.

RO4402257 (6-(2,4-difluorophenoxy)-2-[3-hydroxy-1-(2-hydroxyethyl)propylamino]-8-methyl-8H-pyrido[2,3-d]pyrimidin-7-one), also known in the art as pamapimod, may optionally be used in embodiments disclosed herein as a p38 inhibitor.

RP105 is a TLR homolog which lacks a signaling domain [Divanovic et al., Nat Immunol 2005, 6:571-578]. RP105 interacts with the TLR4-MD2 complex, and may optionally be used in embodiments disclosed herein as a TLR4 inhibitor and/or as an MD-2 inhibitor.

RWJ-67657 (4-[4-(4-fluorophenyl)-1-(3-phenylpropyl)-5-(4-pyridinyl)-1H-imidazol-2-yl]-3-butyn-1-ol) may optionally be used in embodiments disclosed herein as a p38 inhibitor.

Salirasib (S-farnesylthiosalicylic acid) may optionally be used in embodiments disclosed herein as a Ras inhibitor.

SB202190 (4-[4-(4-fluorophenyl)-5-(4-pyridinyl)-1H-imidazol-2-yl]phenol) may optionally be used in embodiments disclosed herein as a p38 inhibitor.

SB203580 (4-[5-(4-fluorophenyl)-2-[4-(methylsulfonyl)phenyl]-1H-imidazol-4-yl]pyridine) may optionally be used in embodiments disclosed herein as a p38 inhibitor.

SB-590885 (N,N-dimethyl-2-[4-[(4Z)-4-(1-nitroso-2, 3-dihydroinden-5-ylidene)-5-(1H-pyridin-4-ylidene)-1H-imidazol-2-yl]phenoxy]ethanamine) inhibits Raf activity, particularly B-Raf activity, and may optionally be used in embodiments disclosed herein as a Raf inhibitor, and optionally for inhibiting the MEK-ERK pathway.

SB-699393 (4-[[(4E)-4-(1-nitroso-2, 3-dihydroinden-5-ylidene)-5-(1H-pyridin-4-ylidene)furan-2-yl]methyl]morpholine) inhibits Raf activity, particularly B-Raf activity, and may optionally be used in embodiments disclosed herein as a Raf inhibitor, and optionally for inhibiting the MEK-ERK pathway.

SCIO-323, an inhibitor developed by Scios Inc. and Johnson & Johnson [Dominguez et al., Curr Opin Drug Discov Devel 2005, 8:421-430], may optionally be used in embodiments disclosed herein as a p38 inhibitor.

SCIO-469 (6-chloro-5-[[(2R,5S)-4-[(4-fluorophenyl)methyl]-2,5-dimethyl-1-piperazinyl]carbonyl]-N,N,1-trimethyl-α-oxo-1H-indole-3-acetamide) may optionally be used in embodiments disclosed herein as a p38 inhibitor.

Selumetinib (6-[(4-bromo-2-chlorophenyl)amino]-7-fluoro-N-(2-hydroxyethoxy)-3-methylbenzimidazole-5-carboxamide), also referred to in the art as AZD6244 and ARRY-142886, may optionally be used in embodiments disclosed herein as a MEK1 inhibitor and/or a MEK2 inhibitor, and optionally for inhibiting the MEK-ERK pathway.

SL-327 (α-[amino[(4-aminophenyl)thio]methylene]-2-(trifluoromethyl)benzeneacetonitrile) may optionally be used in embodiments disclosed herein as a MEK1 inhibitor and/or a MEK2 inhibitor, and optionally for inhibiting the MEK-ERK pathway.

Sorafenib (4-(4-(3-(4-chloro-3-(trifluoromethyl)phenyl)ureido)phenoxy)-N-methylpicolinamide) is an inhibitor of Raf, particularly C-Raf, and targets the Raf-MEK-ERK pathway. Sorafenib may optionally be used in embodiments disclosed herein as a Raf inhibitor, and optionally as a Raf inhibitor for inhibiting the MEK-ERK pathway.

SP600125 (anthra(1,9-cd)pyrazol-6(2H)-one) may optionally be used in embodiments disclosed herein as a JNK inhibitor, and optionally as an MKK3 and/or MKK6 inhibitor.

SR 11302 ((E,E,Z,E)-3-methyl-7-(4-methylphenyl)-9-(2,6,6-trimethyl-1-cyclohexen-1-yl)-2,4,6,8-nonatetraenoic acid) may optionally be used in embodiments disclosed herein as an AP-1 inhibitor.

SR13668 (2,10-dicarbethoxy-6-methoxy-5,7-dihydro-indolo[2,3-b]carbazole) may optionally be used in embodiments disclosed herein as an AKT inhibitor.

Statins are a family of compounds known in the art, each of which may optionally be used in embodiments disclosed herein as a Rac inhibitor.

Tacrolimus, also referred to in the art as FK-506 and fujimycin, may optionally be used in embodiments disclosed herein as a CREB inhibitor.

TAK-242 ((6R)-6-[[(2-chloro-4-fluorophenyl)amino]sulfonyl]-1-cyclohexene-1-carboxylic acid ethyl ester), also known in the art as resatorvid, may optionally be used in embodiments disclosed herein as a TLR4 inhibitor.

TAK-715 (N-[4-[2-ethyl-4-(3-methylphenyl)-1,3-thiazol-5-yl]-2-pyridyl]benzamide) may optionally be used in embodiments disclosed herein as a p38 inhibitor.

Tanshinone IIA (6,7,8,9-tetrahydro-1,6,6-trimethylphenanthro[1,2-b]furan-10,11-dione) is a compound which may be isolated from *Salvia miltiorrhiza*, and may optionally be used in embodiments disclosed herein as an AP-1 inhibitor.

Temsirolimus is a derivative of sirolimus (rapamycin), and may optionally be used in embodiments disclosed herein as an mTORC1 inhibitor and/or as an mTORC2 inhibitor.

TG100-115 (3,3'-(2,4-diamino-6,7-pteridinediyl)bisphenol) may optionally be used in embodiments disclosed herein as a PI3K inhibitor.

TGX-221 (7-methyl-2-(4-morpholinyl)-9-[1-(phenylamino)ethyl]-4H-pyrido[1,2-a]pyrimidin-4-one) may optionally be used in embodiments disclosed herein as a PI3K inhibitor.

Tiam1 (e.g., UniProt Q13009) may optionally be used in embodiments disclosed herein as a Rac inhibitor.

Tipifarnib ((+)-6-[amino(4-chlorophenyl)(1-methyl-1H-imidazol-5-yl)methyl]-4-(3-chlorophenyl)-1-methyl-2(1H)-quinolinone) inhibits Ras activity by inhibiting activity of farnesyltransferase, and may optionally be used in embodiments disclosed herein as a Ras inhibitor.

TNF-α may optionally be used in embodiments disclosed herein as an IRAK3 activator (e.g., by induction of IRAK3).

Triciribine (1,5-dihydro-5-methyl-1-β-D-ribofuranosyl-1,2,5,6,8-pentaazaacenaphthylen-3-amine), also referred to in the art as API-2, may optionally be used in embodiments disclosed herein as an AKT inhibitor.

Triptolide is a compound which may be isolated from *Tripterygium wilfordi,* and may optionally be used in embodiments disclosed herein as an NFκB inhibitor.

U0126 (1,4-diamino-2,3-dicyano-1,4-bis(2-aminophenylthio)-butadiene) inhibits both active and inactive MEK1 and MEK2, may optionally be used in embodiments disclosed herein as a MEK1 inhibitor and/or as a MEK2 inhibitor, and optionally for inhibiting the MEK-ERK pathway.

UCN-01 (7-hydroxystaurosporine) may optionally be used in embodiments disclosed herein as a PDK1 inhibitor.

Vav1 (e.g., UniProt P15498) and Vav2 (e.g., UniProt P52735) may each optionally be used in embodiments disclosed herein as a Rac inhibitor.

VIPER is a viral inhibitor peptide of TLR4 [Lysakova-Devine et al., J Immunol 2010, 185:4261-4271], and may optionally be used in embodiments disclosed herein as a TLR4 inhibitor.

VX-702 (6-[(aminocarbonyl)(2,6-difluorophenyl)amino]-2-(2,4-difluorophenyl)-3-pyridinecarboxamide) may optionally be used in embodiments disclosed herein as a p38 inhibitor.

VX-745 (5-(2,6-dichlorophenyl)-2-[2,4-difluorophenyl)thio]-6*H*-pyrimido[1,6-*b*]pyridazin-6-one) may optionally be used in embodiments disclosed herein as a p38 inhibitor.

Wortmannin may optionally be used in embodiments disclosed herein as a PI3K inhibitor.

XL147 (N-(3-(benzo[c][1,2,5]thiadiazol-5-ylamino)quinoxalin-2-yl)-4-methylbenzenesulfonamide) may optionally be used in embodiments disclosed herein as a PI3K inhibitor.

XL281, also known in the art as BMS-908662, may optionally be used in embodiments disclosed herein as a Raf inhibitor, and optionally as a Raf inhibitor for inhibiting the MEK-ERK pathway.

XL518 ((S)-(3,4-difluoro-2-((2-fluoro-4-iodophenyl)amino)phenyl)(3-hydroxy-3-(piperidin-2-yl)cyclobutyl)methanone) inhibits MEK activity, particularly MEK1 activity. XL518 may optionally be used in embodiments disclosed herein as a MEK1 inhibitor, and optionally for inhibiting the MEK-ERK pathway.

XL-765 (N-[2-[(3,5-dimethoxyphenyl)amino]quinoxalin-3-yl]-4-[(4-methyl-3-methoxyphenyl)carbonyl]aminophenylsulfonamide) may optionally be used in embodiments disclosed herein as a PI3K inhibitor and/or as an mTORC1 inhibitor and/or as an mTORC2 inhibitor.

XX-650-23 (molecular weight 288 Da) may optionally be used in embodiments disclosed herein as a CREB inhibitor.

ZSTK474 (2-(2-difluoromethylbenzimidazol-1-yl)-4,6-dimorpholino-1,3,5-triazine) may optionally be used in embodiments disclosed herein as a PI3K inhibitor.

In some embodiments, one or more agents as described herein are not substantially capable of crossing a cell plasma membrane (e.g., a plasma membrane of an immune cell). In some embodiments, such an agent binds to a target (e.g., protein) located on a cell surface. Examples of such agents disclosed herein include, for example, a TLR2 inhibitor, a TLR1 inhibitor, a CD14 inhibitor, a TLR4 inhibitor, an MD-2 inhibitor, an LBP inhibitor, and a TLR6 inhibitor (e.g., as described herein).

Without being bound by any particular theory, it is believed that absence of an ability to cross a plasma membrane affects what targets may be bound by an agent, and that such an agent may consequently have a target profile similar to that of VB-201, which does not substantially cross plasma membranes and binds to cell surface targets such as CD14 and TLR2.

As used herein throughout, the term "alkyl" refers to a saturated aliphatic hydrocarbon including straight chain and branched chain groups. Preferably, the alkyl group has 1 to 20 carbon atoms. Whenever a numerical range; e.g., "1-20", is stated herein, it implies that the group, in this case the alkyl group, may contain 1 carbon atom, 2 carbon atoms, 3 carbon atoms, etc., up to and including 20 carbon atoms. More preferably, the alkyl is a medium size alkyl having 1 to 10 carbon atoms. Most preferably, unless otherwise indicated, the alkyl is a lower alkyl having 1 to 4 carbon atoms. The alkyl group may be substituted or unsubstituted. When substituted, the substituent group can be, for example, cycloalkyl, alkenyl, alkynyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, sulfonyl, sulfinyl, sulfonamide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein.

A "cycloalkyl" group refers to an all-carbon monocyclic or fused ring *(i.e.,* rings which share an adjacent pair of carbon atoms) group wherein one of more of the rings does not have a completely conjugated pi-electron system. Examples, without limitation, of cycloalkyl groups are cyclopropane, cyclobutane, cyclopentane, cyclopentene, cyclohexane, cyclohexadiene, cycloheptane, cycloheptatriene, and adamantane. A cycloalkyl group may be substituted or unsubstituted. When substituted, the substituent group can be, for example, alkyl, alkenyl, alkynyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, sulfonyl, sulfinyl, sulfonamide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein.

An "alkenyl" group refers to an alkyl group which consists of at least two carbon atoms and at least one carbon-carbon double bond.

An "alkynyl" group refers to an alkyl group which consists of at least two carbon atoms and at least one carbon-carbon triple bond.

An "aryl" group refers to an all-carbon monocyclic or fused-ring polycyclic (*i.e*., rings which share adjacent pairs of carbon atoms) groups having a completely conjugated pi-electron system. Examples, without limitation, of aryl groups are phenyl, naphthalenyl and anthracenyl. The aryl group may be substituted or unsubstituted. When substituted, the substituent group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, sulfonyl, sulfinyl, sulfonamide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein.

A "heteroaryl" group refers to a monocyclic or fused ring (*i.e*., rings which share an adjacent pair of atoms) group having in the ring(s) one or more atoms, such as, for example, nitrogen, oxygen and sulfur and, in addition, having a completely conjugated pi-electron system. Examples, without limitation, of heteroaryl groups include pyrrole, furane, thiophene, imidazole, oxazole, thiazole, pyrazole, pyridine, pyrimidine, quinoline, isoquinoline and purine. The heteroaryl group may be substituted or unsubstituted. When substituted, the substituent group can be, for example, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, sulfonyl, sulfinyl, sulfonamide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein.

A "heteroalicyclic" group refers to a monocyclic or fused ring group having in the ring(s) one or more atoms such as nitrogen, oxygen and sulfur. The rings may also have one or more double bonds. However, the rings do not have a completely conjugated pi-electron system. The heteroalicyclic may be substituted or unsubstituted. When substituted, the substituted group can be, for example, lone pair electrons, alkyl, alkenyl, alkynyl, cycloalkyl, aryl, heteroaryl, heteroalicyclic, halo, hydroxy, alkoxy, aryloxy, thiohydroxy, thioalkoxy, thioaryloxy, sulfinyl, sulfonyl, cyano, nitro, azide, sulfonyl, sulfinyl, sulfonamide, phosphonyl, phosphinyl, oxo, carbonyl, thiocarbonyl, urea, thiourea, O-carbamyl, N-carbamyl, O-thiocarbamyl, N-thiocarbamyl, C-amido, N-amido, C-carboxy, O-carboxy, sulfonamido, and amino, as these terms are defined herein. Representative examples are piperidine, piperazine, tetrahydro furane, tetrahydropyrane, morpholino and the like.

A "hydroxy" group refers to an -OH group.

An "azide" group refers to a -N=N group.

An "alkoxy" group refers to both an -O-alkyl and an -O-cycloalkyl group, as defined herein.

An "aryloxy" group refers to both an -O-aryl and an -O-heteroaryl group, as defined herein.

A "thiohydroxy" group refers to a -SH group.

A "thioalkoxy" group refers to both an -S-alkyl group, and an -S-cycloalkyl group, as defined herein.

A "thioaryloxy" group refers to both an -S-aryl and an -S-heteroaryl group, as defined herein.

A "carbonyl" group refers to a -C(=O)-R group, where R is hydrogen, alkyl, alkenyl, cycloalkyl, aryl, heteroaryl (bonded through a ring carbon) or heteroalicyclic (bonded through a ring carbon) as defined herein.

An "aldehyde" group refers to a carbonyl group, where R is hydrogen.

A "thiocarbonyl" group refers to a -C(=S)-R group, where R is as defined herein.

A "C-carboxy" group refers to a -C(=O)-O-R groups, where R is as defined herein.

An "O-carboxy" group refers to an RC(=O)-O- group, where R is as defined herein.

An "oxo" group refers to a =O group.

A "carboxylic acid" group refers to a C-carboxyl group in which R is hydrogen.

A "halo" group refers to fluorine, chlorine, bromine or iodine.

A "trihalomethyl" group refers to a -CX₃ group wherein X is a halo group as defined herein.

A "sulfinyl" group refers to an -S(=O)-R group, where R is as defined herein.

A "sulfonyl" group refers to an -S(=O)₂-R group, where R is as defined herein.

An "S-sulfonamido" group refers to a -S(=O)₂-NR₂ group, with each of R as is defined herein.

An "N-sulfonamido" group refers to an RS(=O)₂-NR group, where each of R is as defined herein.

An "O-carbamyl" group refers to an -OC(=O)-NR₂ group, where each of R is as defined herein.

An "N-carbamyl" group refers to an ROC(=O)-NR- group, where each of R is as defined herein.

An "O-thiocarbamyl" group refers to an -OC(=S)-NR₂ group, where each of R is as defined herein.

An "N-thiocarbamyl" group refers to an ROC(=S)NR- group, where each of R is as defined herein.

An "amino" group refers to an -NR₂ group where each of R is as defined herein.

A "C-amido" group refers to a -C(=O)-NR₂ group, where each of R is as defined herein.

An "N-amido" group refers to an RC(=O)-NR- group, where each of R is as defined herein.

An "urea" group refers to an -NRC(=O)-NR₂ group, where each of R is as defined herein.

A "guanidino" group refers to an -RNC(=N)-NR₂ group, where each of R is as defined herein.

A "guanyl" group refers to an R₂NC(=N)- group, where each of R is as defined herein.

A "nitro" group refers to an -NO₂ group.

A "cyano" group refers to a -C≡N group.

The term "phosphonyl" or "phosphonate" describes a -P(=O)(OR)₂ group, with R as defined hereinabove.

The term "phosphate" describes an -O-P(=O)(OR)₂ group, with each of R as defined hereinabove.

A "phosphoric acid" is a phosphate group is which each of R is hydrogen.

The term "phosphinyl" describes a -PR₂ group, with each of R as defined hereinabove.

The term "thiourea" describes a -NR-C(=S)-NR- group, with each of R as defined hereinabove.

The term "saccharide" refers to one or more sugar unit, either an open-chain sugar unit or a cyclic sugar unit (e.g., pyranose- or furanose-based units), and encompasses any monosaccharide, disaccharide and oligosaccharide, unless otherwise indicated.

The agent or agents described herein may be administered *per se* or as part of a pharmaceutical composition, which optionally further comprises a carrier.

When two or more agents (e.g., as described herein) are administered as a pharmaceutical composition, each agent may optionally be administered in a separate composition and/or via a different route of administration.

Possible routes of administration for each agent independently include, but are not limited to, parenteral administration, transmucosal administration, rectal administration, buccal administration and/or inhalation (e.g., as described herein)

Alternatively, at least some of the agents (e.g., 2 of 3 agents) are combined in a pharmaceutical composition. Such a combination may facilitate practicing treatment of a disease or disorder (e.g., as described herein).

Hence, according to another aspect of embodiments disclosed herein, there is provided a pharmaceutical composition comprising at least two agents and a pharmaceutically acceptable carrier, the at least two agents being capable of exhibiting at least two of the three activities described herein.

As used herein, a "pharmaceutical composition" refers to a preparation of one or more agents (as active ingredient(s)) as described herein, or physiologically acceptable salts or prodrugs thereof, with other chemical components, including, but not limited to, physiologically suitable carriers, excipients, lubricants, buffering agents, antibacterial agents, bulking agents (e.g. mannitol), antioxidants (e.g., ascorbic acid or sodium bisulfite), and the like. The purpose of the pharmaceutical composition is to facilitate administration of the agent(s) to a subject.

Herein, the phrases "physiologically acceptable carrier" and "pharmaceutically acceptable carrier", which are used interchangeably, describe a carrier or a diluent that does not cause significant irritation to the subject and does not abrogate the biological activity and properties of the agent(s) described herein.

As used herein, the term "carrier" refers to a diluent, adjuvant, excipient, or vehicle with which the therapeutic is administered.

Herein the term "excipient" refers to an inert substance added to a pharmaceutical composition to further facilitate administration of an active ingredient.

As disclosed herein, the at least two agents may optionally be selected from among any of the agents as described herein, as well as from among any combination of at least two agents as described herein. As described herein, such combinations may optionally exhibit any two of the three activities described herein, and may optionally exhibit all three of the activities described herein.

In some embodiments, the agents are selected so as to be suitable for administration via the same route.

In some embodiments, the agents are selected so as to be suitable for oral administration. It is to be appreciated that VB-201 is suitable for oral administration.

In some embodiments, the agents disclosed herein (e.g., agents which do not include VB-201 or a related compound) are formulated for a route of administration other than oral administration. For example, the agents may be formulated for parenteral administration, transmucosal administration, rectal administration and/or inhalation (e.g., as described herein).

Techniques for formulation and administration of drugs may be found in "Remington's Pharmaceutical Sciences" Mack Publishing Co., Easton, PA, latest edition.

Pharmaceutical compositions of embodiments of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more pharmaceutically acceptable carriers comprising excipients and auxiliaries, which facilitate processing of the agent(s) described herein into preparations which, can be used pharmaceutically. Proper formulation is dependent upon the route of administration chosen.

For injection, the agent(s) may be formulated in aqueous solutions, preferably in physiologically compatible buffers such as Hank's solution, Ringer's solution, or physiological saline buffer with or without organic solvents such as propylene glycol, polyethylene glycol.

For transmucosal administration, penetrants are used in the formulation. Such penetrants are generally known in the art.

Pharmaceutical compositions for use in accordance with the present invention thus may be formulated for oral administration in a conventional manner using one or more pharmaceutically acceptable carriers comprising excipients and auxiliaries, which facilitate processing of the compounds into preparations which can be used pharmaceutically.

A pharmaceutical composition according to some embodiments can be formulated readily by combining agents described herein with pharmaceutically acceptable carriers well known in the art. Using such carriers the agent(s) is formulated, for example, as sachets, pills, caplets, capsules, tablets, dragee-cores or discrete (e.g., separately packaged) units of powder, granules, or suspensions or solutions in water or non-aqueous media. Thickeners, diluents, flavorings, dispersing aids, emulsifiers or binders may be desirable.

Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active doses.

Pharmaceutical compositions, which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredient may be dissolved or suspended in suitable liquids, such as fatty oils, liquid paraffin, or liquid polyethylene glycols. In addition, stabilizers may be added. All formulations for oral administration should be in dosages suitable for the chosen route of administration.

Preferably, formulations for oral administration further include a protective coating, aimed at protecting or slowing enzymatic degradation of the preparation in the GI tract.

For buccal administration, the compositions may take the form of tablets or lozenges formulated in conventional manner.

For administration by inhalation, the agent(s) for use according to the present invention are conveniently delivered in the form of an aerosol spray presentation (which typically includes powdered, liquefied and/or gaseous carriers) from a pressurized pack or a nebulizer, with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges of, e.g., gelatin for use in an inhaler or insufflator may be formulated containing a powder mix of the agent(s) and a suitable powder base such as, but not limited to, lactose or starch.

The agent(s) described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the agent(s) preparation in water-soluble form. Additionally, suspensions of the agent(s) may be prepared as appropriate oily injection suspensions and emulsions (e.g., water-in-oil, oil-in-water or water-in-oil in oil emulsions). Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents, which increase the solubility of the agent(s) to allow for the preparation of highly concentrated solutions.

Alternatively, the agent(s) may be in powder form for constitution with a suitable vehicle, e.g., sterile, pyrogen-free water, before use.

The agent(s) described herein may also be formulated in rectal compositions such as suppositories or retention enemas, using, e.g., conventional suppository bases such as cocoa butter or other glycerides.

The pharmaceutical compositions herein described may also comprise suitable solid of gel phase carriers or excipients. Examples of such carriers or excipients include, but are not limited to, calcium carbonate, calcium phosphate, various sugars, starches, cellulose derivatives, gelatin and polymers such as polyethylene glycols.

In one embodiment, VB-201 to be administered to the subject is formulated for oral administration, e.g., in a liquid-fill hard-gelatin capsule. Exemplary VB-201 formulations useful in the context of this disclosure are described in PCT/US2012/053533 to Sher et al.

In another embodiment, VB-201 is formulated using a thermosoftening carrier selected from a poloxamer (e.g., poloxamer 188) and a polyethylene glycol having a molecular weight from about 6000 to about 8000 (e.g., PEG6000), an anti-adherent agent (e.g., talc) at a weight ratio from about 1:4 to about 1:1 (anti-adherent agent:VB-201), and a thixotropic agent (e.g., fumed silicon dioxide) at a concentration relative to the combined weight of the thermosoftening carrier and the thixotropic agent from about 0.5 weight percent to about 5 weight percent (e.g., from about 1 weight percent to about 3 weight percent). In some examples according to any one of the embodiments of Methods 1-12 described herein, the VB-201 is administered to the subject using a formulation comprising a poloxamer (e.g., polaxamer 188) as a thermosoftening carrier, VB-201 from about 20 mg to about 80 mg, talc at a weight ratio of about 1:1 or at a weight ratio of about 1:4 (talc:VB-201), and fumed silicon dioxide as a thixotropic agent at a concentration relative to the combined weight of the poloxamer and the fumed silicon dioxide from about 1 weight percent to about 3 weight percent.

### Thermosoftening Carrier

As used herein, the term "thermosoftening carrier" refers to a carrier which becomes soft (e.g., a fluid) upon heating to a temperature above room temperature. A thermosoftening carrier becomes soft at a temperature which does not damage the active pharmaceutical ingredient (e.g., by oxidation) or the thermosoftening carrier itself. The softening upon heating may be either characterized by a phase transition (e.g., a solid-to-liquid transition), or not characterized by a phase transition (e.g., softening of an amorphous material). The thermosoftening is reversible, such that the softened carrier becomes harder upon being cooled back to room temperature. In some embodiments, the thermosoftening carrier is a mixture of two or more agents.

The thermosoftening carrier facilitates preparation of a liquid fill composition and filling of capsules therewith at a temperature at which the thermosoftening carrier is soft, as well as formation of a solid or semi-solid matrix following cooling (e.g., cooling to room temperature). In one example, the thermosoftening carrier is a solid or a semi-solid at a temperature below 35 °C, or below 30 °C (e.g., at room temperature, i.e., 25 °C). In one example, the thermosoftening carrier is non-hygroscopic. The thermosoftening carrier is a pharmaceutically acceptable carrier.

Optionally, the thermosoftening carrier becomes soft at a temperature of no more than about 150 °C, and optionally at a temperature of no more than about 100 °C, or 90 °C.

In some embodiments, the thermosoftening carrier has a melting point in a range of from about 40 °C to about 100 °C. Optionally, the melting point is in a range of from about 50 °C to about 80 °C. In other examples, the melting point of the thermosoftening carrier is from about 50 °C to about 70 °C, or from about 50 °C to about 60 °C,, and optionally in a range of from about 55 °C to about 65 °C. Accordingly, at such temperatures, the thermosoftening carrier undergoes transformation from a hard to a soft material, and *vice versa.* In one example, the thermosoftening carrier at a temperature above its melting point is sufficiently soft for filling the carrier into a capsule (e.g., into a hard gelatin capsule).

Examples of thermosoftening carriers include waxes, poloxamers (e.g., Poloxamer 188), macrogol glycerides, high-molecular weight PEGs (e.g., PEG6000 or PEG 8000), glycerol monooleates or monostearates, hydrogenated or partially hydrogenated glycerides (e.g., hydrogenated palm kernel oil or hydrogenated cotton seed oil)), Gelucires™, and hard fats such as beeswax. Other exemplary thermosoftening carriers include Softisan™ and hexadecane-1-ol.

In some embodiments, the polyalkylene glycol is a poloxamer. Accordingly, in some embodiments, the thermosoftening carrier is a poloxamer.

Poloxamers are triblock polyalkylene glycols, comprising a central polypropylene glycol chain, which is relatively hydrophobic, flanked by two polyethylene glycol chains, which are relatively hydrophilic. This combination of hydrophobic and hydrophilic chains provides poloxamers with surfactant properties.

Poloxamers are typically characterized by molecular weight of the polypropylene glycol core of the poloxamer and by the proportion of polyethylene glycol versus polypropylene glycol. These parameters are commonly described by characterizing a poloxamer with a three-digit number, wherein the first two digits, when multiplied by 100, give the molecular weight (in daltons) of the polypropylene glycol core, whereas the last digit, when multiplied by 10, gives the percentage of polyethylene glycol. Thus, for example, poloxamer 188 has a polypropylene glycol core with a molecular weight of 1800 daltons and is 80 % polyethylene glycol (and thus has a total molecular weight of approximately 9000 daltons), whereas poloxamer 407 has a polypropylene glycol core with a molecular weight of 4000 daltons and is 70 % polyethylene glycol (and thus has a total molecular weight of approximately 13000 daltons).

Poloxamer 188 is an exemplary poloxamer. Accordingly, in some embodiments, the thermosoftening carrier is poloxamer 188.

In one embodiment, the thermosoftening carrier is selected from PEG6000, poloxamer 188, and combinations thereof.

The thermosoftening carrier may also comprise an oil or a combination of one or more oils. Many oils suitable for use as a thermosoftening carrier for therapeutic applications are known in the art. Examples include, without limitation, esters of fatty acids, such as triglycerides and diesters of a glycol (e.g., propylene glycol). Other oils may be added to the thermosoftening carrier to decrease/fine tune viscosity, e.g., fractioned coconut oil or soybean oil.

### Anti-Adherent Agent

As used herein, the phrase "anti-adherent agent" refers to an agent which reduces the cohesion between particles of a substance (e.g., VB-201) and/or an adherence of such particles to a solid surface (e.g., of a container and/or encapsulation machinery). For example, the reduction of cohesion caused by an anti-adherent agent is greater than a reduction of cohesion caused by mere dilution of the substance by addition of an agent.

Optionally, the anti-adherent agent is a material (e.g., a solid, such as a powder) with little or no solubility in the other components of the capsule (e.g., the thermosoftening carrier). The anti-adherent agent may act by adhering to the VB-201 thereby forming, e.g., grains and/or powder particles. As a result, the adherence of the VB-201 to other surfaces (e.g., other VB-201 grains and/or powder particles, surfaces of containers and/or encapsulation machinery) is reduced.

In exemplary embodiments the anti-adherent agent to VB-201 ratio is about 1:1 or 1:4.

Examples of anti-adherent agents include, but are not limited to, talc, magnesium stearate, cellulose (e.g., microcrystalline cellulose), cellulose derivatives (e.g., hydroxypropyl methylcellulose (HPMC)), lactose, gelatin, alginates, aluminium hydroxide, magnesium oxide, clays, attapulgite, bentonite, carrageenan, copovidone, hectorite, polymethacrylates, sodium docusate, erythritol, povidones, croscarmellose sodium, dextrates, starches, iron oxide, kaolin, silicates (e.g., magnesium aluminium silicate), corn flour, sugars, calcium carbonate, magnesium carbonate, calcium phosphate, calcium sulfate, bicarbonates (e.g., of potassium or sodium), citrate salts (e.g., potassium citrate) and titanium dioxide.

In one example according to any of the embodiments described herein, the anti-adherent agent is talc. Any pharmaceutical-grade or food-grade talc (e.g., powdered talc) may be used. Exemplary grades of talc, which can be used in the pharmaceutical compositions, liquid-fill compositions, capsules and other are embodiments herein are disclosed in Dawoodbhai et al., "Pharmaceutical and Cosmetic Uses of Talc," Drug Development and Industrial Pharmacy, 16(16):2409-2429 (1990); and Dawoodbhai et al., "Glidants and Lubricant Properties of Several Types of Talcs," Drug Development and Industrial Pharmacy, 13(13):2441-2467 (1987). In some examples, the talc is powdered talc. In some examples, the talc is of USP grade. In other example, the talc is powdered talc and of USP grade.

### Thixotropic Agent

As used herein, a "thixotropic agent" refers to an agent which increases a viscosity of a liquid when added to a liquid. As known in the art "thixotropy" is a reversible behaviour of viscous liquids (e.g., gels) that liquefy when subjected to shear stress such as shaking or stirring, or otherwise disturbed.

A viscous liquid containing a thixotropic agent exhibits thixotropy, wherein the viscosity is reduced under stress (e.g., stirring, heating and/or application of shear forces). The ingredients in a liquid fill composition (e.g., carrier, VB-201, thixotropic agent, and/or anti-adherent agent) can therefore be readily mixed by stirring, as the viscosity is reduced during stirring, yet the fill composition is relatively resistant to separation of components, as the viscosity increases when stirring ceases.

Examples of thixotropic agents suitable for use in the context of the present embodiments include, but are not limited to, fumed silica (available, for example as Aerosils® and Cab-O-Sil® products), kieselguhr, gums (e.g., xanthan gum, guar gum, locust bean gum, alginates), cellulose derivatives (e.g., hydroxypropyl methyl cellulose), starches, polymers (e.g., polyvinyl alcohol, polyacrylates, hydrophobically-modified polyacrylates), emulsifiers, and clay derivatives (e.g., amine treated magnesium aluminum silicate, bentonite colloidal silicic acid, white smectite clays and bleaching earth, attapulgite, mica, synthetic magnesium phyllosilicates (Laponite), layered silicates, modified smectites, hectorite, and sepiolite. Optionally, the thixotropic agent comprises fumed silica and/or attapulgite.

The concentration of the thixotropic agent in the pharmaceutical composition (i.e., liquid-fill composition or matrix of the capsule) unless otherwise indicated is determined relative to the combined weight of the thermosoftening carrier and the thixotropic agent. For example, at 2.5 weight percent of thixotropic agent, the pharmaceutical composition may contain 10 mg thixotropic agent and 390 mg of a thermosoftening carrier (10/400 = 2.5 %).

In one example according to any of the embodiments described herein, the thixotropic agent is a different substance than the thermosoftening agent (i.e., the thixotropic agent is chemically distinct from the thermosoftening agent). In other examples according to any of the embodiments described herein, the thixotropic agent is a different substance than the anti-adherent agent (i.e., the thixotropic agent is chemically distinct from the anti-adherent agent). In other examples according to any of the embodiments described herein, the thixotropic agent is a different substance than the thermosoftening agent and the anti-adherent agent (i.e., the thixotropic agent is chemically distinct from both the thermosoftening agent and the anti-adherent agent).

Determination of a therapeutically effective amount is well within the capability of those skilled in the art, especially in light of the detailed disclosure provided herein.

For any agent described herein, the therapeutically effective amount or dose, if not already known in the art, can be estimated initially from activity assays in animals. For example, a dose can be formulated in animal models to achieve a circulating concentration range that includes the IC₅₀ as determined by activity assays (e.g., the concentration of a protein inhibitor described herein, which achieves a half-maximal reduction of the activity of the protein to be inhibited). Such information can be used to more accurately determine useful doses in humans.

A pharmaceutical composition (e.g., a composition for oral administration) may optionally be formulated for slow release and/or delayed release of one or more agents in the composition.

Herein, "slow release" refers to gradual release of an active agent over a relatively long period of time following administration, optionally at least 1 hour, at least 2 hours, at least 4 hours, at least 8 hours, at least 16 hours, and optionally at least 24 hours.

Herein, "delayed release" refers to a formulation wherein release of an agent primarily occurs only after a considerably delay after administration, optionally after at least 1 hour, at least 2 hours, at least 4 hours, and optionally after at least 8 hours. When the delayed release occurs, release may be relatively sudden (e.g., a "burst" release) or a slow release.

Various formulations which provide slow and/or delayed release are known in the art, and can be selected to fit the chemical properties of an agent (e.g., hydrophobicity).

Slow and/or delayed release may be advantageous for agents which have a relatively short half-life in the body. Pharmacokinetic profiles of agents following administration may be determined by techniques known in the art, for example, determining levels of an agent in the blood at various intervals following administration.

Optionally, the time over which release of the agent occurs is selected so as to allow a relatively constant and therapeutic level of the agent in the blood when a composition is administered once daily or twice daily.

Slow and/or delayed release may be advantageous for agents for which it is undesirable to effect release in the stomach, including for example, agents which are sensitive to an acid, and agents which irritate the stomach.

In such embodiments, the time over which release of the agent occurs is optionally selected such that little agent is released before stomach clearance (e.g., within 2 hours).

Compositions may be formulated such that slow and/or delayed release is effected for some but not all of the agents therein. For example, a composition (e.g., a capsule, a tablet) may comprise two portions, wherein one portion (e.g., an inner portion) is characterized by slow release and/or delayed release, and a second portion (e.g., an outer portion) is characterized by regular release (e.g., immediate release).

In some embodiments, compositions that comprise two or more agents are formulated such that all agents are present at a maximum or near maximum plasma level substantially at the same time.

According to another aspect of embodiments disclosed herein, there is provided a kit comprising at least two agents, the at least two agents being capable of exhibiting at least two of the three activities described herein.

The at least two agents may optionally be selected from among any of the agents as described herein, as well as from among any combination of at least two agents as described herein. As described herein, such combinations may optionally exhibit any two of the three activities described herein, and may optionally exhibit all three of the activities described herein.

In some embodiments, each of the at least two agents is individually packaged within the kit. The agents described may be packaged *per se* or as part of a pharmaceutical composition, which may be formulated as described herein.

In alternative embodiments, at least some of the agents in the kit are combined in a pharmaceutical composition (e.g., a pharmaceutical composition described herein).

Compositions described herein may, if desired, be presented in a pack or dispenser device, such as an FDA (the U.S. Food and Drug Administration) approved kit (e.g., a kit as described herein), which may contain one or more unit dosage forms containing agents as described herein. The pack or dispenser device may, for example, comprise metal or plastic foil, such as, but not limited to a blister pack. The pack or dispenser device may be accompanied by instructions for administration. The pack may also be accompanied by a notice associated with the container in a form prescribed by a governmental agency regulating the manufacture, use or sale of pharmaceuticals, which notice is reflective of approval by the agency of the form of the compositions for human administration. Such notice, for example, may be of labeling approved by the U.S. Food and Drug Administration for prescription drugs or of an approved product insert. Compositions and kits described herein, comprising agents described herein (e.g., formulated in a compatible pharmaceutical carrier) may also be prepared, placed in an appropriate container, and identified (e.g., labeled) for treatment of an inflammatory disease or disorder, as defined herein.

In any of the aspects of embodiments disclosed herein, the inflammatory disease or disorder treatable according to said embodiments is optionally an inflammatory disease or disorder associated with an endogenous oxidized lipid.

As used herein, the phrase "an endogenous oxidized lipid" refers to one or more oxidized lipids that are present or formed *in vivo*, as a result of inflammatory and other cell- or humoral-mediated processes. Oxidized low-density lipoprotein (oxidized-LDL) is an example of an endogenous oxidized lipid associated with an inflammatory disease or disorder.

In another embodiment, VB-201 is administered to the subject in a formulation comprising: 40 mg VB-201, 40 mg of an anti-adherent agent (e.g., talc), 12 mg of a thixotropic agent (e.g., fumed silica), and 388 mg of a thermosoftening carrier (e.g., a poloxamer). In other examples, the VB-201 is administered to the subject in a formulation comprising: 40 mg VB-201, 40 mg talc, 12 mg of fumed silicon dioxide, and 388 mg of a poloxamer. In other examples, the VB-201 is administered to the subject in a formulation comprising: 40 mg VB-201, 10 mg of an anti-adherent agent (e.g., talc), 4 mg of a thixotropic agent (e.g., fumed silica), and 396 mg of a thermosoftening carrier (e.g., a poloxamer). In other examples, the VB-201 is administered to the subject in a formulation comprising: 40 mg VB-201, 10 mg talc, 4 mg fumed silicon dioxide, and 396 mg of poloxamer 188. In other examples, the VB-201 is administered to the subject in a formulation comprising: 80 mg VB-201, 80 mg of an anti-adherent agent (e.g., talc), 12 mg of a thixotropic agent (e.g., fumed silica), and 388 mg of a thermosoftening carrier (e.g., a poloxamer). In other examples, the VB-201 is administered to the subject in a formulation comprising: 80 mg VB-201, 80 mg talc, 12 mg fumed silica, and 388 mg of poloxamer 188. In other examples, the VB-201 is administered to the subject in a formulation comprising: 80 mg VB-201, 20 mg of an anti-adherent agent (e.g., talc), 4 mg of a thixotropic agent (e.g., fumed silica), and 396 mg of a thermosoftening agent. In other examples, the VB-201 is administered to the subject in a formulation comprising: 80 mg VB-201, 20 mg talc, 4 mg fumed silicon dioxide, and 396 mg of poloxamer 188.

### Treatment of Vascular Inflammation

### Method 1a

In various aspects disclosed herein, the present disclosure provides the compound VB-201 for use in a method of treating (*e.g*., decreasing) vascular inflammation (*e.g*., vascular inflammation associated with atherosclerotic lesions) in a subject in need thereof (*e.g*., a human patient), the method comprising administering to the subject a therapeutically effective amount of VB-201 (*e.g*., from about 20 mg/day to about 160 mg/day). In some examples, the subject suffers from a chronic autoimmune/inflammatory disease (*e.g*., psoriasis). Examples of therapeutically effective amounts of VB-201 useful in Method 1a are described herein.

### Method 1b

Thus, in other aspects disclosed herein, the present disclosure provides the compound VB-201 for use in a method of treating (*e.g*., decreasing) vascular inflammation (*e.g*., vascular inflammation associated with atherosclerotic lesions) in a subject in need thereof (*e.g*., a human patient), wherein the subject suffers from a chronic autoimmune or chronic inflammatory disease (*e.g*., psoriasis), the method comprising administering to the subject a therapeutically effective amount of VB-201 (*e.g*., from about 20 mg/day to about 160 mg/day). Examples of therapeutically effective amounts of VB-201 useful in Method 1b are described herein.

In some examples according to Methods 1a and 1b, the subject suffers from psoriasis. In other examples according to Methods 1a and 1b, the therapeutically effective amount of VB-201 (*e.g*., from about 20 mg/day to about 160 mg/day) is administered to the subject for at least about 8 weeks (*e.g*., at least about 10 weeks, or at least about 12 weeks).

### Method 2

In other aspects disclosed herein, the present disclosure provides the compound VB-201 for use in a method of treating (*e.g*., decreasing) vascular inflammation in a human subject suffering from a chronic autoimmune or chronic inflammatory disease (*e.g*., psoriasis), the method comprising administering to the subject a therapeutically effective amount of VB-201 (*e.g*., from about 20 mg/day to about 160 mg/day) for at least about 8 weeks (*e.g*., at least about 10 weeks, or at least about 12 weeks). In some examples, the vascular inflammation in the subject is reduced by at least about 10% as compared to vascular inflammation in the subject prior to the administering the VB-201 to the subject (relative to baseline). Examples of therapeutically effective amounts of VB-201 useful in Method 2 are described herein. Alternate percentages of reduction of vascular inflammation in Method 2 are also described herein.

Effect of VB-201 on vascular inflammation in patients treated with statins undergoing statin therapy prior to the onset of treatment with VB-201.

In some instances, the effect seen for VB-201 in patients undergoing statin therapy was even slightly better than in patients not being treated with statins indicating a possible synergy between statins and VB-201 with respect to decreasing vascular inflammation.

Thus, in some examples according to any one of the embodiments of Methods 1a, 1b, and 2 described herein, the subject underwent statin therapy prior to administering the VB-201 (e.g., wherein a therapeutically effective amount of a statin is administered to the subject during a time period immediately prior to first administering the VB-201). In other examples according to any one of the embodiments of Methods 1a, 1b, and 2, the subject is concomitantly treated with a statin.

### Method 3

In various aspects disclosed herein, the current disclosure further provides the compound VB-201 for use in a method of treating (*e.g*., decreasing) vascular inflammation, the method comprising administering to a subject in need thereof a therapeutically effective amount of VB-201 (*e.g*., from about 20 mg/day to about 160 mg/day), wherein the subject underwent statin therapy prior to administering the VB-201 (*e.g*., wherein a therapeutically effective amount of a statin is administered to the subject during a time period immediately prior to first administering the VB-201).

In one example according to any one of the embodiments of Methods 1a, 1b, 2, and 3 described herein, the subject underwent statin therapy (*i*.*e*., administration of a therapeutically effective amount of a statin) for at least about 2 weeks prior to first administering the VB-201. In other examples according to any one of the embodiments described herein, the subject underwent statin therapy for at least about 1 month prior to the administering of the VB-201. In other examples according to any one of the embodiments described herein, the subject underwent statin therapy for at least about 2 months prior to the administering of the VB-201. In other examples according to any one of the embodiments described herein, the subject underwent statin therapy for at least about 3 months prior to the administering of the VB-201. In other examples according to any one of the embodiments described herein, the subject underwent statin therapy for at least about 4 months prior to first administering the VB-201. In other example according to any one of the embodiments described herein, the subject underwent statin therapy for at least about 5 months prior to the administering the VB-201. In other examples according to any one of the embodiments described herein, the subject underwent statin therapy for at least about 6 months prior to the administering the VB-201. In yet other examples according to any one of the embodiments described herein, the subject underwent statin therapy from about 1 week to about 2 months prior to first administering the VB-201.

In some examples according to any one of the embodiments of Methods 1a, 1b, 2, and 3, the subject continues undergoing statin therapy after the onset of treating the subject with the VB-201 (*i.e*., after first administering the VB-201 to the subject).

In some examples according to any one of the embodiments of Methods 1a, 1b, 2, and 3 described herein, the vascular inflammation is measured using positron emission computed tomography (PET/CT) imaging quantifying 18-fluorodeoxyglucose (18-FDG) uptake as a target to background ratio (TBR), e.g., as described herein, or in Example 8 of WO2011/083465.

### Method 4

Thus, disclosed herein is the compound VB-201 for use in a method of decreasing vascular inflammation in a subject, the method comprising administering to a subject a therapeutically effective amount of VB-201, wherein the therapeutically effective amount is about 20 mg/day to about 160 mg/day (*e.g*., 20 mg/day to about 80 mg/day, or about 80 mg/day to about 160 mg/day). In some examples the vascular inflammation after administering the therapeutically effective amount to the subject (*e.g*., for at least about 12 weeks) is reduced by at least about 10% as compared to vascular inflammation in the subject prior to the administering the VB-201 to the subject (relative to baseline). In some examples, the vascular inflammation is measured using positron emission computed tomography (PET/CT) imaging quantifying 18-fluorodeoxyglucose (18-FDG) uptake as a target to background ratio (TBR) (*e.g*., as described herein, or in Example 8 of WO2011/083465).

In some examples according to any one of the embodiments of Methods 1a, 1b, 2, 3, and 4 described herein, vascular inflammation in the subject is decreased within a relatively short treatment period (*e.g*., not more than about 12 weeks) during which vascular inflammation is reduced by a certain percentage (*e.g*., at least about 10 % compared to baseline). In some examples according to any of the embodiments described herein, the vascular inflammation (e.g., after administering the therapeutically effective amount to the subject for about 12 weeks or about 24 weeks) is reduced by at least about 5% when compared to the vascular inflammation prior to the administering (base line). In other examples according to any of the embodiments described herein, the vascular inflammation (e.g., after administering the therapeutically effective amount to the subject for about 12 weeks or about 24 weeks) is reduced by at least about 6% when compared to the vascular inflammation prior to the administering. In other examples according to any of the embodiments described herein, the vascular inflammation (e.g., after administering the therapeutically effective amount to the subject for about 12 weeks or about 24 weeks) is reduced by at least about 8% when compared to the vascular inflammation prior to the administering. In other examples according to any of the embodiments described herein, the vascular inflammation (e.g., after administering the therapeutically effective amount to the subject for about 12 weeks or 24 weeks) is reduced by at least about 10% when compared to the vascular inflammation prior to the administering. In other examples according to any of the embodiments described herein, the vascular inflammation (e.g., after administering the therapeutically effective amount to the subject for about 12 weeks or 24 weeks) is reduced by at least about 12% when compared to the vascular inflammation prior to the administering. In other examples according to any of the embodiments described herein, the vascular inflammation (e.g., after administering the therapeutically effective amount to the subject for about 12 weeks or 24 weeks) is reduced by at least about 14% when compared to the vascular inflammation prior to the administering. In other examples according to any of the embodiments described herein, the vascular inflammation (e.g., after administering the therapeutically effective amount to the subject for about 12 weeks or 24 weeks) is reduced by at least about 16% when compared to the vascular inflammation prior to the administering. In other examples according to any of the embodiments described herein, the vascular inflammation (e.g., after administering the therapeutically effective amount to the subject for about 12 weeks or 24 weeks) is reduced by at least about 18% when compared to the vascular inflammation prior to the administering. In other examples according to any of the embodiments described herein, the vascular inflammation (e.g., after administering the therapeutically effective amount to the subject for about 12 weeks or 24 weeks) is reduced by at least about 20% when compared to the vascular inflammation prior to the administering.

In some examples according to any one of the embodiments of Methods 1a, 1b, 2, 3, and 4 described herein, the therapeutically effective amount is administered to the subject for at least about 8 weeks. In other examples according to any one of the embodiments described herein, the therapeutically effective amount is administered to the subject for about 8 weeks or not more than about 8 weeks. In other examples according to any one of the embodiments described herein, the therapeutically effective amount is administered to the subject for at least about 12 weeks. In other examples according to any one of the embodiments described herein, the therapeutically effective amount is administered to the subject for about 12 weeks or not more than about 12 weeks. In other examples according to any one of the embodiments described herein, the therapeutically effective amount is administered to the subject for at least about 14 weeks. In other examples according to any one of the embodiments described herein, the therapeutically effective amount is administered to the subject for about 14 weeks or not more than about 14 weeks. In other examples according to any one of the embodiments described herein, the therapeutically effective amount is administered to the subject for at least about 16 weeks. In other examples according to any one of the embodiments described herein, the therapeutically effective amount is administered to the subject for about 16 weeks or not more than about 16 weeks. In other examples, the therapeutically effective amount is administered to the subject for at least about 18 weeks. In other examples, the therapeutically effective amount is administered to the subject for about 18 weeks or not more than about 18 weeks. In other examples, the therapeutically effective amount is administered to the subject for at least about 20 weeks. In other examples, the therapeutically effective amount is administered to the subject for about 20 weeks or not more than about 20 weeks. In other examples, the therapeutically effective amount is administered to the subject for at least about 24 weeks. In other examples, the therapeutically effective amount is administered to the subject for about 24 weeks or not more than about 24 weeks.

In other examples according to any one of the embodiments of Methods 1a, 1b, 2, 3, and 4 described herein, the subject has an elevated high sensitivity C-reactive protein (hs-CRP) level prior to first administering the VB-201.

In other examples according to any one of the embodiments of Methods 1a, 1b, 2, 3, and 4 described herein, the subject does not have an elevated high sensitivity C-reactive protein (hs-CRP) level prior to first administering the VB-201.

In other examples according to any one of the embodiments of Methods 1a, 1b, 2, 3, and 4 described herein, the subject has not been on a stable high dose of statin (e.g., ≥20 mg/day atorvastatin; or ≥10 mg/day rosuvastatin; or ≥40 mg/day simvastatin). In another example according to any one of the described embodiments, the subject underwent statin therapy with less than a high dose of statin (e.g., less than 20 mg/day atorvastatin; or less than 10 mg/day rosuvastatin; or less than 40 mg/day simvastatin). In another example according to any one of the described embodiments, the subject underwent statin therapy for less than 3 months prior to first administering the VB-201.

In some examples according to any one of the embodiments of Methods 1a, 1b, 2, 3, and 4 described herein, the vascular inflammation is inflammation of a carotid artery. In another embodiment, the vascular inflammation is inflammation of an aorta.

In some examples according to any one of the embodiments of Methods 1a, 1b, 2, 3, and 4 described herein, the vascular inflammation is associated with atherosclerosis (*i.e*., the subject suffers from atherosclerosis). In another embodiment according to any of the embodiments described herein, the vascular inflammation is associated with cardiovascular disease (*i.e*., the subject suffers from a cardiovascular disease). In yet other examples according to any one of the embodiments described herein, the vascular inflammation is associated with psoriasis (*i.e*., the subject suffers from psoriasis).

In some examples according to any one of the embodiments of Methods 1a, 1b, 2, 3, and 4 described herein, the therapeutically effective amount is from about 5 mg/day to about 240 mg/day, or from about 10 mg/day to about 240 mg/day. In other examples according to any one of the described embodiments, the therapeutically effective amount is from about 20 mg/day to about 240 mg/day, or from about 40 mg/day to about 240 mg/day. In other examples according to any one of the described embodiments, the therapeutically effective amount is from about 20 mg/day to about 200 mg/day, or from about 20 mg/day to about 180 mg/day. In other examples according to any one of the described embodiments, the therapeutically effective amount is from about 10 mg/day to about 160 mg/day, or from about 20 mg/day to about 160 mg/day. In other examples according to any one of the embodiments described herein, the therapeutically effective amount is from about 40 mg/day to about 160 mg/day.

In other examples according to any one of the embodiments described herein, the therapeutically effective amount is from about 40 mg/day to about 160 mg/day, or from about 50 mg/day to about 160 mg/day. In other examples according to any one of the embodiments described herein, the therapeutically effective amount is from about 60 mg/day to about 160 mg/day. In other examples according to any one of the described embodiments, the therapeutically effective amount is from about 80 mg/day to about 160 mg/day. In other examples according to any one of the embodiments described herein, the therapeutically effective amount is about 100 mg/day to about 160 mg/day, or from about 120 mg/day to about 160 mg/day. In other examples according to any one of the embodiments described herein, the therapeutically effective amount is about 80 mg/day. In other examples according to any one of the embodiments described herein, the therapeutically effective amount is about 120 mg/day. In other examples according to any one of the embodiments described herein, the therapeutically effective amount is about 160 mg/day.

In other examples according to any one of the embodiments described herein, the therapeutically effective amount is from about 20 mg/day to about 120 mg/day. In other examples according to any one of the embodiments described herein, the therapeutically effective amount is from about 20 mg/day to about 100 mg/day. In other examples according to any one of the embodiments described herein, the therapeutically effective amount is from about 20 mg/day to about 120 mg/day. In some examples according to any one of the embodiments described herein, the therapeutically effective amount is from about 20 mg/day to about 80 mg/day. In some examples according to any one of the embodiments described herein, the therapeutically effective amount is from about 40 mg/day to about 80 mg/day.

In some examples according to any one of the embodiments described herein, wherein the VB-201 is administered at a daily dose of more than about 80 mg/day (e.g., 120 mg/day or 160 mg/day) then the total VB-201 dose is administered in at least two daily sub-doses, e.g., one in the morning and one in the evening with about 12 hours between sub-doses, e.g., every 12 hours (Q12H). In some examples according to any of the embodiments described herein, when the VB-201 is administered at a daily dose of about 120 mg/day, the VB-201 is administered in two sub-doses of 40 mg and 80 mg (e.g., 40 mg in the morning and 80 mg in the evening, or 80 mg in the morning and 40 mg in the evening) or in two sub-doses of 60 mg each. In other examples according to any of the embodiments described herein, when the VB-201 is administered at a daily dose of about 160 mg/day, the VB-201 is administered in two equal sub-doses of about 80 mg each (e.g., Q12H).

In one embodiment according to any of the embodiments described herein, the therapeutically effective amount is about 20 mg/day administered to the subject in 1 or 2 daily doses. In other examples according to any of the embodiments described herein, the therapeutically effective amount is about 20 mg/day administered to the subject in a single daily dose. In other examples according to any of the embodiments described herein, the therapeutically effective amount is about 40, 60, or 80 mg/day administered to the subject in 1 or 2 daily doses. In other examples according to any of the embodiments described herein, the therapeutically effective amount is about 80 mg/day administered to the subject in a single daily dose.

### Method 5

In some embodiments disclosed herein, the present disclosure provides the compound VB-201 for use in a method of treating (e.g., decreasing) vascular inflammation (*e.g*., vascular inflammation associated with atherosclerotic lesions) in a subject in need thereof (*e.g*., a human patient), the method comprising administering to the subject a therapeutically effective amount of VB-201, wherein the therapeutically effective amount of VB-201 is from about 120 mg/day to about 160 mg/day (e.g., 160 mg/day), and wherein the therapeutically effective amount is administered to the subject in at least two daily sub-doses, wherein each sub-dose is 80 mg or less.

In some examples of Method 5, the total VB-201 dose is administered in two sub-doses, e.g., one in the morning and one in the evening with about 12 hours between sub-doses, e.g., every 12 hours (Q12H). In some examples according to any of the embodiments of Method 5 described herein, when the VB-201 is administered at a daily dose of about 120 mg/day, the VB-201 is administered in two sub-doses of 40 mg and 80 mg (e.g., 40 mg in the morning and 80 mg in the evening, or 80 mg in the morning and 40 mg in the evening), or is administered in two equal sub-doses of 60 mg each. In other examples according to any of the embodiments of Method 5 described herein, when the VB-201 is administered at a daily dose of about 160 mg/day, the VB-201 is administered in two equal sub-doses of about 80 mg each (e.g., Q12H).

### Method 6

In some embodiments disclosed herein, the present disclosure provides the compound VB-201 for use in methods of treating severe psoriasis, wherein severe psoriasis is psoriasis of category 4 according to the Physician Global Assessment (PGA) scale. In some embodiments, the method comprises administering to a subject in need thereof a therapeutically effective amount of VB-201 as defined herein (e.g., the therapeutically effective amount is from about 80 mg/day to about 160 mg/day) for a treatment period. In some examples, the severe psoriasis improves to moderate, mild, almost clear, or no psoriasis (psoriasis of categories 0-3 according to PGA scale) during the treatment period. In some examples according to Method 4, the therapeutically effective amount is administered to the subject for a treatment period of at least about 8 weeks (e.g., 8 weeks, 12 weeks, 16 weeks, 20 weeks, or 24 weeks).

### Method 7

In other embodiments disclosed herein, the present disclosure provides the compound VB-201 for use in a method of treating moderate to severe psoriasis, wherein moderate to severe psoriasis is psoriasis of categories 3 and 4 according to the Physician Global Assessment (PGA) scale. The method comprises administering to a subject in need thereof a therapeutically effective amount of VB-201, wherein the therapeutically effective amount is defined herein (e.g., the therapeutically effective amount is from about 80 mg/day to about 160 mg/day). The therapeutically effective amount is administered to the subject for a treatment period of at least about 8 weeks (e.g., 8 weeks, 12 weeks, 16 weeks, 20 weeks, or 24 weeks). In some examples, the psoriasis improves to mild, almost clear or no psoriasis (psoriasis of categories 0-2 according to PGA scale) during the treatment period.

### Method 8

In other embodiments disclosed herein, the present disclosure provides the compound VB-201 for use in a method of treating moderate, severe, or worse than severe psoriasis, which is psoriasis of categories 3 to 5 according to the Patient Global Assessment (PtGA) scale. The method comprises administering to a subject in need thereof a therapeutically effective amount of VB-201, wherein the therapeutically effective amount is defined herein (e.g., the therapeutically effective amount is from about 80 mg/day to about 160 mg/day) for a treatment period of at least about 8 weeks (e.g., 8 weeks, 12 weeks, 16 weeks, or 24 weeks). In some examples, the severe psoriasis improves to mild, almost clear or no psoriasis (psoriasis of categories 0 to 2 according to the PtGA scale) during the treatment period.

### PASI Score

In some examples according to any one of the embodiments described herein, the subject, prior to the administering the VB-201, has a PASI score of at least about 10 and not more than about 20 (moderate to severe psoriasis based on PASI score). In some examples according to any one of the embodiments described herein, the subject, prior to the administering the VB-201, has a PASI score of less than about 10. In some examples according to any one of the embodiments described herein, the subject, prior to the administering the VB-201, has a PASI score of less than about 14.3. In other examples according to any of the embodiments described herein, the subject, prior to the administering of VB-201, has a PASI score that is from about 10 to about 20. In another example according to any of the embodiments described herein, the subject, prior to the administering of VB-201, has a PASI score that is from about 11 to about 20, or from about 12 to about 20, or from about 13 to about 20, or from about 14 to about 20, or from about 15 to about 20, or from about 10 to about 19, or from about 11 to about 19, or from about 12 to about 19, or from about 13 to about 19, or from about 14 to about 19, or from about 10 to about 18, or from about 11 to about 18, or from about 12 to about 18, or from about 13 to about 18, or from about 14 to about 18, or about 15 to about 18. In other examples according to any one of the embodiments described herein, the subject, prior to the administering of VB-201, has a PASI score that is from about 14.3 to about 18.5. In other examples according to any of the embodiments described herein, the subject, prior to the administering of VB-201, has a PASI score of greater than about 18.5.

### Method 9a

As disclosed herein, the present disclosure further provides the compound VB-201 for use in a method of treating psoriasis, the method comprising administering to a subject in need thereof a therapeutically effective amount of VB-201, wherein the subject prior to the administering the VB-201 has a PASI score that is below 14.3, e.g., from about 10 to about 14 (e.g., from about 10 to about 13, or from about 10 to about 12, or from about 10 to about 11).

### Method 9b

As disclosed herein, the present disclosure further provides the compound VB-201 for use in a method of treating psoriasis, the method comprising administering to a subject in need thereof a therapeutically effective amount of VB-201, wherein the subject prior to the administering the VB-201 has a PASI score that is from about 10 to about 20 (e.g., from about 14 to about 20, or from about 14 to about 19, or from about 14.3 to about 18.5) (e.g., moderate psoriasis). Other suitable ranges for the PASI score are disclosed herein.

### Method 9c

As disclosed herein, the present disclosure further provides the compound VB-201 for use in a method of treating psoriasis, the method comprising administering to a subject in need thereof a therapeutically effective amount of VB-201, wherein the subject prior to the administering has a PASI score that is from about 18 to about 20 (e.g., from about 18.5 to about 20, or from about 19 to about 20).

### Body Surface Area

As disclosed herein, in some examples according to any of the embodiments described herein, the subject, prior to the administering of the VB-201, has psoriasis (e.g., plaque psoriasis) characterized by (covering) a body surface area (BSA) of from about 10% to about 30%. As disclosed herein, in some examples according to any of the embodiments described herein, the subject, prior to the administering, has psoriasis characterized by a BSA of less than or equal to about 16%, e.g., from about 10% to about 16%. As disclosed herein, in other examples according to any of the embodiments described herein, the subject, prior to the administering, has psoriasis characterized by a BSA from about 10% to about 28%, or from about 10% to about 26%, or from about 10% to about 24%, or from about 12% to about 30%, or about 14% to about 30%, or about 16% to about 30%, or from about 12% to about 28%, or about 14% to about 28%, or about 16% to about 28%, or from about 12% to about 26%, or about 14% to about 26%, or about 16% to about 26%, or from about 12% to about 24%, or from about 14% to about 24%, or from about 16% to about 24%. As disclosed herein, in other examples according to any of the embodiments described herein, the subject, prior to the administering, has psoriasis characterized by a BSA of greater than or equal to about 24%, e.g., from about 24% to about 30%, from about 26% to about 30%, or from about 28% to about 30%.

### Method 10a

As disclosed herein, the present disclosure further provides the compound VB-201 for use in a method of treating psoriasis, the method comprising administering to a subject in need thereof a therapeutically effective amount of VB-201, wherein the subject prior to the administering the VB-201 has a BSA of less than or equal to about 16% (e.g., from about 10% to about 16%).

### Method 10b

As disclosed herein, the present disclosure further provides the compound VB-201 for use in a method of treating psoriasis, the method comprising administering to a subject in need thereof a therapeutically effective amount of VB-201, wherein the subject prior to the administering the VB-201 has a BSA from about 10% to about 30% (e.g., from about 14% to about 26%, or from about 16% to about 24%). Other ranges for BSA are disclosed herein.

### Method 10c

As disclosed herein, the present disclosure further provides the compound VB-201 for use in a method of treating psoriasis, the method comprising administering to a subject in need thereof a therapeutically effective amount of VB-201, wherein the subject prior to the administering of the VB-201 has a BSA of greater than about 24%.

### Prior Treatment with Biologics or Immunosuppressants

As disclosed herein, in some examples according to any of the embodiments described herein, the subject, prior to the administering, has not been treated with a biologic psoriasis treatment. The term biologic, biologics, or biologic psoriasis treatment means any biologic drug useful for the treatment of inflammation and/or autoimmune diseases, e.g., any form of psoriasis. Such biologics include, e.g., alefacept, which blocks molecules that dendritic cells use to communicate with T cells and causes natural killer cells to kill T cells as a way of controlling inflammation. Several monoclonal antibodies (MAbs) target inflammatory cytokines. TNF-α is one of the main executor inflammatory cytokines. Four MAbs (infliximab, adalimumab, golimumab and certolizumab pegol) and one recombinant TNF-α decoy receptor, etanercept, have been developed against TNF-α to inhibit TNF-α signaling. Additional monoclonal antibodies have been developed against pro-inflammatory cytokines IL-12/IL-23 and Interleukin-17. The biologic drug adalimumab (Humira) was approved to treat moderate to severe psoriasis. Another biologic that has been approved for the treatment of moderate to severe psoriasis is ustekinumab (Stelara), an IL-12/IL-23 blocker.

### Method 11

As disclosed herein, the present disclosure further provides the compound VB-201 for use in a method of treating psoriasis, the method comprising administering to a subject in need thereof a therapeutically effective amount of VB-201, wherein the subject was not treated with an anti-psoriatic biologic (e.g., did not undergo psoriasis treatment with a biologic) prior to first administering the VB-201 (e.g., during any time period prior to first administering the VB-201, or during a minimum time period immediately prior to first administering the VB-201). For example, the subject did not undergo psoriasis treatment with a biologic for at least about 2 months, at least about 4 months, at least about 6 months, at least about 8 months, at least about 10 months, at least about 12 months, at least about 18 months, at least about 24 months, or at least about 32 months prior to first administering the VB-201). In another example, the subject has never received anti-psoriatic biologic treatment prior to first administering the VB-201.

In other examples according to any of the embodiments described herein, the subject, prior to the administering, has not been treated with an immunosuppressant drug. The term "immunosuppressant" includes all drug molecules known to lessen the immune reaction in a subject (e.g., a human subject), e.g., drugs useful to treat auto-immune diseases, such as psoriasis. The term immunosuppressant includes biologics, such as immunosuppressant antibodies, as well as non-biologic immunosuppressants. Exemplary non-biologic "immunosuppressants" include antimetabolites, such folic acid analogues (e.g., methotrexate); purine analogues (e.g., azathioprine and mercaptopurine); pyrimidine analogues, protein synthesis inhibitors, cytotoxic antibiotics (e.g., dactinomycin, anthracyclines, mitomycin C, bleomycin, and mithramycin); calcineurin inhibitors (CNI) (e.g., ciclosporin, myriocin, tacrolimus, sirolimus), mycophenolate, and fingolimod.

### Method 12

Thus, further disclosed herein is the compound VB-201 for use in a method of treating psoriasis, the method comprising administering to a subject in need thereof a therapeutically effective amount of VB-201, wherein the subject was not treated with an immunosuppressant drug prior to first administering the VB-201 (e.g., during any time period prior to first administering the VB-201, or was not treated for at least a minimum time period prior to first administering the VB-201). For example, the subject did not undergo psoriasis treatment with an immunosuppressant for at least about 2 months, at least about 4 months, at least about 6 months, at least about 8 months, at least about 10 months, at least about 12 months, at least about 18 months, at least about 24 months, or at least about 32 months prior to first administering the VB-201). In another example, the subject has never received immunosuppressant treatment (e.g., for psoriasis or another reason) prior to first administering the VB-201.

### Treatment Period

In some examples according to any one of the embodiments described herein (e.g., any one of the embodiments of Methods 6 to 12 described herein), the treatment period is at least about 12 weeks. In other examples according to any one of the embodiments described herein, the treatment period is at least about 16 weeks. In other examples according to any of the embodiments described herein, the treatment period is at least about 24 weeks. In yet other examples according to any of the embodiments described herein, the subject is treated with the VB-201 for a treatment period between about 12 weeks and about 24 weeks.

As disclosed herein, in some examples according to any one of the embodiments described herein (e.g., any one of the embodiments of Methods 6 to 12 described herein), the psoriasis is moderate to severe, stable, active plaque *psoriasis vulgaris* (psoriasis). In some examples, the moderate to severe, stable, active plaque psoriasis affects between about 10% to about 30% of the body surface of the subject and is characterized by a Psoriasis Area and Severity Index (PASI) score from about 10 to about 20.

As disclosed herein, in other examples according to any of the embodiments described herein (e.g., any of the embodiments of Methods 6 to 12 described herein), the subject has a diagnosis of chronic plaque psoriasis for at least about 6 months prior to administering the VB-201 to the subject. Inflammatory diseases or disorders treatable according to exemplary embodiments disclosed herein include psoriasis (e.g., plaque psoriasis), rheumatoid arthritis, and atherosclerosis and related conditions, such as inflammation of an artery (e.g., inflammation of a carotid artery and/or inflammation of an aorta).

Additional examples of inflammatory diseases or disorders treatable according to exemplary embodiments disclosed herein include multiple sclerosis and inflammatory bowel disease (e.g., chronic inflammatory bowel disease).

Representative inflammatory diseases and disorders treatable according to any of the aspects of embodiments disclosed herein include, for example, idiopathic inflammatory diseases or disorders, chronic inflammatory diseases or disorders, acute inflammatory diseases or disorders, autoimmune diseases or disorders, infectious diseases or disorders, inflammatory malignant diseases or disorders, inflammatory transplantation-related diseases or disorders, inflammatory degenerative diseases or disorders, diseases or disorders associated with a hypersensitivity, inflammatory cardiovascular diseases or disorders, inflammatory cerebrovascular diseases or disorders, peripheral vascular diseases or disorders, inflammatory glandular diseases or disorders, inflammatory gastrointestinal diseases or disorders, inflammatory cutaneous diseases or disorders, inflammatory hepatic diseases or disorders, inflammatory neurological diseases or disorders, inflammatory musculo-skeletal diseases or disorders, inflammatory renal diseases or disorders, inflammatory reproductive diseases or disorders, inflammatory systemic diseases or disorders, inflammatory connective tissue diseases or disorders, inflammatory tumors, necrosis, inflammatory implant-related diseases or disorders, inflammatory aging processes, immunodeficiency diseases or disorders, proliferative diseases and disorders and inflammatory pulmonary diseases or disorders, as is detailed hereinbelow.

Non-limiting examples of hypersensitivities include Type I hypersensitivity, Type II hypersensitivity, Type III hypersensitivity, Type IV hypersensitivity, immediate hypersensitivity, antibody mediated hypersensitivity, immune complex mediated hypersensitivity, T lymphocyte mediated hypersensitivity, delayed type hypersensitivity, helper T lymphocyte mediated hypersensitivity, cytotoxic T lymphocyte mediated hypersensitivity, TH1 lymphocyte mediated hypersensitivity, and TH2 lymphocyte mediated hypersensitivity.

Non-limiting examples of inflammatory cardiovascular disease or disorder include occlusive diseases or disorders, atherosclerosis, a cardiac valvular disease, stenosis, restenosis, in-stent-stenosis, myocardial infarction, coronary arterial disease, acute coronary syndromes, congestive heart failure, angina pectoris, myocardial ischemia, thrombosis, Wegener's granulomatosis, Takayasu's arteritis, Kawasaki syndrome, anti-factor VIII autoimmune disease or disorder, necrotizing small vessel vasculitis, microscopic polyangiitis, Churg and Strauss syndrome, pauci-immune focal necrotizing glomerulonephritis, crescentic glomerulonephritis, antiphospholipid syndrome, antibody induced heart failure, thrombocytopenic purpura, autoimmune hemolytic anemia, cardiac autoimmunity, Chagas' disease or disorder, and anti-helper T lymphocyte autoimmunity.

Stenosis is an occlusive disease of the vasculature, commonly caused by atheromatous plaque and enhanced platelet activity, most critically affecting the coronary vasculature.

Restenosis is the progressive re-occlusion often following reduction of occlusions in stenotic vasculature. In cases where patency of the vasculature requires the mechanical support of a stent, in-stent-stenosis may occur, re-occluding the treated vessel.

Non-limiting examples of cerebrovascular diseases or disorders include stroke, cerebrovascular inflammation, cerebral hemorrhage and vertebral arterial insufficiency.

Non-limiting examples of peripheral vascular diseases or disorders include gangrene, diabetic vasculopathy, ischemic bowel disease, thrombosis, diabetic retinopathy and diabetic nephropathy.

Non-limiting examples of autoimmune diseases or disorders include all of the diseases caused by an immune response such as an autoantibody or cell-mediated immunity to an autoantigen and the like. Representative examples are chronic rheumatoid arthritis, juvenile rheumatoid arthritis, systemic lupus erythematosus, scleroderma, mixed connective tissue disease, polyarteritis nodosa, polymyositis/dermatomyositis, Sjogren's syndrome, Bechet's disease, multiple sclerosis, autoimmune diabetes, Hashimoto's disease, psoriasis, primary myxedema, pernicious anemia, myasthenia gravis, chronic active hepatitis , autoimmune hemolytic anemia, idiopathic thrombocytopenic purpura, uveitis, vasculitides and heparin induced thrombocytopenia.

Non-limiting examples of inflammatory glandular diseases or disorders include pancreatic diseases or disorders, Type I diabetes, thyroid diseases or disorders, Graves' disease, thyroiditis, spontaneous autoimmune thyroiditis, Hashimoto's thyroiditis, idiopathic myxedema, ovarian autoimmunity, autoimmune anti-sperm infertility, autoimmune prostatitis and Type I autoimmune polyglandular syndrome.

Non-limiting examples of inflammatory gastrointestinal diseases or disorders include colitis, ileitis, Crohn's disease, chronic inflammatory intestinal disease, inflammatory bowel syndrome, inflammatory bowel disease, celiac disease, ulcerative colitis, an ulcer, a skin ulcer, a bed sore, a gastric ulcer, a peptic ulcer, a buccal ulcer, a nasopharyngeal ulcer, an esophageal ulcer, a duodenal ulcer and a gastrointestinal ulcer.

Non-limiting examples of inflammatory cutaneous diseases or disorders include acne, an autoimmune bullous skin disease, pemphigus vulgaris, bullous pemphigoid, pemphigus foliaceus, contact dermatitis and drug eruption.

Non-limiting examples of inflammatory hepatic diseases or disorders include autoimmune hepatitis, hepatic cirrhosis, and biliary cirrhosis.

Non-limiting examples of inflammatory neurological diseases or disorders include multiple sclerosis, Alzheimer's disease, Parkinson's disease, myasthenia gravis, motor neuropathy, Guillain-Barre syndrome, autoimmune neuropathy, Lambert-Eaton myasthenic syndrome, paraneoplastic neurological disease or disorder, paraneoplastic cerebellar atrophy, non-paraneoplastic stiff man syndrome, progressive cerebellar atrophy, Rasmussen's encephalitis, amyotrophic lateral sclerosis, Sydeham chorea, Gilles de la Tourette syndrome, autoimmune polyendocrinopathy, dysimmune neuropathy, acquired neuromyotonia, arthrogryposis multiplex, Huntington's disease, AIDS associated dementia, amyotrophic lateral sclerosis (AML), multiple sclerosis, stroke, an inflammatory retinal disease or disorder, an inflammatory ocular disease or disorder, optic neuritis, spongiform encephalopathy, migraine, headache, cluster headache, and stiff-man syndrome.

Non-limiting examples of inflammatory connective tissue diseases or disorders include autoimmune myositis, primary Sjogren's syndrome, smooth muscle autoimmune disease or disorder, myositis, tendinitis, a ligament inflammation, chondritis, a joint inflammation, a synovial inflammation, carpal tunnel syndrome, arthritis, rheumatoid arthritis, osteoarthritis, ankylosing spondylitis, a skeletal inflammation, an autoimmune ear disease or disorder, and an autoimmune disease or disorder of the inner ear.

Non-limiting examples of inflammatory renal diseases or disorders include autoimmune interstitial nephritis and/or renal cancer.

Non-limiting examples of inflammatory reproductive diseases or disorders include repeated fetal loss, ovarian cyst, or a menstruation associated disease or disorder.

Non-limiting examples of inflammatory systemic diseases or disorders include systemic lupus erythematosus, systemic sclerosis, septic shock, toxic shock syndrome, and cachexia.

Non-limiting examples of infectious disease or disorder include chronic infectious diseases or disorders, a subacute infectious disease or disorder, an acute infectious disease or disorder, a viral disease or disorder, a bacterial disease or disorder, a protozoan disease or disorder, a parasitic disease or disorder, a fungal disease or disorder, a mycoplasma disease or disorder, gangrene, sepsis, a prion disease or disorder, influenza, tuberculosis, malaria, acquired immunodeficiency syndrome, and severe acute respiratory syndrome.

Non-limiting examples of inflammatory transplantation-related diseases or disorders include graft rejection, chronic graft rejection, subacute graft rejection, acute graft rejection hyperacute graft rejection, and graft versus host disease or disorder. Exemplary implants include a prosthetic implant, a breast implant, a silicone implant, a dental implant, a penile implant, a cardiac implant, an artificial joint, a bone fracture repair device, a bone replacement implant, a drug delivery implant, a catheter, a pacemaker, an artificial heart, an artificial heart valve, a drug release implant, an electrode, and a respirator tube.

Non-limiting examples of inflammatory tumors include a malignant tumor, a benign tumor, a solid tumor, a metastatic tumor and a non-solid tumor.

Non-limiting examples of inflammatory pulmonary diseases or disorders include asthma, allergic asthma, emphysema, chronic obstructive pulmonary disease or disorder, sarcoidosis and bronchitis.

An example of a proliferative disease or disorder is cancer.

In any of the aspects of embodiments of the invention, the agents described herein may optionally be used in combination with one or more additional agent(s) for treating inflammation, for example, an agent known in the medical arts to be useful for treating inflammation.

The terms "comprises", "comprising", "includes", "including", "having" and their conjugates mean "including but not limited to".

The term "consisting of" means "including and limited to".

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

### MATERIALS AND METHODS

### Materials:

Amine-PEG2-biotin was obtained from Thermo Scientific;
anti-CD14 antibodies (1:500) were from Santa Cruz (Santa Cruz, CA);
anti-CD14 (human) fluorescein isothiocyanate (FITC)-labeled antibodies were obtained from eBioscience;
anti-ERK1/2 antibodies (1:5,000) were obtained from Cell Signaling Technologies (Danvers, MA);
anti-IκBα antibodies (1:1,000) were obtained from Cell Signaling Technologies;
anti-MyD88 antibodies (1:1,000) were obtained from Cell Signaling Technologies;
anti-phospho-ERK1/2 antibodies (1:10,000) were obtained from Sigma;
anti-phospho-IKKα/β antibodies (1:1,000) were obtained from Cell Signaling Technologies;
anti-phospho-p38 antibodies (1:1,000) were obtained from Cell Signaling Technologies;
anti-TLR2 antibodies (1:500), anti-TLR4 antibodies (1:200), and anti-TLR2 (human) phycoerythrin-labeled antibodies were obtained from R&D Systems;
anti-tubulin antibodies (1:5,000) were obtained from Sigma;
biotin-lipopolysaccharide was obtained from InvivoGen (San Diego, CA);
chemoattractants were obtained from Peprotech;
CpG (CpG ODN 1826) was obtained from InvivoGen;
EDC (1-ethyl-3-(3-dimethylaminopropyl)carbodiimide) was obtained from Thermo Scientific;
fetal bovine serum was obtained from Biological Industries Ltd. (Israel);
Ficoll-Paque plus medium was obtained from GE Healthcare;
flagellin was obtained from InvivoGen (San Diego, CA);
GW5074 was obtained from Sigma;
horseradish peroxidase (HRP)-donkey anti-rabbit antibodies (1:5,000) and HRP-goat anti-mouse antibodies (1:3,000) were obtained from Jackson ImmunoResearch;
horseradish peroxidase (HRP)-donkey anti-goat antibodies (1:5,000) were obtained from Santa Cruz;
HUVEC were freshly isolated from blood cords of donors and grown in EGM-2 medium (Lonza);
IL-1β (recombinant human) was obtained from PeproTech Asia;
lipopolysaccharide (LPS) from *E. coli* strain 055:B5 was obtained from Sigma;
ovalbumin was obtained from Sigma;
Pam3CSK4 was obtained from InvivoGen (San Diego, CA);
PAPC was obtained from Avanti Polar Lipids and oxidized for 24 or 72 hours prior to use, to prepare oxidized PAPC (Ox-PAPC), as indicated.
peptidoglycan from *S. aureus* (PGN-SA) was obtained from InvivoGen (San Diego, CA);
plasmids encoding human CD14 were obtained from Origene (Rockville, MD);
plasmids encoding human TLR2-HA were obtained from InvivoGen (San Diego, CA);
R848 was obtained from InvivoGen (San Diego, CA);
RPMI-1640 medium was obtained from Biological Industries Ltd. (Israel);
streptavidin-APC was obtained from eBioscience;
THP-1 cells were obtained from the ATCC;
Thioglycollate was obtained from Difco.
VB-201 ([(R)-1-hexadecyl-2-(4'-carboxy)butyl-*sn*-glycero-3-phosphocholine]) and VB-207 ((R)-1-octyl-2-(4'-carboxy)butyl-sn-glycero-3-phosphocholine) were synthesized as described previously.

### Radio-labeling of VB-201:

Radio-labeled VB-201 ([³H]₂-VB-201) was prepared by hydrogenation of the unsaturated precursor Δ⁹-VB-201 (1-(9'-cis-hexadecenyl)-2-(4'-carboxy)butyl-sn-glycero-3-phosphocholine) with tritium. Δ⁹-VB-201 was prepared as described previously.

### Labeling of VB-201 and VB-207 with ovalbumin-biotin:

To label VB-201 and VB207 with biotin, VB-201 and VB207 were first conjugated to ovalbumin using EDC (1-ethyl-3-(3-dimethylaminopropyl)carbodiimide) at a molar ratio of 100:1:240 (VB-201/VB-2017 : ovalbumin : EDC for 2-3 hours at room temperature. Samples were then transferred to 10 kDa dialysis cassettes (Thermo Scientific) and dialyzed against PBS (phosphate buffer saline) overnight. Ovalbumin-labeled VB201 and VB207 were then conjugated to amine-PEG2-biotin using EDC at a molar ratio of 1:100:700 (ovalbumin : amine-PEG2-biotin : EDC. The reaction was allowed to proceed for 2-3 hours at room temperature. Samples were then transferred to a 10 kDa dialysis cassettes and dialyzed against PBS overnight.

### Isolation of human CD14+ monocytes:

Venous blood samples were obtained from healthy female and male donors who signed an agreement to be tested, were not under steroid treatment, and had no viral or bacterial infection for at least one month before testing. PBMCs (peripheral blood mononuclear cells) were isolated on Ficoll-Paque PLUS density gradient centrifugation medium (GE Healthcare) using 50 ml Leucosep tubes (Greiner Bio One). Cells were washed in phosphate buffer saline (PBS), and incubated at 4 °C for 15 minutes in a buffer containing PBS, 0.5 % bovine serum albumin (BSA) and EDTA (ethylenediaminetetraacetic acid) with human CD14 microbeads (Miltenyi Biotec). Cells were then washed and resuspended in the same buffer, and CD14+ monocytes were then isolated through LS columns (Miltenyi Biotec).

### Human dendritic cells (DCs) derived from CD14+ monocytes:

To generate monocyte-derived dendritic cells, CD14+ monocytes (isolated as described above) were counted, washed and seeded at a concentration of 10⁶/ml, for 5-7 days, in RPMI-1640 medium with L-glutamine, β-mercaptoethanol, 10 % FCS (fetal calf serum), sodium pyruvate, non-essential amino acids, 0.01 M HEPES, antibiotics (penicillin and streptomycin), and 50 ng/ml of human GM-CSF (granulocyte-macrophage colony-stimulating factor) and 20 ng/ml of human IL-4. The medium was replaced every other day.

### Isolation of human CD4+ T cells and CD19+ B cells:

T cells and B cells were isolated using the same procedures described above for isolating CD14+ monocytes, except that human CD4 microbeads (Miltenyi Biotec) were used instead of CD14 microbeads to isolate T cells, and CD19 microbeads (Miltenyi Biotec) were used instead of CD14 microbeads to isolate B cells.

### Isolation of mouse leukocytes:

To generate mouse bone-marrow derived dendritic cells (BMDC), bone-marrow was flushed out with cold RPMI-1640 medium from mice femur and tibia. A cell suspension was prepared and erythrocytes were removed using red blood cell (RBC) lysis buffer (Beit Haemek, Israel). Cells were washed in phosphate buffer saline (PBS) (Beit Haemek, Israel), and incubated in 4 °C for 15 minutes in buffer containing PBS and 0.5 % bovine serum albumin (BSA) with mouse B220 and CD90 microbeads (Miltenyi biotech). Cells were then washed, resuspended in the same buffer, and depleted from on a Midi-Macs separation unit through a LD or LS column (Miltenyi biotech). The depleted cells were then counted, washed and seeded (10⁶/ml) in medium containing RPMI-1640, L-glutamine, β-mercaptoethanol, 10 % fetal calf serum (FCS), antibiotics (penicillin, streptomycin) and 20ng/ml of mouse GM-CSF (Peprotech, Israel). Medium was replaced every other day and cells were used for subsequent experiments on days 5-6 post culturing. Dendritic cells (DCs) were enriched using CD11c microbeads (Miltenyi biotech). Mouse splenic B and T cells were isolated using B220 and CD90 microbeads respectively.

### Radio-labeled VB-201 incorporation assay:

Cells (10⁶) were cultured for 2 hours with [³H]₂-VB-201 mixed with non-labeled VB-201 (3.4 µM). At the end of the 2 hour incubation, cells were collected and washed in PBS twice. Cells were re-suspended in 100 µl of lysis buffer, and 20 µl of the lysis buffer was evaluated for radioactivity using a β-counter (DPM).

### Plasmid transfection:

HEK 293 cells were transfected using Lipofectamine 2000 (Invitrogen) for 24-48 hours with plasmids encoding human CD14 and human TLR2-HA. Transfection efficiency was determined using fluorescein isothiocyanate (FITC)-labeled anti-human CD14 antibodies and phycoerythrin-labeled anti-human TLR2 antibodies.

### Activation of cells and western blotting:

Cells (10⁶/ml) were pretreated for 20 minutes with VB-201 or VB-207 followed by 15 minutes activation with 100 ng/ml lipopolysaccharide (LPS), 200 ng/ml recombinant human IL-1β, 10 µg/ml peptidoglycan (PGN-SA), 300 ng/ml Pam3CSK4, 0.5 µg/ml R848, 10 µg/ml CpG ODN 1826, or 1 µg/ml flagellin. Cells were washed and re-suspended in lysis buffer containing 1:100 dithiothreitol (DTT) (Bio-Lab), and phosphatase and protease inhibitors (Thermo Scientific). Samples were loaded on a precasted Criterion TGX gel (BIO-RAD, UK) and transferred onto nitrocellulose membrane. Blots were blocked with 5 % milk or bovine serum albumin (BSA) in TBST (Tris buffer saline with TWEEN 20) for 1 hour, followed by incubation with primary and secondary antibodies. Membranes were developed using the ECL kit (Thermo Scientific).

### Precipitation of OB-VB201 and OB-VB207:

Cells were lysed with 1 % NP-40 lysis buffer containing 1:100 protease and phosphatase inhibitors, followed by 20 minutes incubation on ice and 15 minutes centrifugation at maximum spin. Samples were incubated overnight at 4 °C with solvent, OB-VB201 or OB-VB207 in a rotator. Streptavidin agarose beads (Sigma) were added for 2 hours. Protein elution was performed with lysis buffer without DTT for 10 minutes at room temperature. Sample loading, transfer and immuno-blotting was performed as described above.

### VB-201 cell surface binding specificity by flow cytometry:

To assess VB-201 interference with LPS binding, VB-201 was incubated for 20 minutes with cells (10⁶/ml) and then 100 ng/ml of biotin-lipopolysaccharide (LPS) was added for additional 15 minutes, all at 4 °C. Cells were washed and LPS binding was detected using streptavidin-APC. Evaluation of OB-VB201 and OB-VB207 binding to cells was also made with streptavidin-APC. To prevent binding of OB-VB201 to CD14, anti human CD14 blocking antibody clone 18D11 (HyCult biotech, Netherlands) or a control anti-6x Histidine antibody (R&D Systems) were employed for 15 minutes before adding OB-VB201 for additional 15 minutes.

### Atherosclerosis study:

VB-201 (0, 0.15 or 1.5 mg/kg) was administered to 9-11-week old male ApoE KO mice (bred in-house at the Bert W. Strassburger Lipid Center, Sheba Medical Center, Israel) by oral gavage. Dosing was performed once a day, for 8 weeks (n=11-12/group). Atherosclerotic lesions were quantified by calculating the lesion size in the aortic sinus as previously described by Davenport & Tipping [The American journal of pathology. 2003, 163:1117-1125]. Lesion area was calculated using a computerized analysis method (Image Pro Plus software, V. 4.5.1.29, Medical Cybernetics Corporation). Aorta lesion area was determined using the Image-Pro Plus software, which measured the staining area covered by Sudan IV stain adhering to the lesioned tissue. Lesion size in the aorta was measured and the percentage of lesioned area was calculated relative to the size of the entire aorta. All animal experiments were approved by the Institutional Animal Care and Use Committee of the Sheba Medical Center, Ramat Gan, Israel.

### In vitro cell migration assay:

Human monocytes (CD14+ cells) and CD4+T-cells were isolated from whole blood of healthy donors. Following PBMCs separation from whole blood using Ficoll-Paque and leucosep tubes (Greiner Bio-One), CD14+/CD4+ cells were separated using specific anti-CD14/anti-CD4 micro beads, respectively (Miltenyi Biotech). Purity of the isolated populations was validated using specific antibodies by FACS analysis. Isolated cells were pre-incubated for 30 minutes with solvent (1 % ethanol/PBS) or VB-201 as indicated. For chemo-attraction of monocytes, RANTES (100 ng/ml), MIP-1α (50 ng/ml) or MCP-1 (50 ng/ml) were dissolved in 0.5 % fetal bovine serum(FBS)/RPMI-1640 medium and placed at the lower chamber of QCM™ 24-well migration assay plate (Corning-Costar; 5µm pores). For chemo-attraction of CD4+ cells, 0.5 % FBS/RPMI-1640 solution containing RANTES (100 ng/ml) and SDF-1α (50 ng/ml) was used, using the same plate setting. Migration assay was conducted by seeding 300,000 treated cells in the upper chamber, followed by incubation of 3-4 hours. Subsequently, the number of cells which migrated to the medium in the lower compartment was determined by FACS.

### Phagocytosis assay:

Phagocytosis assays were conducted using pHrodo™ Phagocytosis Particle Labeling Kit (Invitrogen) according to the manufacturer protocol. Briefly, whole blood samples from healthy donors were incubated for 15 minutes with solvent or VB-201, as indicated. Then, pHrodo dye-labeled *E. coli* bacteria were mixed with the treated blood samples, in 20:1 ratio of pHrodo™ particles to phagocytosing cells and incubated for additional 15 minutes. Each sample was analyzed both at 4 °C (no phagocytosis, negative control) and 37 °C. Following incubation, red blood cells were removed by lysis and the samples were washed 3 times with wash buffer. For gating on specific subpopulations, cells were labeled with anti-CD14-FITC (monocytes and granulocytes marker; BD Biosciences) and anti-CD66b-PerCP/Cy5.5 (granulocytes marker; BioLegend) antibodies. pHrodo fluorescence intensity was analyzed by FACS.

### Cell viability assay:

Following incubation with the indicated doses of VB-201 for 3.5-4 hours, cell viability was analyzed using MTS Cell Proliferation Assay (Promega) or Annexin-V/7-AAD staining kit (BD Pharmingen) according to the manufacturer protocol.

### Flow Cytometry:

Human CD14+ monocytes were incubated for 3.5 hours in 2 % FCS/RPMI medium with solvent or VB-201. Cells were then washed, resuspended in PBS containing 2 % FCS and 0.02 % sodium azide (FACS buffer) and stained for 30 minutes at 4 °C with anti CCR1-Alexa Fluor 647 (BD Biosciences), anti CCR5-PE, anti CXCR1-PE and anti CXCR2-Alexa Fluor 647 (all from BioLegend). Cells were washed, resuspended in FACS buffer and analyzed on a FACS-Calibur.

### Monocyte adhesion assay:

Matrix plates were pre-seeded with HUVEC and subjected to 4 hour stimulation with 10 ng/ml TNFα to induce expression of adhesion molecules. Freshly isolated monocytes were stained for 30 minutes with 5 µM calcein AM (Invitrogen) in serum free RPMI and then washed twice with PBS to remove excess dye. Monocytes were treated with solvent control or increasing concentrations of VB-201 or OxPAPC for 30 minutes. 10⁵ stained monocytes were loaded per well into 96-well dishes and allowed to adhere for 15 minutes. Following 4 washes with PBS, adherent monocyte intensity was read using FLUOROSKAN Ascent FL device using excitation at 485 nm and emission at 538 nm.

### GPCR activation assay:

Analyses were performed by Millipore GPCR Profiler Service, by measuring intracellular calcium signal in real time using FLIPR^{TETRA} device. VB-201 agonist percentage activation was calculated relative to the Eₘₐₓ control for each GPCR tested. Antagonist percentage inhibition was referenced to EC₈₀ control wells for each tested GPCR.

### cAMP accumulation assay:

Human monocyte-derived dendritic cells (DCs) grown in 24-well plates were incubated for 2 hrs with RPMI-1640 medium containing 5 µCi/ml [³H] adenine (American Radiolabeled Chemicals, Inc.). Following RPMI wash, the medium was replaced with 10 % FBS/RPMI medium supplemented with either solvent (1 % ethanol/PBS), 10 µM forskolin (Sigma), VB-201 or oxidized PAPC as indicated. Cells were incubated for 4 hours at 37 °C in the presence of the phosphodiesterase inhibitors IBMX (Calbiochem; 0.5 mM) and RO-20-1724 (Biomol; 0.5 mM) to prevent cAMP degredation. For the quantification of [³H]cAMP formation, the incubation was terminated by removal of the medium and addition of 2.5 % perchloric acid containing 0.1 mM unlabeled cAMP (Sigma), followed by neutralization with 4.2 M KOH and 1.85 M K₂CO₃. Intracellular [³H]cAMP was separated from other labeled nucleotides by two-step column chromatography, using ion exchange Dowex 50WX4 resin (Bio-Rad) and alumina column (Sigma) and quantified by measuring the radioactivity in the eluates.

### Determination of cholesterol and triglyceride levels:

Total plasma cholesterol and triglyceride levels were determined using an automated enzymatic technique (Roche Cobas Mira Analyzer). The triglyceride and cholesterol content were measured using a micro-plate assay (Roche/Hitachi enzymatic assay reagent kits: thermo - Triglycerides Reagent TR22421 and Cholesterol Reagent 12016630 122).

### Immunohistochemistry:

Immunostaining was performed on frozen sections which were fixed with cold acetone and blocked with 4 % normal rabbit serum/PBS (Vector Lab). Following incubation with Rat anti-mouse CD68 (Serotec NCA1957; 1:250) and anti-Rat biotinylated antibody (Vector Lab AB-4001; 1:200), signal was developed using Vectastain ABC-Alkaline phosphatase kit and Vector Red Substrate (Vector Lab). Mayer's Hematoxylin was used for counterstaining.

### Statistical analysis:

Mean ± standard deviation/standard error was calculated using Excel or Sigma-Stat software, and the statistical significance was calculated by two-tailed Student t-test. To compare between experimental groups one way ANOVA or t-test was used. A value of P<0.05 was considered statistically significant.

### EXAMPLE 1

### Uptake of VB-201 by immune cells

In order to characterize the effects of VB-201 on the immune system, isolated subsets mononuclear cells from mouse and human were exposed to [³H]₂-radio-labeled VB-201 and subsequently analyzed for incorporation of radio-labeled VB-201, as described in the Materials and Methods section.

Cells from the following sources were treated with ³H₂-VB-201:
Human CD14+ monocytes;
Human dendritic cells (DCs) derived from CD14+ monocytes;
Human CD4+ T cells;
Human CD19+ B cells;
Mouse CD11c+ dendritic cells derived from bone marrow (BMDCs);
Mouse CD90+ T cells;
Mouse B220+ B cells.

The cells were obtained as described in the Materials and Methods section.

As shown in Figures 1A and 1B, uptake of VB-201 is considerably greater in human (Figure 1A) and mouse (Figure 1B) dendritic cells and in human monocytes (Figure 1A) than in human and mouse T cells and B cells.

These results indicate that there is a significant difference in VB-201 association between myeloid and lymphoid cells. VB-201 associates primarily with myeloid cells such as dendritic cells and monocytes, the professional antigen presenting cells, and considerably less with lymphocytes. Thus, these results indicate specificity to VB-201 target cell population.

### EXAMPLE 2

### Effect of VB-201 on Toll-like receptor (TLR) signaling

In order to gain a better understanding of the anti-inflammatory mechanism of VB-201, the effect of VB-201 on the signaling pathways of different toll-like receptors (TLRs) was determined.

Toll-like receptors were activated in mouse BMDCs (bone marrow dendritic cells) by specific agonists, and the effect of receptor activation was determined via Western blot for phosphorylation of p38 MAP kinase, which is downstream of TLRs. Similarly, the effect of VB-201 on activation of IL-1 receptor (IL-1R), which shares the adaptor molecule MyD88 with TLRs, was determined via Western blot for phosphorylation of p38 MAP kinase.

The BMDCs were pre-treated with VB-201 or solvent and then stimulated as described in the Materials and Methods section, using the following agonists: Pam3CSK4 was used as an agonist of TLR2:TLR1 dimer; peptidoglycan (PGN) was used as an agonist of TLR2:TLR6 dimer; lipopolysaccharide (LPS) was used as an agonist of TLR4; flagellin was used as an agonist of TLR5; R848 was used as an agonist of TLR7; CpG was used as an agonist of TLR9; and IL-1β was used as an agonist of IL-1R.

As shown in Figures 2A-2G, VB-201 inhibited p38 phosphorylation in a dose-dependent manner in mouse BMDCs following activation of TLR4 (Figure 2C) or dimers of TLR2 (Figures 2A and 2B), but had no apparent effect on p38 phosphorylation following activation of TLR5 (Figure 2D), TLR7 (Figure 2E), TLR9 (Figure 2F) or IL-1 receptor (Figure 2G).

These results suggest that VB-201 specifically inhibits signaling of TLR2 and TLR4 pathways.

The above results were confirmed by results obtained upon receptor activation in human CD14+ monocytes and in mouse peritoneal macrophages. TLRs were activated by specific agonists, as described hereinabove, resulting in downstream phosphorylations of p38, Erkl/2 and IKKα/β, along with degradation of IκBα. The effect of VB-201 on these signaling mechanisms was observed via Western blot.

As shown in Figure 3, VB-201 inhibited activation of p38, ERK1/2 and the NFκB pathway in a dose-dependent manner, following activation of TLR4, but had no apparent effect following TLR5 activation.

As shown in Figure 4, VB-201 inhibited activation of p38, ERK1/2 and the NFκB pathway in a dose-dependent manner, following activation of TLR4 or TLR2:TLR1 dimer.

The inhibition of TLR2 signaling was further confirmed by examining the effect of VB-201 on serum amyloid A-induced TLR2 signaling in human CD14+ monocytes and in THP-1 cells. The effect of VB-201 on TLR2 signaling was observed via Western blot, as described hereinabove.

As shown in Figure 5, VB-201 inhibited activation of p38, ERK1/2 and the NFκB pathway, following activation TLR2 by serum amyloid A, in both human CD14+ cells and in THP-1 cells.

These results indicate that VB-201 inhibits TLR2-mediated signaling events induced by non-microbial motifs (e.g., as with SAA).

Taken together, the above results indicate that inhibition of TLR signaling by VB-201 is restricted to TLR2 and TLR4, which are present at the plasma membrane surface.

### EXAMPLE 3

### Binding of VB-201 to molecular targets

In view of the results presented in Example 2, showing that VB-201 inhibits TLR2 and TLR4 pathways, experiments were performed in order to determine which molecules are bound by VB-201. For this purpose, VB-201 and its R1 truncated inactive form VB-207 were labeled with biotinylated ovalbumin, as described in the Materials and Methods section.

The biotinylated ovalbumin-labeled VB-201 and VB-207 (OB-VB201 and OB-VB207, respectively) were initially tested for activity against TLR2 and TLR4 signaling in RAW 264.7 murine macrophages, using methods similar to those described in Example 2.

As shown in Figures 6A and 6B, both VB-201 and OB-VB201 inhibited LPS-induced signaling in a dose-dependent manner, whereas neither VB-207 nor OB-VB207 exhibited such an inhibitory effect.

Similar results were obtained following PGN-induced signaling (data not shown).

These results confirm that the labeled VB-201 and VB-207 exhibit similar biological effects as non-labeled VB-201 and VB-207, respectively.

OB-VB201 and OB-VB207 were then applied to lysates from primary human monocytes and a human monocyte line (THP-1 cells) and precipitated as described in the Materials and Methods section, in order to test for their binding potential to TLR4 and TLR2.

As shown in Figures 7A and 7B, OB-VB201 bound TLR2 in both human primary monocytes (Figure 7A) and in THP-1 cells (Figure 7B), whereas OB-VB207, in line with its lack of function, did not bind to TLR2. As further shown therein, no binding of either OB-VB201 or OB-VB207 to TLR4 was detected.

In order to determine the mechanism by which VB-201 affects TLR4 signaling without binding to TLR4, precipitated lysates were analyzed for CD14, an essential co-receptor for TLR4 activity, for MyD88, a promiscuous TLR downstream signaling adaptor molecule, and for CD36, which is widely described to interact with oxidized phospholipids, and which interacts with TLR4.

As further shown in Figure 7A, OB-VB201 bound CD14 in human primary monocytes, whereas OB-VB207 did not bind to CD14.

As further shown in Figures 7A and 7B, neither MyD88 (Figures 7A and 6B) nor CD36 (Figure 7B) were bound by either OB-VB201 or OB-VB207.

As CD14 has been described as a co-receptor for TLR2 as well as for TLR4, it was ascertained that detection of both TLR2 and CD14 following incubation with OB-VB201 was not due to co-precipitation. Accordingly, CD14 and TLR2 were transfected separately into HEK293 cells (as described in the Materials and Methods section) and the cell lysates were then precipitated with OB-VB201.

As shown in Figure 8, OB-VB201 bound to both CD14 and to TLR2.

The above results indicate that VB-201 binds separately to both CD14 and TLR2.

The results further indicate that the effect of VB-201 on TLR4 signaling is via binding to CD14, and that the effect of VB-201 on TLR2 signaling is via direct binding to TLR2.

CD14 is known to bind to lipopolysaccharide (LPS). In order to determine whether VB-201 impairs the ability of cells to bind LPS, RAW 264.7 macrophages were contacted with biotinylated LPS, and then stained using streptavidin-APC (allophycocyanin), and the cells were then analyzed by flow cytometry.

As shown in Figure 9, VB-201 inhibited binding of biotinylated LPS to macrophages in a dose dependent manner.

These results indicate that binding of VB-201 to CD14 inhibits CD14 activity by impairing the ability of CD14 to bind LPS.

In order to confirm that VB-201 binds to CD14, transfected HEK293 cells expressing high levels of CD14 were contacted with biotinylated VB-201 or VB-207 (OB-VB201 and OB-VB207, respectively), and then stained using streptavidin-APC, and the cells were then analyzed by flow cytometry. As shown in Figures 10A-10D, CD14 transfected cells were positively stained in the presence of 10 µg/ml (Figure 10A) or 5 µg/ml (Figure 10B) OB-VB-201, whereas no staining could be observed in cells incubated with OB-VB207 (Figures 10C and 10D).

In addition, the correlation between the degree of CD14 expression and the degree of VB-201 binding was examined by distinguishing between high and low expression of CD14 via flow cytometry, as shown in Figure 11A.

As shown in Figures 11B and 11C, cells exhibiting high expression of CD14 bound more OB-VB201 than did cells exhibiting low expression of CD14.

In order to confirm that VB-201 binds to CD14, the effect of anti-CD 14 antibodies on VB-201 binding was examined in HEK-Blue-4 cells and in human primary monocytes. Antibodies which are not specific for CD14 were used as a control.

As shown in Figures 12A-12D, anti-CD14 antibodies reduced binding of OB-VB201 to both HEK-Blue-4 cells overexpressing CD14 (Figure 12A) and human primary monocytes (Figure 12C) in a dose dependent manner, whereas control antibodies had no effect on binding of OB-VB201 to cells (Figures 12B and 12D).

The above results provide further confirmation that VB-201 binds to CD14.

### EXAMPLE 4

### Effect of oxidized phospholipids on chemotaxis

In order to investigate the effects of oxidized phospholipids such as VB-201 on chemotaxis, monocytes were tested in the absence of other cell types (which could complicate interpretation of the results), in *in vitro* trans-well assays using isolated monocytes, defined phospholipids and recombinant chemoattractants.

To this end, monocytes from healthy donors were pre-incubated for 30 minutes with solvent (1 % ethanol/PBS) or 5 µg/ml of either VB-201 (an oxidized phospholipid), PGPC (1-palmitoyl-2-glutaryl-phosphatidylcholine, an oxidized phospholipid), phosphatidylcholine (a non-oxidized phospholipid) or with 40 µg/ml of PAPC (1-palmitoyl-2-arachidonyl-phosphatidylcholine) following 24 hours oxidation (a partially oxidized phospholipid). Following incubation, cells were loaded to the upper chamber and allowed to migrate for 3.5 hours towards 0.5 % fetal bovine serum/RPMI-1640 medium supplemented with recombinant human MCP-1 and RANTES (50 ng/ml each).

In addition to testing primary human CD14⁺ cells, the effect of VB-201 on the human monocyte cell line THP-1 was also examined in a trans-well assay.

As shown in Figure 13, VB-201 significantly inhibited *in vitro* migration of THP-1 cells in a dose-dependent manner.

Viability of the THP-1 cells was determined by an MTS assay, which showed that VB-201 did not affect viability of the cells (data not shown), thereby confirming that observed reduction in migrating cells reflected reduction of cell migration and not reduction of cell viability.

Inhibition of monocyte chemotaxis by VB-201 has been further described in International Patent Application PCT/IL2011/000012 (published as WO 2011/083469).

However, the above results indicate that a variety of oxidized phospholipids, including VB-201 and PGPC, inhibit monocyte chemotaxis.

### EXAMPLE 5

### Effect of VB-201 on monocyte activity

In order to determine whether VB-201 has any antagonist (or agonist) activity towards chemokine receptors, VB-201 underwent G-protein coupled profiling using ChemiScreen stable cell lines expressing different chemokine receptors, and measurement of Ca⁺⁺ flux.

As shown in Figure 14A, treatment with VB-201 yielded less than 5 % activation relative to positive controls, suggesting that it is not an agonist of any of the tested chemokine receptors.

A CCR2B antagonist assay was performed in presence of escalating doses of VB-201, VB-207 (inactive molecule, negative control) or RS102895 (CCR2B antagonist, positive control). C. CCR5 antagonist assay performed in presence of escalating doses of VB-201 or VB-207. As shown in Figures 14B and 14C, VB-201 did not induce an antagonistic effect against CCR2B or CCR5, in ChemiScreen cell lines.

These results indicate that VB-201 does not affect chemotaxis by acting directly on chemokine receptors.

In addition, the effect of VB-201 on cAMP levels was tested in monocyte-derived dendritic cells.

As shown in Figure 15, intracellular cAMP levels were increased by oxidized PAPC (1-palmitoyl-2-arachidonyl-phosphatidylcholine) in a dose dependent manner and by forskolin (an activator of adenylate cyclase, used as a positive control), whereas cAMP levels were not affected by treatment with 1-5 µg/ml VB-201.

In addition, the ability of VB-201 to act as a cell attractant was tested by placing it in the lower chamber of a trans-well assay. However, no monocyte chemotaxis towards VB-201 occurred (data not shown).

The above results indicate that VB-201 does not affect calcium-based or cAMP-based cell signaling in monocytes, is not a chemoattractant or chemokine receptor antagonist for monocytes, and does not affect monocyte functions other than chemotaxis. Taken together, the results indicate that VB-201 specifically inhibits chemotaxis in monocytes, by inhibiting an intracellular process downstream of chemokine receptors, and not by inhibiting monocyte activity in general.

### EXAMPLE 6

### Effect of MEK-ERK pathway inhibition by VB-201 on monocyte chemotaxis

In view of the specific inhibition of chemotaxis-associated signaling by VB-201, as indicated by the results presented in Example 4, the effect of VB-201 on chemokine-induced signaling pathways was investigated.

Human monocytes were pre-treated with 5 µg/ml VB-201 (or solvent as a control) and then stimulated with the chemokines MCP-1, MCP-3 or Fractalkine (CX3CL1). Lysates were at different time points following chemokine stimulation and the phosphorylation levels of Akt, MEK1/2, ERK1/2 and p38 were determined. Total levels of Akt, MEK1/2, ERK1/2 and p38 were determined as a control.

As shown in Figure 16A, VB-201 pretreatment caused a considerable reduction in the phosphorylation levels of Akt, MEK1/2, and ERK1/2, but not in phosphorylation levels of p38, in both MCP-1 and MCP-3-stimulated cells.

Similarly, as shown in Figure 16B, VB-201 pretreatment caused a considerable reduction in the phosphorylation levels of ERK1/2 in Fractalkine-stimulated cells.

Similar results, showing inhibition by VB-201 of ERK1/2 phosphorylation and MEK1/2 phosphorylation, were obtained following stimulation with the chemokines MCP-1, MIP-1α or RANTES, as shown in International Patent Application PCT/IL2011/000012 (published as WO 2011/083469).

The above results suggest that the MEK-ERK pathway and the Akt pathway, but not the p38 pathway, are important in chemokine-induced signaling in monocytes.

In order to further ascertain the significance of ERK1/2 phosphorylation and MEK1/2 phosphorylation to monocyte chemotaxis, the effect of the RAF inhibitor GW5074, which reduces MEK and ERK phosphorylation, on monocyte chemotaxis was determined in a trans-well migration assay using a combination of MCP-1 (50 ng/nl) and RANTES (70 ng/ml) as attractant. Monocytes were pre-treated with 5 µM GW5074 for 30 minutes prior to seeding in the migration chamber.

As shown in Figure 17, GW5074 reduced migration by about 80 %. This result indicates that monocyte chemotaxis is dependent on activation of the RAF-MEK-ERK pathway.

The effects of VB-201, GW5074, and combinations thereof on ERK1/2 phosphorylation were then compared. Monocytes were pre-treated for 30 minutes with 5 µg/ml VB-201 and/or 2.5 µM GW5074, prior to following stimulation for 2 minutes with 20 ng/ml MCP-1.

As shown in Figure 18, the combination of VB-201 and GW5074 inhibited ERK1/2 phosphorylation to a considerably greater extent than either VB-201 alone or GW5074 alone.

The above results indicate that inhibition by VB-201 of the MEK-ERK pathway in chemokine-stimulated monocytes results in reduced chemotaxis in monocytes.

### EXAMPLE 7

### Effect of VB-201 on monocyte chemotaxis in vivo

To test our hypothesis that VB-201 may act to modulate inflammation via inhibition of monocyte trafficking, VB-201 was employed in the mouse ApoE knockout model of atherosclerosis. ApoE knockout mice exhibit severe hypercholesterolaemia and develop spontaneous atherosclerotic lesions. To evaluate the possible effect of VB-201 on atherosclerosis development, VB-201 or PBS were administered by daily oral gavage to young ApoE mice (9-11 weeks old) for 8 weeks (n=11-12 per group), after which animals were scarified and lesions were examined.

As shown in Figure 19A, fatty streaks were markedly reduced in VB-201 treated animals, and 1.5 mg/kg VB-201 significantly reduced plaque formation relative to the PBS control.

This effect was clear in spite of the fact that VB-201 administration did not modify metabolic parameters associated with elevated risk for the development of atherosclerosis such as total plasma cholesterol or triglycerides (data not shown).

To test whether the VB-201-mediated inhibition of atherosclerosis development correlated with reduced monocyte/macrophage accumulation, aortic sinus lesion specimens were sectioned and were stained for plaque content as well as for macrophage abundance using anti-CD68 antibody.

As shown in Figure 19B, macrophage accumulation in the aortic sinus lesion was reduced in VB-201-treated animals relative to controls.

These results indicate that VB-201 inhibits macrophage accumulation in atherosclerosis plaques *in vivo*, and that this inhibition of macrophage accumulation is associated with smaller and more stable lesions.

### EXAMPLE 8

### PGPC and VB-201 inhibit human monocyte chemotaxis in vitro

Investigating potential anti-inflammatory roles of oxidative phospholipids, we were interested in testing possible effects of native phospholipids on monocyte chemotaxis directly. Oxidative phospholipids were previously reported to increase monocyte adhesion to endothelial cells (Berliner, J.A., et al., Free Radic Biol Med. 2008;45:119-123; Cole, A.L. et al., Arterioscler Thromb Vasc Biol. 2003;23:1384-1390). However, these effects resulted from activation of the endothelial cells which was followed by cytokine secretion and enhanced expression of adhesion molecules, while the monocytes themselves were not treated or studied. We therefore chose to avoid the presence of other cell types and performed *in vitro* monocyte chemotaxis assays using isolated human monocytes and recombinant chemoattractants.

For this purpose, monocytes from healthy donors were pre-incubated for 30 min with either solvent (0.005% ethanol/PBS), phosphatidylcholine (PC; 5 µg/ml), partially oxidized PAPC (24h oxidation; 40 µg/ml) or PGPC (5 µg/ml), and allowed to migrate towards recombinant human MCP-1 (CCL2; ligand for CCR2) and RANTES (CCL5; ligand for CCR1,CCR3,CCR5, US28) as attractants (50 ng/ml each). Interestingly, whereas PC and partially oxidized PAPC did not reduce the number of migrating cells, treatment with pure PGPC, an oxidized product of PAPC, significantly attenuated monocyte chemotaxis by ∼50% (Figure 22A).

This unexpected result led us to screen through a library of lecinoxoids for additional compounds that may inhibit monocyte chemotaxis. Interestingly, the screen yielded VB-201, a synthetic saturated phospholipid which is structurally related to PGPC that demonstrated profound inhibition of monocyte chemotaxis (Figure 22A).

We have repeated assays, employing MCP-1, RANTES or MIP-1α (CCL3; ligand for CCR1,CCR5) as chemoattractants and found that compared to the solvent control, VB-201 significantly and profoundly reduced monocyte migration by approximately 80% for MCP-1, 72% for RANTES and 68% for MIP-1α (Figure 22B). In addition, we tested the effect of VB-201 on monocyte migration when growth medium taken from a culture of human umbilical cord endothelial cells (HUVEC) was used as attractant. This culture medium contains 2% serum, supplementary growth factors and HUVEC-secreted factors (such as IL-8, data not shown). In our hands, it induced a more robust monocyte attraction than any of the single chemoattractants described above. In this setting, VB-201 reduced the number of migrating cells in a dose-dependent fashion, yielding about 60% inhibition at dose of 5 µg/ml. Inhibition of migration was not seen with fully oxidized PAPC (oxidized for 72h), for any of the doses tested (Figure 22C).

To exclude the possibility that inhibition of monocyte migration by VB-201 results from cell death, monocytes were treated with escalating doses of VB-201 for 16 hours, after which cell viability was determined by MTS assay. As shown in Figure 22D, VB-201 had no effect on monocyte viability at doses of 1-10 µg/ml. In addition to testing primary human CD14⁺ cells, we also examined the effect of VB-201 on the human monocyte cell line THP-1. As seen with primary monocytes, VB-201 significantly inhibited *in vitro* migration of THP-1 cells without affecting cell viability, again ruling out any artificial effect of VB-201 on chemotaxis due to toxicity.

To ensure that VB-201 is not an oxidative molecule which may induce ROS and damage cells, human monocytes were incubated overnight with PBS, VB-201 (10 µg/ml), PGPC (10 µg/ml) or OxPAPC (100 µg/ml; PAPC oxidized for 24h) and ROS formation was tested as described in the methods section. As expected, the positive control mixture of OxPAPC significantly elevated ROS in human monocytes relative to PBS-treated cells. However, VB-201 and PGPC had no effect on ROS formation in primary monocytes (Figures 23A and 23B).

Collectively, these findings indicate that some phospholipid molecules, such as PGPC and VB-201, can inhibit monocyte chemotaxis *in vitro.* Notably, this effect is compound-specific and does not result from a general phospholipid-related phenomenon (a class effect).

### EXAMPLE 9

### Monocyte Phagocytosis and Adhesive Properties Are Not Affected by VB-201

Given the inhibition of monocyte chemotaxis by VB-201, we were interested to test whether VB-201 affects additional monocytic functions. To this end, a phagocytosis assay was performed on whole blood from healthy donors, and the capacity of leukocyte subsets to phagocytose PE-labeled E.Coli particles was tested in presence or absence of VB-201. As a negative control, we used blood without bacterial particles, while a positive control included blood and labeled-bacterial particles incubated at 37°C. Cells were then stained with CD14 and CD66b antibodies to differentiate between monocytes and granulocytes and were analyzed by FACS. Interestingly, VB-201 doses of 5 or 10 µg/ml that were effective in inhibition of monocyte migration did not have any significant effect on monocyte phagocytosis capacity (Figure 24A). In addition, no VB-201 effect was seen on granulocyte phagocytosis (Figure 24B).

Next, we examined whether VB-201 or oxidized PAPC treatment of monocytes affects their interaction with endothelial cells. HUVEC were plated in 96-wells and treated with TNFα to induce expression of adhesion molecules. Monocytes were fluorescently pre-labeled with calcein AM and treated with either solvent, increasing doses of VB-201 or oxidized PAPC, and subsequently allowed to adhere to the endothelial cells (which were not treated with VB-201 or oxidized PAPC). Following extensive washes, adherent monocytes were quantified by fluorescence. As expected, TNFα treatment increased solvent-treated monocyte adhesion by ∼5-fold relative to the no-TNFα control. However, treatment of monocytes with 1-10 µg/ml VB-201 had no effect on their adhesion to HUVEC compared to the solvent control (Figure 24C). These experiments suggest that the effect of VB-201 is process-specific, regulating monocyte chemotaxis but not their phagocytosis or adhesive properties.

### EXAMPLE 10

### Cell Specificity of VB-201

Since chemokine receptors are expressed on different immune cells, we asked whether the effect of VB-201 is of broad "phospholipid" nature, or perhaps is cell-specific. To this end, CD4⁺ T-cells were isolated from healthy volunteers and following 30 min pre-incubation with VB-201, were employed in chemotaxis assays. At a dose of 5 µg/ml of VB-201, which dramatically inhibits monocytes chemotaxis, we were unable to find any inhibition of CD4⁺ cell chemotaxis in multiple tested donors (FIG. 25A). Similarly, VB-201 had no effect on chemotaxis of human Neutrophils towards LPS or RPMI-1640 medium supplemented with 10% FBS/ MCP-3 (Online Figure FIG. 25B). The effect of VB-201 on cell motility seems to be cell-specific.

The effect of VB-201 on cell signaling in monocytes and T-cells was determined by a Western Blot for phosphorylated ERK1/2, using monocytes and T-cells isolated from the same donor.

As shown in Figure 25C, VB-201 inhibited ERK1/2 phosphorylation in stimulated monocytes but not in stimulated T-cells.

In addition, VB-201 had no effect on cell migration in a wound-healing 'scratch' assay performed using NIH-3T3 mouse fibroblasts or MCF-7 human breast cancer cells (data not shown).

These results indicate that inhibition of chemotaxis by oxidized phospholipids, and the associated effect on cell signaling, is cell-specific, being limited to monocyte chemotaxis.

### EXAMPLE 11 (for reference only)

### VB-201 Inhibits In Vivo Monocyte Migration in the Mouse Peritonitis Model

To test whether VB-201 can inhibit monocyte migration *in vivo*, we took advantage of the well-characterized mouse model of thioglycollate-induced peritonitis. In this model, injection of thioglycollate to the peritoneal cavity induces a "sterile" inflammatory response recruiting peripheral monocytes to the peritoneum, where they differentiate into macrophages (Li, Y.M., et al., J. Immunol. Methods. 1997;201:183-188). Mice were fed with different doses of VB-201 for 5 days prior to thioglycollate injection. Upon sacrifice, the number and identity of the cells accumulating in the peritoneum was determined by FACS, and plasma samples were taken for quantification by Liquid Chromatography-Tandem Mass Spectrometry. Three days after thioglycollate injection 95% of the cells in the peritoneum were macrophages (F4/80 positive, GR-1 negative cells) (Figure 26B).

Comparing the number of accumulating macrophages in the control animals versus VB-201-treated groups, we noticed that the lowest dose of 0.04 mg/kg/day of VB-201 had no inhibitory effect on macrophage accumulation, as was expected with such a low dose. However, a dose of 0.4 mg/kg, which yielded 0.8±0.3 µmol/L VB-201 in mice plasma, resulted in ∼25% inhibition. Notably, when mice were fed with 4 mg/kg VB-201, dosing which yielded plasma level of 8.5±1.5 µmol/L VB-201 that was active in human monocytes, we observed a profound (>60%) significant reduction in the number of peritoneal macrophages relative to the PBS-treated animals (Figure 26A).

These data demonstrate a dose-dependent *in vivo* effect of VB-201 on monocyte migration in a mouse peritonitis model. Furthermore, the effective dose of VB-201 in mice plasma correlated with the dose of VB-201 that inhibited chemotaxis of human monocytes *in vitro.*

### EXAMPLE 12

### VB-201 Inhibits Phosphorylation of AKT and ERK1/2

VB-201 inhibits GTP-γ-S-induced intracellular activation of the AKT and ERK pathways in the monocytic THP-1 Cell line. THP-1 cells were incubated for 16 hrs in RPMI-1640 medium supplemented with 0.5% FBS and 1% Pen/Strep/Glutamine to reduce basal signaling readouts. Subsequently, cells were divided into 1.5ml tubes (1.5*10⁵ cells/tube) and were pre-treated with a solvent control or 8.5 µM VB-201 for 20 min. Stimulation with 100µM GTP-γ-s, an activator of G-proteins, was performed for 2, 5 or 15 minutes. Cells were harvested and analyzed by SDS-PAGE. As shown in FIG. 27, Western blot analysis shows reduction in phospho-ERK1/2 and phospho-AKT (Ser473) levels in VB-201 treated cells relative to the solvent control. Total ERK1/2 levels serve as loading control.

This example demonstrates the ability of VB-201 to inhibit phosphorylation of AKT and ERK1/2, even if the activation is induced using an agonist that works intracellularly (not via ligand of a surface receptor).

### EXAMPLE 13

### VB-201 Inhibits EGF-Induced Activation of ERK

VB-201 inhibits epidermal growth factor (EGF)-induced activation of ERK in THP-1, but not HEK293 Cells. The THP1 or HEK293 cell lines were grown in medium supplemented with 0.5% FBS and 1% Pen/Strep/Glutamine. Cells were treated with solvent or with VB-201 (8.5 µM) for 20 min, followed by stimulation of EGF (200ng/ml) for different time points. Cells were washed twice and harvested with lysis buffer and analyzed by SDS-PAGE. As shown in FIG. 28, Western blot analysis shows inhibition of ERK1/2 phosphorylation in the THP-1 monocytic cell line but not in HEK293 cells. Alpha Tubulin levels serve as loading control.

This example demonstrates the specificity of VB-201 - the same ligand used for stimulation of different cell types. VB-201 inhibits the downstream signaling in monocytes but not embryonic kidney cells. This shows that the identity of the ligand/means of activation is not affecting the ability of VB-201 to inhibit ERK activation/phosphorylation.

### EXAMPLE 14

### VB-201 Inhibits ERK1/2 Phosphorylation

VB-201 decreases ERK1/2 phosphorylation in human monocytes which were pre-stimulated with MCP-1 overnight. CD14 cells were isolated from a healthy donor and were incubated for 16 hrs in RPMI-1640 medium supplemented with 10% FBS, 1% Pen/Strep/Glutamine and 10ng/ml MCP-1. Subsequently, cells were treated with solvent, or with VB-201 (8.5µM) for different time points (2, 5 or 15 min). Untreated cells were used as reference for MCP-1 activation. Following treatment, cells were washed twice, harvested with lysis buffer and analyzed by SDS-PAGE. As shown in FIG. 29, Western blot analysis shows reduction of ERK1/2 phosphorylation in starting at 5 min following VB-201 addition. Alpha Tubulin levels serve as loading control.

This example demonstrates the capacity of VB-201 to reduce ERK1/2 phosphorylation in pre-activated monocytes. Mechanistically - proves that the VB-201 effect is not via interference of ligand-receptor interaction/activation.

### EXAMPLE 15

### VB-201 Inhibits CRP-Induced Activation of AKT and ERK And Inhibits C-Reactive Protein-Induced Chemotaxis

VB-201 inhibits C-reactive protein (CRP)-induced activation of the AKT and ERK pathways in the monocytic THP-1 Cell line. THP-1 cells were incubated for 16 hrs in RPMI-1640 medium supplemented with 0.5% FBS and 1% Pen/Strep/Glutamine to reduce basal signaling readouts. Subsequently, cells were divided into 1.5ml tubes (1.5*10⁵ cells/tube) and were pre-treated with a solvent control or 8.5 µM VB-201 for 20 min. Stimulation with 30µg/ml of CRP, activator the CD32 and CD64 receptors, was performed for 2, 5 or 15 min. Cells were harvested and analyzed by SDS-PAGE. As shown in FIG. 30A, Western blot analysis shows reduction in phospho-ERK1/2 and phospho-AKT(Ser473) levels in VB-201 treated cells relative to the solvent control. Total ERK1/2 levels serve as loading control.

This example demonstrates the ability of VB-201 to inhibit phosphorylation of AKT and ERK1/2, induced by CRP, a pro-inflammatory marker and a monocyte chemoattractant. May support VB-201 anti-inflammatory effect even without reducing CRP blood levels.

CD14+ monocytes isolated from blood of healthy donors were pre-treated with a solvent control or 8.5 µM VB-201 for 30 min. Chemotaxis assay was performed using 50ug/ml CRP (MP Biomedicals) for 3.5 hours. In FIG. 30B, the data represent 3 different donors. The effect of VB-201 is statistically significant (p< 0.01). This demonstrates the ability of VB-201 to inhibit chemotaxis induced by CRP, a pro-inflammatory marker and a monocyte chemoattractant. May support VB-201 anti-inflammatory effect even without reducing CRP blood levels.

### EXAMPLE 16

### VB-201 binds cell surface expressed TLR2

Human monocytes were incubated for 15min with anti-human TLR2 blocking antibody. For control, matched isotype antibody was used at the indicated concentrations before BO-VB201 was added for additional 15min. Streptavidin-APC (1:200) was used to detect binding. The results are shown in FIGS. 31A and 31B.

### EXAMPLE 17

### Combination of VB-201 and Other Inhibitors

As shown in FIG. 32, Wortmannin (1 µmol/L), Rapamycin (1.1 µmol/L) and the RAF inhibitor GW5074 (1 µmol/L) decrease chemotaxis of THP-1 cells *in vitro,* but their effect (black bars) is further enhanced by VB-201 (8.5 µM; grey bars). Data are mean ± SD from 4 experiments in triplicates, normalized to untreated cells. **, p<0.001.

These data demonstrate that the effect of VB-201 on inhibition of chemotaxis can be achieved via other inhibitors as well, but with VB-201 the effect is stronger (most probably since VB-201 inhibits both PI3K-AKT and MEK-ERK, not only one of the pathways). Unfortunately, we can't say that this is a synergistic effect.

### EXAMPLE 18

### Binding properties of VB-201 and VB-207 in an in silico model

The binding properties of VB-201 and VB-207 were determined using *in silico* modeling. Potential interactions between VB-201 and VB-207 and the proteins TLR1-TLR2 (human), TLR4 (human), CD14 (human), and PI3K-γ (human) were analyzed by *in silico* docking experiments.

Modeling was performed using CDOCKER docking method based on CHARMm (Chemistry at HARvard Macromolecular Mechanics) simulation minimization.

Docking interactions (not shown) were observed between VB-201 and VB-207 and the potential targets TLR1-TLR2, TLR4, CD14 and PI3K-γ. However, the docking interactions between VB-207 and the targets failed to show any consistency in docking orientation, indicating that VB-207 does not specifically bind to these targets. In contrast, all binding affinities were stronger for VB-201 than for VB-207, and docking interactions between VB-207 and targets showed some consistency in docking orientation, indicating that VB-201 is more likely to bind specifically to one or more of the targets.

The above results are consistent with the abovementioned findings that VB-201, but not VB-207, binds to CD14 and TLR2.

However, the assay results described hereinabove do not support binding of TLR4 by VB-201.

In addition, VB-201 was found to be unlikely to be capable of crossing a cell membrane, based on analysis of its molecular structure.

This is consistent with the abovementioned finding that VB-201 binds only toll-like receptors present at the plasma membrane surface.

### EXAMPLE 19

### Selectivity of VB-201 binding

In order to assess the selectivity and significance of VB-201 binding to TLR2 and CD14, VB-201 was assayed for effect on activity of a variety of additional proteins, particularly proteins having a pro-inflammatorry activity, using standard procedures.

No inhibition of COX-1, COX-2, HMG-CoA reductase or 12-LO (12-lipoxygenase) was observed for VB-201 at concentrations of up to 10 µM (data not shown).

No inhibition of TNFα receptor was observed for VB-201 at concentrations of up to 17 µM (data not shown).

No inhibition of Lp-PLA2 was observed for VB-201 at concentrations of up to 40 µM (data not shown).

No inhibition by VB-201 of kinases, phosphatases, ion channels/ransporters was detected.

In addition, no agonistic or antagonistic effect of VB-201 on G-coupled protein receprors (GPRC) was observed.

Taken in combination with the above results showing that VB-201 does not affect activity of a variety of toll-like receptors (other than TLR2 and TLR4), these results indicate that VB-201 selectively inhibits CD14- and TLR2-associated pathways, and further confirm that the anti-inflammatory effects of VB-201 are mediated by CD14 and TLR2.

The citation or identification of any reference in this specification shall not be construed as an admission that such reference is available as prior art to the present invention. To the extent that section headings are used, they should not be construed as necessarily limiting.

## Claims

1. A compound, which is 1-hexadecyl-2-(4'-carboxybutyl)-glycerol-3-phosphocholine, for use in the treatment of an inflammatory disease or disorder, wherein said inflammatory disease or disorder is non-alcoholic steatohepatitis (NASH).

2. The compound for use according to claim 1, wherein said compound is (*R*)-1-hexadecyl-2-(4'-carboxybutyl)-sn-glycerol-3-phosphocholine.

3. The compound for use according to claim 1 or 2, wherein said compound exhibits at least two activities selected from the group consisting of:
a) inhibiting CD14 activity and/or a signaling pathway associated with CD14 activity;
b) inhibiting TLR2 activity and/or a signaling pathway associated with TLR2 activity; and
c) inhibiting monocyte chemotaxis.

4. The compound for use according to claim 3, wherein said activities are: inhibiting CD14 activity and/or a signaling pathway associated with CD14 activity; and inhibiting TLR2 activity and/or a signaling pathway associated with TLR2 activity.

5. The compound for use according to claim 3, wherein said activities are: inhibiting CD14 activity and/or a signaling pathway associated with CD14 activity; and inhibiting monocyte chemotaxis.

6. The compound for use according to claim 3, wherein said activities are: inhibiting TLR2 activity and/or a signaling pathway associated with TLR2 activity; and inhibiting monocyte chemotaxis.

7. The compound for use according to claim 3, wherein said activities are: inhibiting CD14 activity and/or a signaling pathway associated with CD14 activity; inhibiting TLR2 activity and/or a signaling pathway associated with TLR2 activity; and inhibiting monocyte chemotaxis.

8. The compound for use according to any of claims 1 to 7, wherein said compound is to be administered to a human.

9. The compound for use according to any of claims 1 to 8, wherein said compound inhibits TLR4 activity.

10. The compound for use according to any of claims 1 to 9, wherein the 1-hexadecyl-2-(4'-carboxybutyl)-glycerol-3-phosphocholine is in the form of a pharmaceutically acceptable salt, hydrate or solvate.

11. The compound for use according to any of claims 1 to 9, wherein said compound is at least one agent capable of exhibiting at least two activities selected from (a) inhibiting CD14 activity and/or a signaling pathway associated with CD14 activity, (b) inhibiting TLR2 activity and/or a signaling pathway associated with TLR2 activity, and (c) inhibiting monocyte chemotaxis, and wherein said agent is selected from 1-hexadecyl-2-(4'-carboxybutyl)-glycerol-3-phosphocholine and pharmaceutically acceptable salts, hydrates and solvates thereof.

12. The compound for use according to any of claims 1 to 11, wherein said compound is to be administered orally.

## Patentansprüche

1. Eine Verbindung, nämlich 1-Hexadecyl-2-(4'-carboxybutyl)-glycerin-3-phosphocholin, zur Verwendung bei der Behandlung einer entzündlichen Erkrankung oder Störung, wobei die entzündliche Erkrankung oder Störung nicht-alkoholische Steatohepatitis (NASH) ist.

2. Die Verbindung zur Verwendung nach Anspruch 1, wobei die Verbindung (*R*)-1-Hexadecyl-2-(4'-carboxybutyl)-*sn*-glycerin-3-phosphocholin ist.

3. Die Verbindung zur Verwendung nach Anspruch 1 oder 2, wobei die Verbindung mindestens zwei Aktivitäten aufweist, ausgewählt aus der Gruppe bestehend aus:
a) Inhibieren der CD14-Aktivität und/oder eines mit CD14-Aktivität in Verbindung stehenden Signalwegs;
b) Inhibieren der TLR2-Aktivität und/oder eines mit TLR2-Aktivität in Verbindung stehenden Signalwegs; und
c) Inhibieren der Monocytenchemotaxis.

4. Die Verbindung zur Verwendung nach Anspruch 3, wobei die Aktivitäten sind: Inhibieren der CD14-Aktivität und/oder eines mit CD14-Aktivität in Verbindung stehenden Signalwegs; und Inhibieren der TLR2-Aktivität und/oder eines mit TLR2-Aktivität in Verbindung stehenden Signalwegs.

5. Die Verbindung zur Verwendung nach Anspruch 3, wobei die Aktivitäten sind: Inhibieren der CD14-Aktivität und/oder eines mit CD14-Aktivität in Verbindung stehenden Signalwegs; und Inhibieren der Monocytenchemotaxis.

6. Die Verbindung zur Verwendung nach Anspruch 3, wobei die Aktivitäten sind: Inhibieren der TLR2-Aktivität und/oder eines mit TLR2-Aktivität in Verbindung stehenden Signalwegs; und Inhibieren der Monocytenchemotaxis.

7. Die Verbindung zur Verwendung nach Anspruch 3, wobei die Aktivitäten sind: Inhibieren der CD14-Aktivität und/oder eines mit CD14-Aktivität in Verbindung stehenden Signalwegs; Inhibieren der TLR2-Aktivität und/oder eines mit TLR2-Aktivität in Verbindung stehenden Signalwegs; und Inhibieren der Monocytenchemotaxis.

8. Die Verbindung zur Verwendung nach einem der Ansprüche 1 bis 7, wobei die Verbindung einem Menschen zu verabreichen ist.

9. Die Verbindung zur Verwendung nach einem der Ansprüche 1 bis 8, wobei die Verbindung die TLR4-Aktivität inhibiert.

10. Die Verbindung zur Verwendung nach einem der Ansprüche 1 bis 9, wobei das 1-Hexadecyl-2-(4'-carboxybutyl)-glycerin-3-phosphocholin in Form eines pharmazeutisch verträglichen Salzes, Hydrats oder Solvats vorliegt.

11. Die Verbindung zur Verwendung nach einem der Ansprüche 1 bis 9, wobei die Verbindung mindestens ein Mittel ist, das in der Lage ist, mindestens zwei Aktivitäten, ausgewählt aus (a) Inhibieren der CD14-Aktivität und/oder eines mit CD14-Aktivität in Verbindung stehenden Signalwegs, (b) Inhibieren der TLR2-Aktivität und/oder eines mit TLR2-Aktivität in Verbindung stehenden Signalwegs und (c) Inhibieren von Monocytenchemotaxis, aufzuweisen, und wobei das Mittel ausgewählt ist aus 1-Hexadecyl-2-(4'-carboxybutyl)-glycerin-3-phosphocholin und pharmazeutisch verträglichen Salzen, Hydraten und Solvaten davon.

12. Die Verbindung zur Verwendung nach einem der Ansprüche 1 bis 11, wobei die Verbindung oral zu verabreichen ist.

## Revendications

1. Composé, qui est une 1-hexadécyl-2-(4'-carboxybutyl)-glycérol-3-phosphocholine, pour une utilisation dans le traitement d'une maladie ou d'un trouble inflammatoire, où ladite maladie ou ledit trouble inflammatoire est la stéatohépatite non alcoolique (NASH).

2. Composé pour une utilisation selon la revendication 1, dans lequel ledit composé est la (*R*)-1-hexadécyl-2-(4'-carboxybutyl)-*sn*-glycérol-3-phosphocholine.

3. Composé pour une utilisation selon la revendication 1 ou 2, dans lequel ledit composé manifeste au moins deux activités choisies dans le groupe constitué par :
a) l'inhibition de l'activité CD14 et/ou d'une voie de signalisation associée à l'activité CD14 ;
b) l'inhibition de l'activité TLR2 et/ou d'une voie de signalisation associée à l'activité TLR2 ; et
c) l'inhibition de la chimiotaxie des monocytes.

4. Composé pour une utilisation selon la revendication 3, dans lequel lesdites activités sont : l'inhibition de l'activité CD14 et/ou d'une voie de signalisation associée à l'activité CD14 ; et l'inhibition de l'activité TLR2 et/ou d'une voie de signalisation associée à l'activité TLR2.

5. Composé pour une utilisation selon la revendication 3, dans lequel lesdites activités sont : l'inhibition de l'activité CD14 et/ou d'une voie de signalisation associée à l'activité CD14 ; et l'inhibition de la chimiotaxie des monocytes.

6. Composé pour une utilisation selon la revendication 3, dans lequel lesdites activités sont : l'inhibition de l'activité TLR2 et/ou d'une voie de signalisation associée à l'activité TLR2 ; et l'inhibition de la chimiotaxie des monocytes.

7. Composé pour une utilisation selon la revendication 3, dans lequel lesdites activités sont : l'inhibition de l'activité CD14 et/ou d'une voie de signalisation associée à l'activité CD14 ; l'inhibition de l'activité TLR2 et/ou d'une voie de signalisation associée à l'activité TLR2 ; et l'inhibition de la chimiotaxie des monocytes.

8. Composé pour une utilisation selon l'une quelconque des revendications 1 à 7, dans lequel ledit composé est destiné à être administré à un sujet humain.

9. Composé pour une utilisation selon l'une quelconque des revendications 1 à 8, dans lequel ledit composé inhibe l'activité TLR4.

10. Composé pour une utilisation selon l'une quelconque des revendications 1 à 9, dans lequel la l-hexadécyl-2-(4'-carboxybutyl)-glycérol-3-phosphocholine est sous la forme d'un sel, hydrate ou solvate pharmaceutiquement acceptable.

11. Composé pour une utilisation selon l'une quelconque des revendications 1 à 9, dans lequel ledit composé est au moins un agent capable de manifester au moins deux activités choisies parmi (a) l'inhibition de l'activité CD14 et/ou d'une voie de signalisation associée à l'activité CD14, (b) l'inhibition de l'activité TLR2 et/ou d'une voie de signalisation associée à l'activité TLR2, et (c) l'inhibition de la chimiotaxie des monocytes, et dans lequel ledit agent est choisi parmi la 1-hexadécyl-2-(4'-carboxybutyl)-glycérol-3-phosphocholine et ses sels, hydrates et solvates pharmaceutiquement acceptables.

12. Composé pour une utilisation selon l'une quelconque des revendications 1 à 11, dans lequel ledit composé est destiné à être administré par voie orale.
